# EUROPEAN PATENT SPECIFICATION

(11) **EP 3 102 231 B1**
(45) Date of publication and mention of the grant of the patent: **11.09.2019**
(21) Application number: 15710295.5
(22) Date of filing: 06.02.2015
(51) Int. Cl.: A61K 39/00, A61P 25/28, C07K 16/18

(54) **METHODS OF TREATING ALZHEIMER'S DISEASE**
VERFAHREN ZUR BEHANDLUNG VON MORBUS ALZHEIMER
MÉTHODE DE TRAITEMENT DE LA MALADIE D'ALZHEIMER

(30) Priority: 08.02.2014 US 201461937472 P; 27.03.2014 US 201461971479 P; 10.06.2014 US 201462010259 P; 19.11.2014 US 201462081992 P
(43) Date of publication of application: 14.12.2016
(60) Divisional application: 19188687.8
(73) Proprietor: F. Hoffmann-La Roche AG, 4070 Basel (CH)
(72) Inventor: CHO, William, South San Francisco, California 94080 (US); FRIESENHAHN, Michel, South San Francisco, California 94080 (US); PAUL, Robert, South San Francisco, California 94080 (US); WARD, Michael, South San Francisco, California 94080 (US)
(74) Representative: Mewburn Ellis LLP
(86) International application number: PCT/US2015/014758
(87) International publication number: WO 2015/120233

(56) References cited:
- WO-A2-2006/066049
- WO-A2-2012/016173
- RACHELLE S. DOODY ET AL: "Phase 3 Trials of Solanezumab for Mild-to-Moderate Alzheimer's Disease", NEW ENGLAND JOURNAL OF MEDICINE, vol. 370, no. 4, 23 January 2014 (2014-01-23), pages 311-321, XP055183832, ISSN: 0028-4793, DOI: 10.1056/NEJMoa1312889
- NIELS D PRINS ET AL: "Treating Alzheimer?s disease with monoclonal antibodies: current status and outlook for the future", ALZHEIMERS RES THER, BIOMED CENTRAL LTD, LONDON, UK, vol. 5, no. 6, 11 November 2013 (2013-11-11), page 56, XP021193607, ISSN: 1758-9193, DOI: 10.1186/ALZRT220
- O. ADOLFSSON ET AL: "An Effector-Reduced Anti- -Amyloid (A ) Antibody with Unique A Binding Properties Promotes Neuroprotection and Glial Engulfment of A", JOURNAL OF NEUROSCIENCE, vol. 32, no. 28, 11 July 2012 (2012-07-11) , pages 9677-9689, XP055097358, ISSN: 0270-6474, DOI: 10.1523/JNEUROSCI.4742-11.2012
- Charlotte Jago: "Alzheimer's Disease: One Year Later", , 3 February 2014 (2014-02-03), XP055183816, Retrieved from the Internet: URL:http://lsconnect.thomsonreuters.com/al zheimers-disease-year-later/ [retrieved on 2015-04-17]
- "Crenezumab Phase II cognition data in Alzheimer's disease presented", , 16 July 2014 (2014-07-16), XP055183813, Retrieved from the Internet: URL:http://www.acimmune.com/content/img/pa ges/ACImmune_crenezumab_phase2_ENGLISH_201 40716_final.pdf [retrieved on 2015-04-17]

## Description

### FIELD

Methods of treating patients suffering from mild to moderate Alzheimer's Disease using antibodies that target amyloid β are provided.

### BACKGROUND

Alzheimer's Disease (AD) is the most common cause of dementia, affecting an estimated 4.5 million individuals in the United States and 26.6 million worldwide (Hebert et al, Arch. Neurol. 2003; 60:1119-22; Brookmeyer et al., Alzheimers Dement. 2007; 3:186-91). The disease is characterized pathologically by the accumulation of extracellular β-amyloid ("Aβ") plaques and intracellular neurofibrillary tangles in the brain. Diagnosis is made through the clinical assessment of the neurologic and neuropsychiatric signs and symptoms of AD and the exclusion of other causes of dementia. AD is commonly classified into mild, moderate and severe stages by a brief cognitive screening examination, the Mini-Mental State Examination ("MMSE"). Approved medical therapies that inhibit acetylcholinesterase ("AChE") activity or antagonize N-methyl-D-aspartate receptors in the brain may temporarily improve the symptoms of AD in some patients but do not modify the progression of the disease (Cummings, N. Engl. J. Med. 2004; 351:56-67).

Genetic factors in early- and late-onset familial AD are now well documented. The ApoE4 allele is strongly associated with late-onset familial and sporadic AD, with a reported allele frequency of 50%-65% in patients with AD, which is approximately three times that in the general population and for other neurologic disorders (Saunders et al., Neurology 1993; 43:1467-72,; Prekumar et al., Am. J. Pathol. 1996; 148:2083-95). In addition to AD, the ApoE4 allele has been implicated in other amyloid-forming disorders, including cerebral amyloid angiopathy ("CAA") (Prekumar et al., Am. J. Pathol. 1996; 148:2083-95). Thus, patients who carry the ApoE4 allele may represent an etiologically distinct population of patients with AD.

The deposition of extracellular amyloid plaques in the brain is a hallmark pathologic finding in AD, first reported by Alois Alzheimer in 1906. These amyloid plaques are primarily composed of Abeta peptides (Haass and Selkoe, Nature 2007; 8:656-67) generated by the sequential cleavage of amyloid precursor protein ("APP") via β and γ-secretase activity. Abeta, particularly in its oligomerized forms, is toxic to neurons and is believed to be causative in AD. Therapies that reduce Abeta levels in the brain may alleviate cognitive dysfunction and block further synaptic loss, axon degeneration, and neuronal cell death. Abeta can be transported actively across the blood-brain barrier (Deane et al., Stroke 2004; 35(Suppl I):2628-31). In murine models of AD, systemic delivery of antibodies to Abeta increases Abeta levels in plasma while reducing levels in the central nervous system (CNS) through several proposed mechanisms, including dissolution of brain Abeta plaque, phagocytic removal of opsonized Abeta, and finally via efflux of Abeta from the brain as a result of an equilibrium shift of Abeta resulting from circulating antibodies (Morgan, Neurodegener. Dis. 2005; 2:261-6).

Significant failures have marked the development of therapeutic antibodies for the treatment of AD. Large-scale phase three clinical trials of bapineuzumab, an antibody binding specifically to the N-terminal portion of Abeta, were halted when administration of the drug failed to arrest cognitive decline in treated patients (Miles et al., Scientific Reports 2013; 3:1-4 Johnston & Johnson press release dated August 6, 2012, entitled "Johnson & Johnson Announces Discontinuation of Phase 3 Development of Bapineuzumab Intravenous (IV) in Mild-To-Moderate Alzheimer's Disease"). Notably, bapineuzumab did appear to stabilize plaque levels and decreased phosphorylated tau levels in cerebrospinal fluid - suggesting that modification of these biomarkers alone is not necessarily predictive of clinical efficacy (Miles et al., Scientific Reports 2013; 3:1-4). Similarly, in phase three clinical trials of solanezumab, an antibody specific for monomeric Abeta that binds in the middle portion of the peptide, the primary cognitive and functional endpoints were not met (Eli Lilly and Company press release dated August 24, 2012, "Eli Lilly and Company Aanounces Top-Line Results on Solanezumab Phase 3 Clinical Trials in Patients with Alzheimer's Disease"). Safety concerns have also been raised during the investigation of certain immunotherapies for AD; for example, incidence of amyloid-related imaging abnormalities (ARIA-E and ARIA-H) was over 20% among drug-treated patients in phase two clinical trials of bapineuzumab (Sperling et al., The Lancet 2012; 11:241-249). It is estimated that one in nine people over the age of 65 have AD -- the aggregated yearly costs for health care, long-term care and hospice care by and on behalf of individuals afflicted with AD are over $200 billion in 2013, and are estimated to rise to $1.2 trillion by 2050 (by and on behalf of affected individuals) (Alzheimer's Association 2013 Alzheimer's Disease Facts and Figures, Alzheimer's and Dementia 9:2). AD is the sixth-leading cause of death in the United States as of 2013 (*id*.). Current approved therapies treat only some of the symptoms of AD, and not the underlying degeneration. There is a tremendous unmet need for a disease-modifying therapeutic for AD.

### SUMMARY

Crenezumab (also known as MABT5102A) is a fully humanized IgG4 monoclonal antibody to Abeta selected for its ability to bind both monomeric and oligomeric forms of Abeta *in vitro.* Crenezumab binds both Abeta1-40 and Abeta 1-42, inhibits Abeta aggregation, and promotes Abeta disaggregation. Because crenezumab is a human IgG4 backbone antibody, it has reduced Feγ receptor ("FcλR") binding affinity compared with human IgG1 or IgG2, which is predictive of reduced immune effector response. These properties, combined with the ability of systemically delivered crenezumab to decrease Abeta CNS levels in a murine model of AD, have suggested that this anti-Abeta therapeutic approach may offer clinical efficacy while reducing risk of toxicity, and might potentially be able to modify the disease progression of AD with lower risk of the potentially deleterious side effects, such as cerebral vasogenic edema or hemorrhages, which had previously been seen in the clinical trials of other Abeta antibody therapies.

The results of phase two clinical studies in AD patients described herein demonstrate that crenezumab indeed slows the progression of disease in mild to moderate AD, has an even stronger effect in ApoE4 positive patients and in patients suffering from mild AD, and shows the greatest therapeutic benefit in patients with the mildest AD. Furthermore, the effect is seen in patients having a brain amyloid load that is typically seen in patients diagnosed with AD. Additionally, the results demonstrate that these effects occur without significant incidence of adverse events such as ARIA-E and ARIA-H. This application thus provides methods for treating and monitoring patients diagnosed with mild to moderate AD, especially mild AD, and ApoE4 positive patients, as well as patients having brain amyloid accumulation that is typically seen in patients diagnosed with AD. As exemplified herein, it has now been discovered that a humanized monoclonal anti-amyloid beta antibody with a conformational epitope specific for the middle region of amyloid beta (Aβ) peptide (*i*.*e*., within amino acids 13-24, such as crenezumab) is effective to treat mild to moderate AD, especially ApoE4 positive patients and patients with milder forms of AD, such as, but not limited to, mild AD, without an increased incidence of ARIA-E or ARIA-H. Accordingly, this application provides therapeutic agents for modulating the severity of AD and improved methods of using the same.

The present invention is defined in the claims, and relates to Crenezumab for use in a method of treating Alzheimer's Disease (AD) comprising delaying the development of AD or slowing the progression of AD in a patient with an MMSE score of 20 or above, without increasing the risk of an adverse event in said patient relative to a placebo, wherein the adverse event is selected from: (i) Amyloid-Related Imaging Abnormality-Edema (ARIA-E) and(ii) Amyloid-Related Imaging Abnormality-Hemorrhage (ARIA-H); wherein the crenezumab is administered at a dose of 15 mg/kg to 100 mg/kg of the patient's body weight, said dose being administered repeatedly every four weeks or every month.

The present application also describes methods of treating patients suffering from AD and other amyloidoses, comprising administering a humanized monoclonal anti-amyloid beta (Aβ or Abeta) antibody, or antigen-binding fragment thereof, that binds within residues 13 and 24 of amyloid β (1-42)(SEQ ID NO:1). In some embodiments, the antibody, or antigen-binding fragment thereof, is capable of binding fibrillar, oligomeric, and monomeric forms of Abeta. In some embodiments, the antibody is an IgG4 antibody. In particular embodiments, the antibody, or antigen-binding fragment thereof, comprises six hypervariable regions (HVRs) wherein HVR-H1 is SEQ ID NO:2, HVR-H2 is SEQ ID NO:3, HVR-H3 is SEQ ID NO:4, HVR-L1 is SEQ ID NO:6, HVR-L2 is SEQ ID NO:7, and HVR-L3 is SEQ ID NO:8. In some embodiments, the antibody, or antigen-binding fragment thereof, comprises a heavy chain having the amino acid sequence of SEQ ID NO:5, comprising a heavy chain variable region, and a light chain having the amino acid sequence of SEQ ID NO:9, comprising a light chain variable region. In a specific example, the antibody is crenezumab.

The methods of treatment described herein can be applied to patients suffering from AD or other amyloidosis, as described further herein. Suitable patients include patients suffering from mild to moderate AD, patients with an MMSE score of 18 to 26, patients suffering from mild AD, patients with an MMSE score of 20 or above (*e.g*., 20-30, 20-26, 24-30, 21-26, 22-26, 22-28, 23-26, 24-26, or 25-26), patients suffering from early AD (including patients having mild cognitive impairement due to AD and patients having preclinical AD), amyloid positive patients (or patients having brain amyloid load consistent with that seen in patients diagnosed with AD), and ApoE4 positive patients suffering from mild to moderate or mild AD.

In some aspects, the methods described herein are methods of reducing decline due to AD in patients suffering from early, mild, or mild to moderate AD. In some embodiments, the decline is one or more of: clinical decline, cognitive decline, and functional decline. In some embodiments, the decline is clinical decline. In some embodiments, the decline is a decline in cognitive capacity or cognitive decline. In some embodiments, the decline comprises a decline in functional capacity or functional decline. Various tests and scales have been developed to measure cognitive capacity (including memory) and/or function. In various embodiments, one or more test is used to measure clinical, functional, or cognitive decline. A standard measurement of cognitive capacity is the Alzheimer's Disease Assessment Scale Cognitive (ADAS-Cog) test, for example, the 12-item ADAS-Cog or ADAS-Cog12. Thus, in some embodiments, the reduction or slowing in decline in cognitive capacity (or cognitive decline) in patients being treated with the antibodies as described herein is determined using the ADAS-Cog12 test. An increase in ADAS-Cog12 score is indicative of worsening in a patient's condition. In some embodiments, the reduction or slowing in cognitive decline (or decline in cognitive capacity) in patients being treated with the antibodies as described herein is determined by a Clinical Dementia Rating Scale / Sum of Boxes (CDR-SOB) score. In some embodiments, reduction or slowing in functional decline (or decline in functional ability) in patients being treated with the antibodies as described herein is determined using the Instrumental Activities of Daily Living (iADL) scale. In some embodiments, decline of one or more types is assessed and one or more of the foregoing tests or scales is used to measure reduction or slowing in decline.

An antibody, or antigen-binding fragment thereof, as described herein is administered at a dose that is effective to treat the AD or other amyloidosis, as described herein. Suitable dosages are described herein and can range from about 0.3 mg/kg to 100 mg/kg. In an exemplary embodiment, the dosage is 15 mg/kg. In a further exemplary embodiment, the dosage is 30 mg/kg. In a further exemplary embodiment, the dosage is 45 mg/kg. In some embodiments, the dosage is between 500 mg and 1000mg, for example 500 mg, 700 mg, 720 mg, 750 mg, 800 mg, 820 mg, 900 mg, or between 1000 mg and 2500 mg, for example 1050 mg, 1500 mg, or 2100 mg. In the methods described herein, a variety of dosage regimens are contemplated including dosage regimens in which the antibody is administered repeatedly, *e.g.,* on a weekly or monthly schedule, over an extended period of time, *e.g.,* months to years.

The humanized monoclonal anti- Abeta antibody of the present disclosure provides a further benefit in that it does not increase the incidence of adverse events such as ARIA-E and ARIA-H. As shown herein, there was no increase in these adverse events in the treatment arm relative to the placebo arm. Thus, the present disclosure further provides methods of treating patients suffering from mild to moderate AD or mild AD without increasing the incidence of adverse events such as ARIA-E and/or ARIA-H.

The present disclosure further provides pharmaceutical formulations suitable for use in the methods of treatment disclosed herein. The pharmaceutical formulations can be formulated for any convenient route of administration, *e.g*., parenteral or intravenous injection, and will typically include, in addition to the anti-Abeta of the present disclosure, one or more acceptable carriers, excipients, and/or diluents suited to the desired mode of administration. In some embodiments, an antibody described herein may be formulated for intravenous administration. In some embodiments, an antibody of the invention may be formulated in an arginine buffer, e.g., an arginine succinate buffer. The buffer can contain one or more surfactants, *e.g*., a polysorbate. In certain embodiments, the buffer concentration is 50 mM or greater. In some embodiments, the pH is between 4.5 and 7.0, e.g., pH 5.5. Further embodiments are described herein. The pharmaceutical formulations can be package in unit dosage forms for ease of use.

Treatment with anti-Abeta antibodies for treatment of AD or other amyloidosis, as described herein, can be combined with other therapy, including one or more anti-Abeta antibodies other than crenezumab. Non-limiting examples of other therapy include neurological drugs, corticosteroids, antibiotics, and antiviral agents. Non-limiting examples of anti-Abeta antibodies other than crenezumab include solanezumab, bapineuzumab, aducanumab, and gantenerumab.

### BRIEF DESCRIPTION OF THE DRAWINGS

**FIG 1** provides the amino acid sequence of Abeta(1-42) (SEQ ID NO:1) with amino acids 13 to 24 underlined.
**FIG 2** provides the amino acid sequence of three heavy chain hypervariable regions (HVR-H1, HVR-H2, and HVR-H3, respectively) and the amino acid sequence of three light chain regions (HVR-L1, HVR-L2, HVR-L3, respectively).
**FIG 3** provides the amino acid sequence of heavy chain (SEQ ID NO:5), comprising the heavy chain variable region spanning amino acids 1 to 112 of SEQ ID NO:5, and light chain (SEQ ID NO:9), comprising the light chain variable region spanning amino acids 1 to 112 of SEQ ID NO:9, of crenezumab. The underlining in SEQ ID NOs:5 and 9 shows the amino acid sequences of the three heavy chain HVR corresponding to SEQ ID NOs:2-4 and the three light chain HVR corresponding to SEQ ID NOs:6-8, respectively.
**FIG 4A-B** provides a summary of the patients enrolled in the clinical trial described in Example 1, tabulating the number of patients enrolled in each arm (treatment versus placebo), ApoE4 status (ApoE4 negative/ ApoE4 positive), stage of AD (mild or moderate), and MMSE scores at screening, existence and type of concurrent therapy (conmed use) for AD symptoms.
**FIG 5** provides a schematic of the clinical trials described in Example 1, showing the dosing schedule, amount, and route.
**FIG 6A-B** provide data tables showing the change in ADAS-Cog12 scores at 73 weeks relative to baseline, in the treatment arm and the placebo arm. **FIG 6A** provides data for patients with mild to moderate AD, mild AD, moderate AD, and ApoE4 positive and negative patients. **FIG 6B** provides data for patients according to MMSE score.
**FIG 7** provides a chart of the change in ADAS-Cog12 scores for patients with mild AD having an MMSE score between 20 and 26, treated with crenezumab (dark solid line) or placebo (light solid line).
**FIG 8** provides a chart of the change in ADAS-Cog12 scores for patients with mild to moderate AD having an MMSE score between 18 and 26 treated with crenezumab (dark solid line) or placebo (light solid line).
**FIG 9** provides a chart of the change in ADAS-Cog12 scores for ApoE4 positive patients with mild-to-moderate AD treated with crenezumab (dark solid line) or placebo (light solid line).
**FIG 10** provides a chart of the change in ADAS-Cog12 scores across all ApoE4 positive patients and patients with mild AD treated with crenezumab (dark solid line) or placebo (light solid line).
**FIG 11** provides a chart of the change in ADAS-Cog12 scores for patients with mild AD having an MMSE score between 22 and 26, treated with crenezumab or placebo.
**FIG 12A-B** provide data tables showing the change in CDR-SOB scores at 73 weeks relative to baseline, in the treatment arm and the placebo arm. **FIG 12A** provides data for the change in CDR-SOB scores for patients according to MMSE score. **FIG 12B** provides data for CDR-SOB scores as well as CDR Judgment and Problem solving scores and CDR Memory scores for patients with MMSE scores ranging from 18-26, 20-26, and 22-26.
**FIG 13** provides a chart of the change in CDR-SOB scores for patients with mild AD, having an MMSE score of 25 or 26, treated with crenezumab or placebo as indicated.
**FIG 14A-B** provides a summary of the patients enrolled in the clinical trial described in Example 2 at baseline and after treatment, including adverse event data (A) and a timeline showing when PET scans, MRI scans, and CSF sampling was performed in the clinical trial (B).
**FIG 15A-B** provides charts showing amyloid levels in patients receiving placebo (dashed line) or crenezumab (solid line), as measured by imaging of florbetapir by PET analysis (A) and CSF Abeta levels levels in patients receiving placebo or crenezumab (B).

### DETAILED DESCRIPTION

Unless defined otherwise, technical and scientific terms used herein have the same meaning as commonly understood by one of ordinary skill in the art to which this invention belongs. Singleton et al. et al., Dictionary of Microbiology and Molecular Biology 2nd ed., J. Wiley & Sons (New York, N.Y. 1994), and March, Advanced Organic Chemistry Reactions, Mechanisms and Structure 4th ed., John Wiley & Sons (New York, N.Y. 1992), provide one skilled in the art with a general guide to many of the terms used in the present application.

### Certain Definitions and Abbreviations

For purposes of interpreting this specification, the following definitions will apply and whenever appropriate, terms used in the singular will also include the plural and vice versa. In the event that any definition set forth below conflicts with any document incorporated herein by reference, the definition set forth below shall control.

As used in this specification and the appended claims, the singular forms "a," "an" and "the" include plural referents unless the context clearly dictates otherwise. Thus, for example, reference to "a protein" or an "antibody" includes a plurality of proteins or antibodies, respectively; reference to "a cell" includes mixtures of cells, and the like.

Ranges provided in the specification and appended claims include both end points and all points between the end points. Thus, for example, a range of 2.0 to 3.0 includes 2.0, 3.0, and all points between 2.0 and 3.0.

The phrase "substantially similar," or "substantially the same," as used herein, denotes a sufficiently high degree of similarity between two numeric values (generally one associated with an antibody as described herein and the other associated with a reference/comparator antibody) such that one of skill in the art would consider the difference between the two values to be of little or no biological and/or statistical significance within the context of the biological characteristic measured by said values (*e.g*., Kd values). The difference between said two values is less than about 50%, less than about 40%, less than about 30%, less than about 20%, less than about 10% as a function of the value for the reference/comparator antibody.

The term "sample," or "test sample" as used herein, refers to a composition that is obtained or derived from a subject of interest that contains a cellular and/or other molecular entity that is to be characterized and/or identified, for example based on physical, biochemical, chemical and/or physiological characteristics. In one embodiment, the definition encompasses blood and other liquid samples of biological origin and tissue samples such as a biopsy specimen or tissue cultures or cells derived therefrom. The source of the tissue sample may be solid tissue as from a fresh, frozen and/or preserved organ or tissue sample or biopsy or aspirate; blood or any blood constituents; bodily fluids; and cells from any time in gestation or development of the subject or plasma. The term "biological sample" as used herein includes, but is not limited to, blood, serum, plasma, sputum, tissue biopsies (*e.g*., lung samples), and nasal samples including nasal swabs or nasal polyps.

The term "sample," "biological sample,' or "test sample" includes biological samples that have been manipulated in any way after their procurement, such as by treatment with reagents, solubilization, or enrichment for certain components, such as proteins or polynucleotides, or embedding in a semi-solid or solid matrix for sectioning purposes. For the purposes herein a "section" of a tissue sample is meant a single part or piece of a tissue sample, e.g. a thin slice of tissue or cells cut from a tissue sample. Samples include, but are not limited to, whole blood, blood-derived cells, serum, plasma, lymph fluid, synovial fluid, cellular extracts, and combinations thereof. In one embodiment, the sample is a clinical sample. In another embodiment, the sample is used in a diagnostic assay.

In one embodiment, a sample is obtained from a subject or patient prior to treatment with an anti-Abeta antibody. In another embodiment, a sample is obtained from a subject or patient following at least one treatment with an anti-Abeta antibody.

A "reference sample," as used herein, refers to any sample, standard, or level that is used for comparison purposes. In one embodiment, a reference sample is obtained from a healthy and/or non-diseased part of the body (e.g., tissue or cells) of the same subject or patient. In another embodiment, a reference sample is obtained from an untreated tissue and/or cell of the body of the same subject or patient. In yet another embodiment, a reference sample is obtained from a healthy and/or non-diseased part of the body (e.g., tissues or cells) of an individual who is not the subject or patient. In even another embodiment, a reference sample is obtained from an untreated tissue and/or cell part of the body of an individual who is not the subject or patient.

In certain embodiments, a reference sample is a single sample or combined multiple samples from the same subject or patient that are obtained at one or more different time points than when the test sample is obtained. For example, a reference sample is obtained at an earlier time point from the same subject or patient than when the test sample is obtained. In certain embodiments, a reference sample includes all types of biological samples as defined above under the term "sample" that is obtained from one or more individuals who is not the subject or patient. In certain embodiments, a reference sample is obtained from one or more individuals with amyloidosis, e.g., Alzheimer's Disease, who is not the subject or patient.

In certain embodiments, a reference sample is a combined multiple samples from one or more healthy individuals who are not the subject or patient. In certain embodiments, a reference sample is a combined multiple samples from one or more individuals with a disease or disorder (e.g., amyloidosis such as, for example, Alzheimer's Disease) who are not the subject or patient. In certain embodiments, a reference sample is pooled RNA samples from normal tissues or pooled plasma or serum samples from one or more individuals who are not the subject or patient.

The term "small molecule" refers to an organic molecule having a molecular weight between 50 Daltons to 2500 Daltons.

The terms "antibody" and "immunoglobulin" ("Ig") are used interchangeably in the broadest sense and include, but are not limited to, monoclonal antibodies (for example, full length or intact monoclonal antibodies), polyclonal antibodies, multivalent antibodies, antibodies with polyepitopic specificity, single chain antibodies, multi-specific antibodies (for example, bispecific antibodies, trispecific antibodies, tetraspecific antibodies), and fragments of antibodies, provided they exhibit the desired biological activity. Such antibodies can be chimeric, humanized, human, synthetic, and/or affinity matured. Such antibodies and methods of generating them are described in more detail herein.

"Antibody fragments" comprise only a portion of an intact antibody, wherein the portion preferably retains at least one, and typically most or all, of the functions normally associated with that portion when present in an intact antibody. In one embodiment, an antibody fragment comprises an antigen binding site of the intact antibody and thus retains the ability to bind antigen. In another embodiment, an antibody fragment, for example one that comprises the Fc region, retains at least one of the biological functions normally associated with the Fc region when present in an intact antibody, such as FcRn binding, antibody half life modulation, ADCC function and complement binding. In one embodiment, an antibody fragment is a monovalent antibody that has an *in vivo* half life substantially similar to an intact antibody. For example, such an antibody fragment may comprise an antigen binding arm linked to an Fc sequence capable of conferring *in vivo* stability to the fragment. Examples of antibody fragments include but are not limited to Fv, Fab, Fab', Fab'-SH, F(ab')2; diabodies; linear antibodies; single-chain antibody molecules (*e.g*. scFv); and multispecific antibodies formed from antibody fragments.

The term "target," as used herein, refers to any native molecule from any vertebrate source, including mammals such as primates (*e.g.* humans) and rodents (*e.g.,* mice and rats), unless otherwise indicated. The term encompasses "full-length," unprocessed target as well as any form of target that results from processing in the cell. The term also encompasses naturally occurring variants of targets, *e.g*., splice variants or allelic variants.

The terms "amyloid beta," "beta-amyloid," "Abeta," "amyloidβ," and "Aβ", used interchangeably herein, refer to the fragment of amyloid precursor protein ("APP") that is produced upon β-secretase 1 ("BACE1") cleavage of APP, as well as modifications, fragments and any functional equivalents thereof, including, but not limited to, Aβ1-40, and Aβ1-42. Aβ is known to exist in monomeric form, as well as to associate to form oligomers and fibril structures, which may be found as constituent members of amyloid plaque. The structure and sequences of such Aβ peptides are well known to one of ordinary skill in the art and methods of producing said peptides or of extracting them from brain and other tissues are described, for example, in Glenner and Wong, Biochem Biophys Res. Comm. 129: 885-890 (1984). Moreover, Aβ peptides are also commercially available in various forms. An exemplary amino acid sequence of human Aβ1-42 is DAEFRHDSGYEVHHQKLVFFAED VGSNKGAIIGLMVGGVVIA (SEQ ID NO: 1).

The terms "anti-target antibody" and "an antibody that binds to target" refer to an antibody that is capable of binding the target with sufficient affinity such that the antibody is useful as a diagnostic and/or therapeutic agent in targeting the target. In one embodiment, the extent of binding of an anti-target antibody to an unrelated, non-target protein is less than about 10% of the binding of the antibody to target as measured, *e.g*., by a radioimmunoassay (RIA) or biacore assay. In certain embodiments, an antibody that binds to a target has a dissociation constant (Kd) of ≤ 1µM, ≤ 100 nM, ≤ 10 nM, ≤ 1 nM, ≤ 0.1 nM, ≤ 0.01 nM, or ≤ 0.001 nM (*e.g*., 10⁻⁸ M or less, *e.g*., from 10⁻⁸ M to 10⁻¹³ M, *e.g*., from 10⁻⁹ M to 10⁻¹³ M). In certain embodiments, an anti-target antibody binds to an epitope of a target that is conserved among different species.

"Anti-Abeta immunoglobulin," "anti-Abeta antibody," and "antibody that binds Abeta" are used interchangeably herein, and refer to an antibody that specifically binds to human Abeta. A nonlimiting example of an anti-Abeta antibody is crenezumab. Other non-limiting examples of anti-Abeta antibodies are solanezumab, bapineuzumab, aducanumab, and gantenerumab.

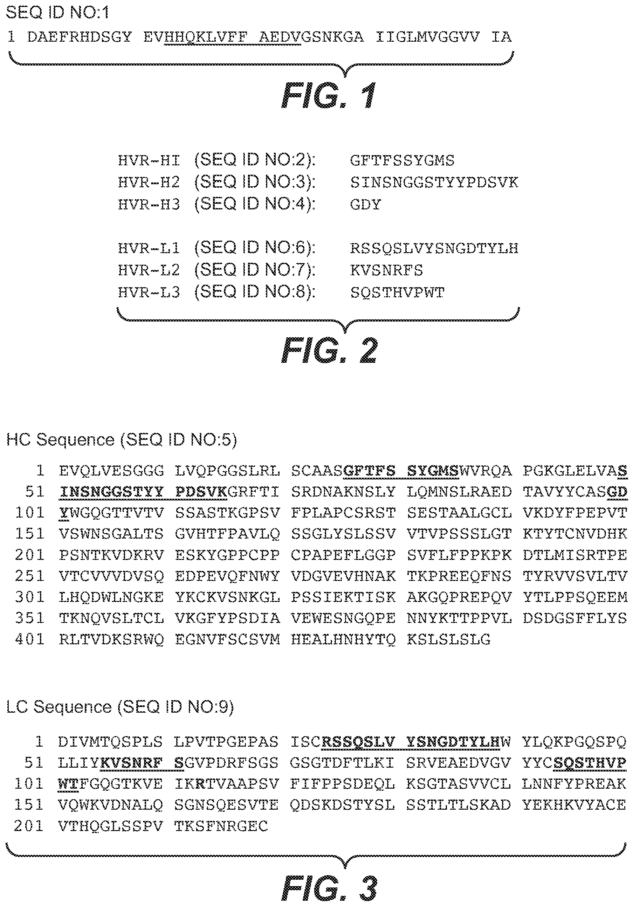

The terms "crenezumab" and "MABT5102A" are used interchangeably herein, and refer to a specific anti-Abeta antibody that binds to monomeric, oligomeric, and fibril forms of Abeta, and which is associated with CAS registry number 1095207. In one embodiment, such antibody comprises HVR region sequences set forth in **FIG 2**. In another such embodiment, such antibody comprises: (1) an HVR-H1 comprising the amino acid sequence SEQ ID NO: 2; (2) an HVR-H2 sequence comprising the amino acid sequence SEQ ID NO: 3; (3) an HVR-H3 sequence comprising the amino acid sequence SEQ ID NO: 4; (4) an HVR-L1 sequence comprising the amino acid sequence SEQ ID NO: 6; (5) an HVR-L2 sequence comprising the amino acid sequence SEQ ID NO: 7; and (6) an HVR-L3 sequence comprising the amino acid sequence SEQ ID NO: 8. In another embodiment, the specific anti-Abeta antibody comprises VH and VL domains having the amino acid sequences set forth in **FIG 3**. In another such embodiment, such specific anti-Abeta antibody comprises a VH domain comprising the amino acid sequence SEQ ID NO: 5 and a VL domain comprising the amino acid sequence SEQ ID NO: 9. In another embodiment, the antibody is an IgG4 antibody. In another such embodiment, the IgG4 antibody comprises a mutation in its constant domain such that serine 228 is instead a proline.

The term "amyloidosis," as used herein, refers to a group of diseases and disorders caused by or associated with amyloid or amyloid-like proteins and includes, but is not limited to, diseases and disorders caused by the presence or activity of amyloid-like proteins in monomeric, fibril, or polymeric state, or any combination of the three, including by amyloid plaques. Such diseases include, but are not limited to, secondary amyloidosis and age-related amyloidosis, such as diseases including, but not limited to, neurological disorders such as Alzheimer's Disease ("AD"), diseases or conditions characterized by a loss of cognitive memory capacity such as, for example, mild cognitive impairment (MCI), Lewy body dementia, Down's syndrome, hereditary cerebral hemorrhage with amyloidosis (Dutch type), the Guam Parkinson-Demential complex and other diseases which are based on or associated with amyloid-like proteins such as progressive supranuclear palsy, multiple sclerosis, Creutzfeld Jacob disease, Parkinson's disease, HIV-related dementia, ALS (amyotropic lateral sclerosis), inclusion-body myositis (IBM), adult onset diabetes, endocrine tumor and senile cardiac amyloidosis, and various eye diseases including macular degeneration, drusen-related optic neuropathy, glaucoma, and cataract due to beta-amyloid deposition.

Glaucoma is a group of diseases of the optic nerve involving loss of retinal ganglion cells (RGCs) in a characteristic pattern of optic neuropathy. RGCs are the nerve cells that transmit visual signals from the eye to the brain. Caspase-3 and Caspase-8, two major enzymes in the apoptotic process, are activated in the process leading to apoptosis of RGCs. Caspase-3 cleaves amyloid precursor protein (APP) to produce neurotoxic fragments, including Abeta. Without the protective effect of APP, Abeta accumulation in the retinal ganglion cell layer results in the death of RGCs and irreversible loss of vision.

Glaucoma is often, but not always, accompanied by an increased eye pressure, which may be a result of blockage of the circulation of aqueous, or its drainage. Although raised intraocular pressure is a significant risk factor for developing glaucoma, no threshold of intraocular pressure can be defined which would be determinative for causing glaucoma. The damage may also be caused by poor blood supply to the vital optic nerve fibers, a weakness in the structure of the nerve, and/or a problem in the health of the nerve fibers themselves. Untreated glaucoma leads to permanent damage of the optic nerve and resultant visual field loss, which can progress to blindness.

The different types of glaucomas are classified as open-angle glaucomas, if the condition is chronic, or closed-angle glaucomas, if acute glaucoma occurs suddenly. Glaucoma usually affects both eyes, but the disease can progress more rapidly in one eye than in the other.

Chronic open-angle glaucoma (COAG), also known as primary open angle glaucoma (POAG), is the most common type of glaucoma. COAG is caused by microscopic blockage in the trabecular meshwork, which decreases the drainage of the aqueous outflow into the Schlemm's canal and raises the intraocular pressure (IOP). POAG usually affects both eyes and is strongly associated with age and a positive family history. Its frequency increases in elderly people as the eye drainage mechanism may gradually become clogged with aging. The increase in intraocular pressure in subjects affected by chronic open-angle glaucoma is not accompanied by any symptoms until the loss is felt on the central visual area.

Acute Angle Closure Glaucoma (AACG) or closed-angle glaucoma is a relatively rare type of glaucoma characterized by a sudden increase in intraocular pressure to 35 to 80 mmHg, leading to severe pain and irreversible loss of vision.. The sudden pressure increase is caused by the closing of the filtering angle and blockage of the drainage channels. Individuals with narrow angles have an increased risk for a sudden closure of the angle. AACG usually occurs monocularly, but the risk exists in both eyes. Age, cataract and pseudoexfoliation are also risk factors since they are associated with enlargement of the lens and crowding or narrowing of the angle. A sudden glaucoma attack may be associated with severe eye pain and headache, inflamed eye, nausea, vomiting, and blurry vision.

Mixed or Combined Mechanism Glaucoma is a mixture or combination of open and closed angle glaucoma. It affects patients with acute ACG whose angle opens after laser iridotomy, but who continue to require medications for IOP control, as well as patients with POAG or pseudoexfoliative glaucoma who gradually develop narrowing of the angle.

Normal tension glaucoma (NTG), also known as low tension glaucoma (LTG), is characterized by progressive optic nerve damage and loss of peripheral vision similar to that seen in other types of glaucoma; however, the intraocular pressure is the normal range or even below normal.

Congenital (infantile) glaucoma is a relatively rare, inherited type of open-angle glaucoma. Insufficient development of the drainage area results in increased pressure in the eye that can lead to the loss of vision from optic nerve damage and to an enlarged eye. Early diagnosis and treatment are critical to preserve vision in infants and children affected by the disease.

Secondary glaucoma may result from an ocular injury, inflammation in the iris of the eye (iritis), diabetes, cataract, or use of steroids in steroid-susceptible individuals. Secondary glaucoma may also be associated with retinal detachment or retinal vein occlusion or blockage.

Pigmentary glaucoma is characterized by the detachment of granules of pigment from the iris. The granules cause blockage of the drainage system of the eye, leading to elevated intraocular pressure and damage to the optic nerve. Exfoliative glaucoma (pseudoexfoliation) is characterized by deposits of flaky material on the anterior capsule and in the angle of the eye. Accumulation of the flaky material blocks the drainage system and raises the eye pressure.

Diagnosis of glaucoma may be made using various tests. Tonometry determines the pressure in the eye by measuring the tone or firmness of its surface. Several types of tonometers are available for this test, the most common being the applanation tonometer. Pachymetry determines the thickness of the cornea which, in turn, measures intraocular pressure. Gonioscopy allows examination of the filtering angle and drainage area of the eye. Gonioscopy can also determine if abnormal blood vessels may be blocking the drainage of the aqueous fluid out of the eye. Ophthalmoscopy allows examination of the optic nerve and can detect nerve fiber layer drop or changes in the optic disc, or indentation (cupping) of this structure, which may be caused by increased intraocular pressure or axonal drop out. Gonioscopy is also useful in assessing damage to the nerve from poor blood flow or increased intraocular pressure. Visual Field testing maps the field of vision, subjectively, which may detect signs of glaucomatous damage to the optic nerve. This is represented by specific patterns of visual field loss. Ocular coherence tomography, an objective measure of nerve fiber layer loss, is carried out by looking at the thickness of the optic nerve fiber layer (altered in glaucoma) via a differential in light transmission through damaged axonal tissue.

An "antibody that binds to the same epitope" as a reference antibody refers to an antibody that blocks binding of the reference antibody to its antigen in a competition assay by 50% or more, and conversely, the reference antibody blocks binding of the antibody to its antigen in a competition assay by 50% or more. An exemplary competition assay is provided herein.

The term "therapeutic agent" refers to any agent that is used to treat a disease, including but not limited to an agent that treats a symptom of the disease.

As used herein, "treatment" (and grammatical variations thereof such as "treat" or "treating") refers to clinical intervention in an attempt to alter the natural course of the individual being treated, and can be performed during the course of clinical pathology. Desirable effects of treatment include, but are not limited to, alleviation or amelioration of one or more symptoms, diminishment of or delay in the appearance of or worsening of any direct or indirect pathological consequences of the disease, decrease of the rate of disease progression, and amelioration or palliation of the disease state. In some embodiments, antibodies are used to delay development of a disease or to slow the progression of a disease.

The term "treatment emergent" as used herein refers to an event that occurs after a first dose of a therapeutic agent is administered. For example, a "treatment emergent adverse event" is an event that is identified upon or after the first dose of a treatment in a clinical study.

"Treatment regimen" refers to a combination of dosage, frequency of administration, or duration of treatment, with or without addition of a second medication.

"Effective treatment regimen" refers to a treatment regimen that will offer beneficial response to a patient receiving the treatment.

"Modifying a treatment" refers to changing the treatment regimen including, changing dosage, frequency of administration, or duration of treatment, and/or addition of a second medication.

An "effective amount" or "effective dose" of an agent refers to an amount or dose effective, for periods of time necessary, to achieve the desired result. For example, a "therapeutically effective amount" is an amount effective, for periods of time necessary, to treat the indicated disease, condition, clinical pathology, or symptom, *i*.*e*., to modify the course of progression of AD and/or to alleviate and/or prevent one or more symptoms of AD.

"Affinity" or "binding affinity" refers to the strength of the sum total of noncovalent interactions between a single binding site of a molecule (*e.g*., an antibody) and its binding partner (*e.g*., an antigen). Unless indicated otherwise, as used herein, "binding affinity" refers to intrinsic binding affinity which reflects a 1:1 interaction between members of a binding pair (*e.g*., antibody and antigen binding arm). The affinity of a molecule X for its partner Y can generally be represented by the dissociation constant (Kd). Affinity can be measured by common methods known in the art, including those described herein, any of which can be used for purposes of the present invention. Specific illustrative and exemplary embodiments for measuring binding affinity are described herein.

An "affinity matured" antibody refers to an antibody with one or more alterations in one or more hypervariable regions (HVRs), compared to a parent antibody which does not possess such alterations, such alterations resulting in an improvement in the affinity of the antibody for antigen.

As used herein, the term "patient" refers to any single subject for which treatment is desired. In certain embodiments, the patient herein is a human.

A "subject" herein is typically a human. In certain embodiments, a subject is a non-human mammal. Exemplary non-human mammals include laboratory, domestic, pet, sport, and stock animals, *e.g*., mice, cats, dogs, horses, and cows. Typically, the subject is eligible for treatment, *e.g*., displays one or more indicia of disease. Generally, such subject or patient is eligible for treatment for amyloidosis, e.g., AD. In one embodiment, such eligible subject or patient is one that is experiencing or has experienced one or more signs, symptoms, or other indicators of AD or has been diagnosed with AD, whether, for example, newly diagnosed, previously diagnosed or at risk for developing AD. Diagnosis of AD may be made based on clinical history, clinical examination, and established imaging modalities. A "patient" or "subject" herein includes any single human subject eligible for treatment who is experiencing or has experienced one or more signs, symptoms, or other indicators of AD. Intended to be included as a subject are any subjects involved in clinical research trials, or subjects involved in epidemiological studies, or subjects once used as controls. The subject may have been previously treated with an anti-Abeta antibody, or antigen-binding fragment thereof, or another drug, or not so treated. The subject may be naive to an additional drug(s) being used when the treatment herein is started, i.e., the subject may not have been previously treated with, for example, a therapy other than anti-Abeta at "baseline" (i.e., at a set point in time before the administration of a first dose of anti-Abeta in the treatment method herein, such as the day of screening the subject before treatment is commenced). Such "naive" subjects are generally considered to be candidates for treatment with such additional drug(s).

As used herein, "lifetime" of a subject refers to the remainder of the life of the subject after starting treatment.

The term "monoclonal antibody" as used herein refers to an antibody obtained from a population of substantially homogeneous antibodies, *i.e*., the individual antibodies comprising the population are identical except for possible naturally occurring mutations that may be present in minor amounts. Monoclonal antibodies are highly specific, being directed against a single antigen. Furthermore, in contrast to polyclonal antibody preparations that typically include different antibodies directed against different determinants (epitopes), each monoclonal antibody is directed against a single determinant on the antigen.

The monoclonal antibodies herein specifically include "chimeric" antibodies in which a portion of the heavy and/or light chain is identical with or homologous to corresponding sequences in antibodies derived from a particular species or belonging to a particular antibody class or subclass, while the remainder of the chain(s) is identical with or homologous to corresponding sequences in antibodies derived from another species or belonging to another antibody class or subclass, as well as fragments of such antibodies, so long as they exhibit the desired biological activity (U.S. Patent No. 4,816,567; and Morrison et al., Proc. Natl. Acad. Sci. USA 81:6851-6855 (1984)).

The "class" of an antibody refers to the type of constant domain or constant region possessed by its heavy chain. There are five major classes of antibodies: IgA, IgD, IgE, IgG, and IgM, and several of these may be further divided into subclasses (or "isotypes"), *e.g*., IgG1, IgG2, IgG3, IgG4, IgA1, and IgA2. The heavy chain constant domains that correspond to the different classes of immunoglobulins are called α, δ, ε, γ, and µ, respectively.

"Humanized" forms of non-human (*e*.*g*., murine) antibodies are chimeric antibodies that contain minimal sequence derived from non-human immunoglobulin. For the most part, humanized antibodies are human immunoglobulins (recipient antibody) in which residues from a hypervariable region of the recipient are replaced by residues from a hypervariable region of a non-human species (donor antibody) such as mouse, rat, rabbit or nonhuman primate having the desired specificity, affinity, and capacity. In some instances, framework region (FR) residues of the human immunoglobulin are replaced by corresponding non-human residues. Furthermore, humanized antibodies may comprise residues that are not found in the recipient antibody or in the donor antibody. These modifications are made to further refine antibody performance. In general, the humanized antibody will comprise substantially all of at least one, and typically two, variable domains, in which all or substantially all of the hypervariable loops correspond to those of a non-human immunoglobulin and all or substantially all of the FRs are those of a human immunoglobulin lo sequence. The humanized antibody optionally will also comprise at least a portion of an immunoglobulin constant region (Fc), typically that of a human immunoglobulin. For further details, see Jones et al., Nature 321:522-525 (1986); Riechmann et al., Nature 332:323-329 (1988); and Presta, Curr. Op. Struct. Biol. 2:593-596 (1992). See also the following review articles and references cited therein: Vaswani and Hamilton, Ann. Allergy, Asthma & Immunol. 1: 105-115 (1998); Harris, Biochem. Soc. Transactions 23:1035-1038 (1995); Hurle and Gross, Curr. Op. Biotech. 5:428-433 (1994).

A "human antibody" is one which comprises an amino acid sequence corresponding to that of an antibody produced by a human or a human cell and/or has been derived from a non-human source that utilizes human antibody repertoires or other human antibody-encoding sequences, for example made using any of the techniques for making human antibodies as disclosed herein. Such techniques include, but are not limited to, screening human-derived combinatorial libraries, such as phage display libraries (see, *e.g.,* Marks et al., J. Mol. Biol., 222: 581-597 (1991) and Hoogenboom et al., Nucl. Acids Res., 19: 4133-4137 (1991)); using human myeloma and mouse-human heteromyeloma cell lines for the production of human monoclonal antibodies (see, *e.g.,* Kozbor J. Immunol., 133: 3001 (1984); Brodeur et al., Monoclonal Antibody Production Techniques and Applications, pp. 55-93 (Marcel Dekker, Inc., New York, 1987); and Boerner et al., J. Immunol., 147: 86 (1991)); and generating monoclonal antibodies in transgenic animals (*e.g.,* mice) that are capable of producing a full repertoire of human antibodies in the absence of endogenous immunoglobulin production (see, *e.g.,* Jakobovits et al., Proc. Natl. Acad. Sci USA, 90: 2551 (1993); Jakobovits et al., Nature, 362: 255 (1993); Bruggermann et al.,Year in Immunol., 7: 33 (1993)). This definition of a human antibody specifically excludes a humanized antibody comprising antigen-binding residues from a non-human animal.

An "isolated" antibody is one which has been identified and separated and/or recovered from a component of its natural environment. Contaminant components of its natural environment are materials which would interfere with diagnostic or therapeutic uses for the antibody, and may include enzymes, hormones, and other proteinaceous or nonproteinaceous solutes. In some embodiments, an antibody is purified to greater than 95% or 99% purity as determined by, for example, electrophoretic (e.g., SDS-PAGE, isoelectric focusing (IEF), capillary electrophoresis) or chromatographic (e.g., ion exchange or reverse phase HPLC). For review of methods for assessment of antibody purity, see, *e.g.,* Flatman et al., J. Chromatogr. B 848:79-87 (2007).

The term "variable region" or "variable domain" refers to the domain of an antibody heavy or light chain that is involved in binding the antibody to antigen. The variable domains of the heavy chain and light chain (VH and VL, respectively) of a native antibody generally have similar structures, with each domain comprising four conserved framework regions (FRs) and three hypervariable regions (HVRs). (See, *e.g.,* Kindt et al. Kuby Immunology, 6th ed., W.H. Freeman and Co., page 91 (2007).) A single VH or VL domain may be sufficient to confer antigen-binding specificity. Furthermore, antibodies that bind a particular antigen may be isolated using a VH or VL domain from an antibody that binds the antigen to screen a library of complementary VL or VH domains, respectively. *See, e.g.,* Portolano et al., J. Immunol. 150:880-887 (1993); Clarkson et al., Nature 352:624-628 (1991).

The term "hypervariable region," "HVR," or "HV," when used herein refers to the regions of an antibody variable domain which are hypervariable in sequence and/or form structurally defined loops. Generally, antibodies comprise six hypervariable regions; three in the VH (HI, H2, H3), and three in the VL (LI, L2, L3). A number of hypervariable region delineations are in use and are encompassed herein. The Kabat Complementarity Determining Regions (CDRs) are based on sequence variability and are the most commonly used (Kabat et al., Sequences of Proteins of Immunological Interest, 5th Ed. Public Health Service, National Institutes of Health, Bethesda, Md. (1991)). Chothia refers instead to the location of the structural loops (Chothia and Lesk J. Mol. Biol. 196:901-917 (1987)). The AbM hypervariable regions represent a compromise between the Kabat CDRs and Chothia structural loops, and are used by Oxford Molecular's AbM antibody modeling software. The "contact" hypervariable regions are based on an analysis of the available complex crystal structures. The residues from each of these HVRs are noted below.

| Loop | Kabat | AbM | Chothia | Contact |
|---|---|---|---|---|
| L1 | L24-L34 | L24-L34 | L26-L32 | L30-L36 |
| L2 | L50-L56 | L50-L56 | L50-L52 | L46-L55 |
| L3 | L89-L97 | L89-L97 | L91-L96 | L89-L96 |
| H1 | H31-H35B | H26-H35B | H26-H32 | H30-H35B (Kabat Numbering) |
| H1 | H31-H35 | H26-H35 | H26-H32 | H30-H35 (Chothia Numbering) |
| H2 | H50-H65 | H50-H58 | H53-H55 | H47-H58 |
| H3 | H95-H102 | H95-H102 | H96-H101 | H93-H101 |

Hypervariable regions may comprise "extended hypervariable regions" as follows: 24-36 or 24-34 (LI), 46-56 or 49-56 or 50-56 or 52-56 (L2) and 89-97 (L3) in the VL and 26-35 (HI), 50-65 or 49-65 (H2) and 93-102, 94-102 or 95-102 (H3) in the VH. The variable domain residues are numbered according to Kabat *et al*., *supra* for each of these definitions.

"Framework" or "FR" residues are those variable domain residues other than the hypervariable region residues as herein defined. The FR of a variable domain generally consists of four FR domains: FR1, FR2, FR3, and FR4. Accordingly, the HVR and FR sequences generally appear in the following sequence in VH (or VL): FR1-H1(L1)-FR2-H2(L2)-FR3-H3(L3)-FR4.

An "acceptor human framework" for the purposes herein is a framework comprising the amino acid sequence of a light chain variable domain (VL) framework or a heavy chain variable domain (VH) framework derived from a human immunoglobulin framework or a human consensus framework, as defined below. An acceptor human framework "derived from" a human immunoglobulin framework or a human consensus framework may comprise the same amino acid sequence thereof, or it may contain amino acid sequence changes. In some embodiments, the number of amino acid changes are 10 or less, 9 or less, 8 or less, 7 or less, 6 or less, 5 or less, 4 or less, 3 or less, or 2 or less. In some embodiments, the VL acceptor human framework is identical in sequence to the VL human immunoglobulin framework sequence or human consensus framework sequence.

A "human consensus framework" is a framework which represents the most commonly occurring amino acid residue in a selection of human immunoglobulin VL or VH framework sequences. Generally, the selection of human immunoglobulin VL or VH sequences is from a subgroup of variable domain sequences. Generally, the subgroup of sequences is a subgroup as in Kabat et al. Sequences of Proteins of Immunological Interest, Fifth Edition, NIH Publication 91-3242, Bethesda MD (1991), vols. 1-3. *et al*. *et al.*

The term "Amyloid-Related Imaging Abnormality - Edema" or "ARIA-E" encompasses cerebral vasogenic edema and sulcal effusion.

The term "Amyloid-Related Imaging Abnormality - Hemorrhage" or "ARIA-H" encompasses microhemorrhage and superficial siderosis of the central nervous system.

"Apolipoprotein E4 carrier" or "ApoE4 carrier," used interchangeably herein with "apolipoprotein E4 positive" or "ApoE4 positive," refers to an individual having at least one apolipoprotein E4 (or "ApoE4") allele. An individual with zero ApoE4 alleles is referred to herein as being "ApoE4 negative" or an "ApoE4 non-carrier." *See also* Prekumar, et al., 1996, Am. J Pathol. 148:2083-95.

The term "cerebral vasogenic edema" refers to an excess accumulation of intravascular fluid or protein in the intracellular or extracellular spaces of the brain. Cerebral vasogenic edema is detectable by, *e.g.,* brain MRI, including, but not limited to FLAIR MRI, and can be asymptomatic ("asymptomatic vasogenic edema") or associated with neurological symptoms, such as confusion, dizziness, vomiting, and lethargy ("symptomatic vasogenic edema") (*see* Sperling et al. Alzheimer's & Dementia, 7:367, 2011).

The term "cerebral macrohemorrhage" refers to an intracranial hemorrhage, or bleeding in the brain, of an area that is more than about 1 cm in diameter. Cerebral macrohemorrhage is detectable by, *e.g.,* brain MRI, including but not limited to T2*-weighted GRE MRI, and can be asymptomatic ("asymptomatic macrohemorrhage") or associated with symptoms such as transient or permanent focal motor or sensory impairment, ataxia, aphasia, and dysarthria ("symptomatic macrohemorrhage") (see, *e.g.,* Chalela JA, Gomes J. Expert Rev. Neurother. 2004 4:267, 2004 and Sperling et al. Alzheimer's & Dementia, 7:367, 2011).

The term "cerebral microhemorrhage" refers to an intracranial hemorrhage, or bleeding in the brain, of an area that is less than about 1 cm in diameter. Cerebral microhemorrhage is detectable by, *e.g.,* brain MRI, including, but not limited to T2*-weighted GRE MRI, and can be asymptomatic ("asymptomatic microhemorrhage") or can potentially be associated with symptoms such as transient or permanent focal motor or sensory impairment, ataxia, aphasia, and dysarthria ("symptomatic microhemorrhage"). *See, e.g.,* Greenberg, et al., 2009, Lancet Neurol. 8:165-74.

The term "sulcal effusion" refers to an effusion of fluid in the furrows, or sulci, of the brain. Sulcal effusions are detectable by, *e.g.,* brain MRI, including but not limited to FLAIR MRI. *See* Sperling et al. Alzheimer's & Dementia, 7:367, 2011.

The term "superficial siderosis of the central nervous system" refers to bleeding or hemorrhage into the subarachnoid space of the brain and is detectable by, *e.g.,* brain MRI, including but not limited to T2*-weighted GRE MRI. Symptoms indicative of superficial siderosis of the central nervous system include sensorineural deafness, cerebellar ataxia, and pyramidal signs. *See* Kumara-N, Am J Neuroradiol. 31:5, 2010.

The term "progression" as used herein refers to the worsening of a disease over time. The "progression rate" or "rate of progression" of a disease refers to how fast or slow a disease develops over time in a patient diagnosed with the disease. The progression rate of a disease can be represented by measurable changes over time of particular characteristics of the disease. A patient carrying particular genetic trait is said to have, or more likely to have, "increased progression rate" if her disease state progresses faster than those patients without such genetic trait. On the other hand, a patient responding to a therapy is said to have, or more likely to have, "decreased progression rate" if her disease progression slows down after the therapy, when compared to her disease state prior to the treatment or to other patients without the treatment.

"More likely to respond" as used herein refers to patients that are most likely to demonstrate a slowing down or prevention of progression of amyloidosis, e.g., AD. With regard to AD, "more likely to respond" refers to patients that are most likely to demonstrate a reduction in loss of function or cognition with treatment. The phrase "responsive to" in the context of the present invention indicates that a patient suffering from, being suspected to suffer or being prone to suffer from, or diagnosed with a disorder as described herein, shows a response to anti-Abeta treatment.

The phrase "selecting a patient" or "identifying a patient" as used herein refers to using the information or data generated relating to the presence of an allele in a sample of a patient to identify or select the patient as more likely to benefit to benefit from a treatment comprising anti-Abeta antibody. The information or data used or generated may be in any form, written, oral or electronic. In some embodiments, using the information or data generated includes communicating, presenting, reporting, storing, sending, transferring, supplying, transmitting, dispensing, or combinations thereof. In some embodiments, communicating, presenting, reporting, storing, sending, transferring, supplying, transmitting, dispensing, or combinations thereof are performed by a computing device, analyzer unit or combination thereof. In some further embodiments, communicating, presenting, reporting, storing, sending, transferring, supplying, transmitting, dispensing, or combinations thereof are performed by a laboratory or medical professional. In some embodiments, the information or data includes an indication that a specific allele is present or absent in the sample. In some embodiments, the information or data includes an indication that the patient is more likely to respond to a therapy comprising anti-Abeta.

"Effector functions" refer to those biological activities attributable to the Fc region of an antibody, which vary with the antibody isotype. Examples of antibody effector functions include: C1q binding and complement dependent cytotoxicity (CDC); Fc receptor binding; antibody-dependent cell-mediated cytotoxicity (ADCC); phagocytosis; down regulation of cell surface receptors *(e.g.* B cell receptor); and B cell activation. It is known in the art that wild-type IgG4 antibodies have less effector function than wild-type IgG1 antibodies.

The term "Fc region" herein is used to define a C-terminal region of an immunoglobulin heavy chain that contains at least a portion of the constant region. The term includes native sequence Fc regions and variant Fc regions. In one embodiment, a human IgG heavy chain Fc region extends from Cys226, or from Pro230, to the carboxyl-terminus of the heavy chain. However, the C-terminal lysine (Lys447) of the Fc region may or may not be present. Unless otherwise specified herein, numbering of amino acid residues in the Fc region or constant region is according to the EU numbering system, also called the EU index, as described in Kabat et al., Sequences of Proteins of Immunological Interest, 5th Ed. Public Health Service, National Institutes of Health, Bethesda, MD, 1991.

The terms "full length antibody," "intact antibody," and "whole antibody" are used herein interchangeably to refer to an antibody having a structure substantially similar to a native antibody structure or having heavy chains that contain an Fc region as defined herein.

The terms "host cell," "host cell line," and "host cell culture" are used interchangeably and refer to cells into which exogenous nucleic acid has been introduced, including the progeny of such cells. Host cells include "transformants" and "transformed cells," which include the primary transformed cell and progeny derived therefrom without regard to the number of passages. Progeny may not be completely identical in nucleic acid content to a parent cell, but may contain mutations. Mutant progeny that have the same function or biological activity as screened or selected for in the originally transformed cell are included herein.

An "immunoconjugate" is an antibody conjugated to one or more heterologous molecule(s), including but not limited to a further therapeutic agent.

An "isolated" nucleic acid refers to a nucleic acid molecule that has been separated from a component of its natural environment. An isolated nucleic acid includes a nucleic acid molecule contained in cells that ordinarily contain the nucleic acid molecule, but the nucleic acid molecule is present extrachromosomally or at a chromosomal location that is different from its natural chromosomal location.

"Isolated nucleic acid encoding an anti-Abeta antibody" refers to one or more nucleic acid molecules encoding antibody heavy and light chains (or fragments thereof), including such nucleic acid molecule(s) in a single vector or separate vectors, and such nucleic acid molecule(s) present at one or more locations in a host cell.

The term "early Alzheimer's Disease" or "early AD" as used herein (e.g., a "patient diagnosed with early AD" or a "patient suffering from early AD") includes patients with mild cognitive impairement, such as a memory deficit, due to AD and patients having AD biomarkers, for example amyloid positive patients.

The term "mild Alzheimer's Disease" or "mild AD" as used herein *(e.g.,* a "patient diagnosed with mild AD") refers to a stage of AD characterized by an MMSE score of 20 to 26.

The term "mild to moderate Alzheimer's Disease" or "mild to moderate AD" as used herein encompasses both mild and moderate AD, and is characterized by an MMSE score of 18 to 26.

The term "moderate Alzheimer's Disease" or "moderate AD" as used herein (*e.g.,* a "patient diagnosed with moderate AD") refers to a stage of AD characterized by an MMSE score of 18 to 19.

A "naked antibody" refers to an antibody that is not conjugated to a heterologous moiety (*e.g.,* a further therapeuticmoiety) or radiolabel. The naked antibody may be present in a pharmaceutical formulation.

"Native antibodies" refer to naturally occurring immunoglobulin molecules with varying structures. For example, native IgG antibodies are heterotetrameric glycoproteins of about 150,000 daltons, composed of two identical light chains and two identical heavy chains that are disulfide-bonded. From N- to C-terminus, each heavy chain has a variable region (VH), also called a variable heavy domain or a heavy chain variable domain, followed by three constant domains (CHI, CH2, and CH3). Similarly, from N- to C-terminus, each light chain has a variable region (VL), also called a variable light domain or a light chain variable domain, followed by a constant light (CL) domain. The light chain of an antibody may be assigned to one of two types, called kappa (κ) and lambda (λ), based on the amino acid sequence of its constant domain.

The term "package insert" is used to refer to instructions customarily included in commercial packages of therapeutic products, that contain information about the indications, usage, dosage, administration, combination therapy, contraindications and/or warnings concerning the use of such therapeutic products. The term "package insert" is also used to refer to instructions customarily included in commercial packages of diagnostic products that contain information about the intended use, test principle, preparation and handling of reagents, specimen collection and preparation, calibration of the assay and the assay procedure, performance and precision data such as sensitivity and specificity of the assay.

"Percent (%) amino acid sequence identity" with respect to a reference polypeptide sequence is defined as the percentage of amino acid residues in a candidate sequence that are identical with the amino acid residues in the reference polypeptide sequence, after aligning the sequences and introducing gaps, if necessary, to achieve the maximum percent sequence identity, and not considering any conservative substitutions as part of the sequence identity. Alignment for purposes of determining percent amino acid sequence identity can be achieved in various ways that are within the skill in the art, for instance, using publicly available computer software such as BLAST, BLAST-2, ALIGN or Megalign (DNASTAR) software. Those skilled in the art can determine appropriate parameters for aligning sequences, including any algorithms needed to achieve maximal alignment over the full length of the sequences being compared. For purposes herein, however, % amino acid sequence identity values are generated using the sequence comparison computer program ALIGN-2. The ALIGN-2 sequence comparison computer program was authored by Genentech, Inc., and the source code has been filed with user documentation in the U.S. Copyright Office, Washington D.C., 20559, where it is registered under U.S. Copyright Registration No. TXU510087. The ALIGN-2 program is publicly available from Genentech, Inc., South San Francisco, California, or may be compiled from the source code. The ALIGN-2 program should be compiled for use on a UNIX operating system, including digital UNIX V4.0D. All sequence comparison parameters are set by the ALIGN-2 program and do not vary.

In situations where ALIGN-2 is employed for amino acid sequence comparisons, the % amino acid sequence identity of a given amino acid sequence A to, with, or against a given amino acid sequence B (which can alternatively be phrased as a given amino acid sequence A that has or comprises a certain % amino acid sequence identity to, with, or against a given amino acid sequence B) is calculated as follows:
100 times the fraction (X/Y)
where X is the number of amino acid residues scored as identical matches by the sequence alignment program ALIGN-2 in that program's alignment of A and B, and where Y is the total number of amino acid residues in B. It will be appreciated that where the length of amino acid sequence A is not equal to the length of amino acid sequence B, the % amino acid sequence identity of A to B will not equal the % amino acid sequence identity of B to A. Unless specifically stated otherwise, all % amino acid sequence identity values used herein are obtained as described in the immediately preceding paragraph using the ALIGN-2 computer program.

The terms "pharmaceutical formulation" and "pharmaceutical composition" are used interchangeably herein and refer to a preparation which is in such form as to permit the biological activity of an active ingredient contained therein to be effective, and which contains no additional components which are unacceptably toxic to a subject to which the formulation would be administered.

A "pharmaceutically acceptable carrier" refers to an ingredient in a pharmaceutical formulation, other than an active ingredient, which is nontoxic to a subject. A pharmaceutically acceptable carrier includes, but is not limited to, a buffer, excipient, stabilizer, or preservative.

The term "vector," as used herein, refers to a nucleic acid molecule capable of propagating another nucleic acid to which it is linked. The term includes the vector as a self-replicating nucleic acid structure as well as the vector incorporated into the genome of a host cell into which it has been introduced. Certain vectors are capable of directing the expression of nucleic acids to which they are operatively linked. Such vectors are referred to herein as "expression vectors."

An "imaging agent" is a compound that has one or more properties that permit its presence and/or location to be detected directly or indirectly. Examples of such imaging agents include proteins and small molecule compounds incorporating a labeled moiety that permits detection.

A "label" is a marker coupled with a molecule to be used for detection or imaging. Examples of such labels include: a radiolabel, a fluorophore, a chromophore, or an affinity tag. In one embodiment, the label is a radiolabel used for medical imaging, for example tc99m or 1123, or a spin label for nuclear magnetic resonance (NMR) imaging (also known as magnetic resonance imaging, mri), such as iodine-123 again, iodine-131, indium-111, fluorine-19, carbon-13, nitrogen-15, oxygen-17, gadolinium, manganese, iron, etc.

### METHODS AND COMPOSITIONS

The present disclosure provides compositions and methods for the treatment, prognosis, selection and/or identification of patients at risk for or having amyloidosis. In one aspect, the invention is based, in part, on improved methods of treatment.

In the present application, antibodies that bind to Abeta are provided. Antibodies as described herein are useful, *e.g.,* for the diagnosis or treatment of Alzheimer's Disease ("AD") and other diseases.

### Exemplary Antibodies

The present application describes isolated antibodies that bind to Abeta. In certain embodiments, the application describes an anti-Abeta antibody that can bind to monomeric, oligomeric and fibril forms of human Abeta with good affinity. In one embodiment, the anti-Abeta antibody is an antibody that binds to an epitope of Abeta within residues 13-24 of Abeta. In one such embodiment, the antibody is crenezumab.

In one embodiment, the antibody comprises the heavy chain amino acid sequence set forth in SEQ ID NO:5 and the light chain amino acid sequence set forth in SEQ ID NO:9. In another embodiment, the antibody comprises the heavy chain variable region of amino acids 1 to 112 of the amino acid sequence set forth in SEQ ID NO:5 and the light chain variable region of amino acids 1 to 112 of the amino acid sequence set forth in SEQ ID NO:9. In another embodiment, the antibody comprises the HVR sequences of SEQ ID NO:5 and SEQ ID NO:9. In another embodiment, the antibody comprises HVR sequences that are 95%, 96%, 97%, 98%, or 99% or more identical to the HVR sequences of SEQ ID NO:5 and SEQ ID NO:9.

In any of the above embodiments, an anti-Abeta antibody is humanized. In one embodiment, an anti-Abeta antibody comprises HVRs as in any of the above embodiments, and further comprises an acceptor human framework, *e.g*. a human immunoglobulin framework or a human consensus framework.

In another aspect, an anti-Abeta antibody comprises a heavy chain variable domain (VH) sequence having at least 90%, 91%, 92%, 93%, 94%, 95%, 96%, 97%, 98%, 99%, or 100% sequence identity to amino acids 1 to 112 of the amino acid sequence of SEQ ID NO:5. In certain embodiments, a VH sequence having at least 90%, 91%, 92%, 93%, 94%, 95%, 96%, 97%, 98%, or 99% identity contains substitutions (*e.g*., conservative substitutions), insertions, or deletions relative to the reference sequence, but an anti-Abeta antibody comprising that sequence retains the ability to bind to Abeta. In certain embodiments, a total of 1 to 10 amino acids have been substituted, inserted and/or deleted in SEQ ID NO:5. In certain embodiments, substitutions, insertions, or deletions occur in regions outside the HVRs (*i*.*e*., in the FRs). Optionally, the anti-Abeta antibody comprises the VH sequence in SEQ ID NO:5, including post-translational modifications of that sequence.

In another aspect, an anti-Abeta antibody is provided, wherein the antibody comprises a light chain variable domain (VL) having at least 90%, 91%, 92%, 93%, 94%, 95%, 96%, 97%, 98%, 99%, or 100% sequence identity to amino acids 1 to 112 of the amino acid sequence of SEQ ID NO:9. In certain embodiments, a VL sequence having at least 90%, 91%, 92%, 93%, 94%, 95%, 96%, 97%, 98%, or 99% identity contains substitutions (e.g., conservative substitutions), insertions, or deletions relative to the reference sequence, but an anti-Abeta antibody comprising that sequence retains the ability to bind to Abeta. In certain embodiments, a total of 1 to 10 amino acids have been substituted, inserted and/or deleted in SEQ ID NO:9. In certain embodiments, the substitutions, insertions, or deletions occur in regions outside the HVRs (*i*.*e*., in the FRs). Optionally, the anti-Abeta antibody comprises the VL sequence in SEQ ID NO:9, including post-translational modifications of that sequence.

In another aspect, an anti-Abeta antibody is provided, wherein the antibody comprises a VH as in any of the embodiments provided above, and a VL as in any of the embodiments provided above.

In a further aspect, the application describes an antibody that binds to the same epitope as an anti-Abeta antibody provided herein. For example, in certain embodiments, an antibody is provided that binds to the same epitope as an anti-Abeta antibody comprising a VH sequence in SEQ ID NO:5 and a VL sequence in SEQ ID NO:9.

In a further aspect as described in the application, an anti-Abeta antibody according to any of the above embodiments is a monoclonal antibody, including a chimeric, humanized or human antibody. In one embodiment, an anti-Abeta antibody is an antibody fragment, *e.g*., a Fv, Fab, Fab', scFv, diabody, or F(ab')2 fragment. In another embodiment, the antibody is a full length antibody, e.g., an intact IgG4 antibody or other antibody class or isotype as defined herein. In another embodiment, the antibody is a bispecific antibody.

In a further aspect, an anti-Abeta antibody according to any of the above embodiments may incorporate any of the features, singly or in combination, as described in Sections 1-7 below.

In one embodiment, the anti-Abeta antibody comprises a HVR-L1 comprising amino acid sequence SEQ ID NO:6; an HVR-L2 comprising amino acid sequence SEQ ID NO:7; an HVR-L3 comprising amino acid sequence SEQ ID NO: 8; an HVR-H1 comprising amino acid sequence SEQ ID NO:2; an HVR-H2 comprising amino acid sequence SEQ ID NO: 3; and an HVR-H3 comprising amino acid sequence SEQ ID NO: 4.

In another embodiment, the antibody comprises the heavy and light sequences SEQ ID NO:5 and SEQ ID NO:9.

In another embodiment, the antibody comprises the variable region sequences in SEQ ID NO:5 and SEQ ID NO:9.

In any of the above embodiments, an anti-Abeta antibody can be humanized. In one embodiment, an anti-Abeta antibody comprises HVRs as in any of the above embodiments, and further comprises an acceptor human framework, *e.g*. a human immunoglobulin framework or a human consensus framework.

### 1. Antibody Affinity

In certain embodiments, an antibody provided herein has a dissociation constant (Kd) of ≤ 1µM, ≤ 100 nM, ≤ 10 nM, ≤ 1 nM, ≤ 0.1 nM, ≤ 0.01 nM, or ≤ 0.001 nM *(e.g.* 10⁻⁸ M or less, *e.g.* from 10⁻⁸ M to 10⁻¹³ M, *e.g.,* from 10⁻⁹ M to 10⁻¹³ M).

In one embodiment, Kd is measured by a radiolabeled antigen binding assay (RIA) performed with the Fab version of an antibody of interest and its antigen as described by the following assay. Solution binding affinity of Fabs for antigen is measured by equilibrating Fab with a minimal concentration of (¹²⁵I)-labeled antigen in the presence of a titration series of unlabeled antigen, then capturing bound antigen with an anti-Fab antibody-coated plate (*see*, *e.g*., Chen et al.et al., J. Mol. Biol. 293:865-881(1999)). To establish conditions for the assay, MICROTITER® multi-well plates (Thermo Scientific) are coated overnight with 5 µg/ml of a capturing anti-Fab antibody (Cappel Labs) in 50 mM sodium carbonate (pH 9.6), and subsequently blocked with 2% (w/v) bovine serum albumin in PBS for two to five hours at room temperature (approximately 23°C). In a non-adsorbent plate (Nunc #269620), 100 pM or 26 pM [125I]-antigen are mixed with serial dilutions of a Fab of interest (*e*.*g*., consistent with assessment of the anti-VEGF antibody, Fab-12, in Presta et al., Cancer Res. 57:4593-4599 (1997)). The Fab of interest is then incubated overnight; however, the incubation may continue for a longer period (*e*.*g*., about 65 hours) to ensure that equilibrium is reached. Thereafter, the mixtures are transferred to the capture plate for incubation at room temperature (*e*.*g*., for one hour). The solution is then removed and the plate washed eight times with 0.1% polysorbate 20 (TWEEN-20®) in PBS. When the plates have dried, 150 µl/well of scintillant (MICROSCINT-20 TM; Packard) is added, and the plates are counted on a TOPCOUNT TM gamma counter (Packard) for ten minutes. Concentrations of each Fab that give less than or equal to 20% of maximal binding are chosen for use in competitive binding assays.

According to another embodiment, Kd is measured using surface plasmon resonance assays using a BIACORE®-2000 or a BIACORE ®-3000 (BIAcore, Inc., Piscataway, NJ) at 25°C with immobilized antigen CM5 chips at ∼10 response units (RU). Briefly, carboxymethylated dextran biosensor chips (CM5, BIACORE, Inc.) are activated with N-ethyl-N'- (3-dimethylaminopropyl)-carbodiimide hydrochloride (EDC) and N-hydroxysuccinimide (NHS) according to the supplier's instructions. Antigen is diluted with 10 mM sodium acetate, pH 4.8, to 5 µg/ml (∼0.2 µM) before injection at a flow rate of 5 µl/minute to achieve approximately 10 response units (RU) of coupled protein. Following the injection of antigen, 1 M ethanolamine is injected to block unreacted groups. For kinetics measurements, two-fold serial dilutions of Fab (0.78 nM to 500 nM) are injected in PBS with 0.05% polysorbate 20 (TWEEN-20TM) surfactant (PBST) at 25°C at a flow rate of approximately 25 µl/min. Association rates (kon) and dissociation rates (koff) are calculated using a simple one-to-one Langmuir binding model (BIACORE ® Evaluation Software version 3.2) by simultaneously fitting the association and dissociation sensorgrams. The equilibrium dissociation constant (Kd) is calculated as the ratio koff/kon. See, *e.g.,* Chen et al., J. Mol. Biol. 293:865-881 (1999). If the on-rate exceeds 10⁶ M⁻¹ s⁻¹ by the surface plasmon resonance assay above, then the on-rate can be determined by using a fluorescent quenching technique that measures the increase or decrease in fluorescence emission intensity (excitation = 295 nm; emission = 340 nm, 16 nm band-pass) at 25°C of a 20 nM antigen antibody (Fab form) in PBS, pH 7.2, in the presence of increasing concentrations of antigen as measured in a spectrometer, such as a stop-flow equipped spectrophometer (Aviv Instruments) or a 8000-series SLM-AMINCO TM spectrophotometer (ThermoSpectronic) with a stirred cuvette.

### 2. Antibody Fragments

In certain embodiments, an antibody provided herein is an antibody fragment. Antibody fragments include, but are not limited to, Fab, Fab', Fab'-SH, F(ab')2, Fv, and scFv fragments, and other fragments described below. For a review of certain antibody fragments, see Hudson et al. Nat. Med. 9:129-134 (2003). For a review of scFv fragments, see, *e.g.,* Pluckthun, in The Pharmacology of Monoclonal Antibodies, vol. 113, Rosenburg and Moore eds., (Springer-Verlag, New York), pp. 269-315 (1994); see also WO 93/16185; and U.S. Patent Nos. 5,571,894 and 5,587,458. For discussion of Fab and F(ab')2 fragments comprising salvage receptor binding epitope residues and having increased in vivo half-life, see U.S. Patent No. 5,869,046.

Diabodies are antibody fragments with two antigen-binding sites that may be bivalent or bispecific. See, for example, EP 404,097; WO 1993/01161; Hudson et al., Nat. Med. 9:129-134 (2003); and Hollinger et al., Proc. Natl. Acad. Sci. USA 90: 6444-6448 (1993). Triabodies and tetrabodies are also described in Hudson et al., Nat. Med. 9:129-134 (2003).

Single-domain antibodies are antibody fragments comprising all or a portion of the heavy chain variable domain or all or a portion of the light chain variable domain of an antibody. In certain embodiments, a single-domain antibody is a human single-domain antibody (Domantis, Inc., Waltham, MA; see, *e.g.,* U.S. Patent No. 6,248,516 B1). In certain embodiments, two or more single-domain antibodies may be joined together to form an immunoglobulin construct with multivalent affinity (*i*.*e*., the N- or C-terminus of a first single-domain antibody may be fused or otherwise joined to the N- or C-terminus of a second single-domain antibody).

Antibody fragments can be made by various techniques, including but not limited to proteolytic digestion of an intact antibody as well as production by recombinant host cells (*e.g. E. coli* or phage), as described herein.

### 3. Chimeric and Humanized Antibodies

In certain embodiments, an antibody provided herein is a chimeric antibody. Certain chimeric antibodies are described, *e.g.,* in U.S. Patent No. 4,816,567; and Morrison et al., Proc. Natl. Acad. Sci. USA, 81:6851-6855 (1984)). In one example, a chimeric antibody comprises a non-human variable region (*e.g.,* a variable region derived from a mouse, rat, hamster, rabbit, or non-human primate, such as a monkey) and a human constant region. In a further example, a chimeric antibody is a "class switched" antibody in which the class or subclass has been changed from that of the parent antibody. Chimeric antibodies include antigen-binding fragments thereof.

In certain embodiments, a chimeric antibody is a humanized antibody. Typically, a non-human antibody is humanized to reduce immunogenicity to humans, while retaining the specificity and affinity of the parental non-human antibody. Generally, a humanized antibody comprises one or more variable domains in which HVRs, e.g., CDRs, (or portions thereof) are derived from a non-human antibody, and FRs (or portions thereof) are derived from human antibody sequences. A humanized antibody optionally will also comprise at least a portion of a human constant region. In some embodiments, some FR residues in a humanized antibody are substituted with corresponding residues from a non-human antibody *(e.g.,* the antibody from which the HVR residues are derived), *e.g.,* to restore or improve antibody specificity or affinity.

Humanized antibodies and methods of making them are reviewed, e.g., in Almagro and Fransson, Front. Biosci. 13:1619-1633 (2008), and are further described, e.g., in Riechmann et al., Nature 332:323-329 (1988); Queen et al., Proc. Nat'l Acad. Sci. USA 86:10029-10033 (1989); US Patent Nos. 5, 821,337, 7,527,791, 6,982,321, and 7,087,409; Kashmiri et al., Methods 36:25-34 (2005) (describing SDR (a-CDR) grafting); Padlan, Mol. Immunol. 28:489-498 (1991) (describing "resurfacing"); Dall'Acqua et al., Methods 36:43-60 (2005) (describing "FR shuffling"); and Osbourn et al., Methods 36:61-68 (2005) and Klimka et al., Br. J. Cancer, 83:252-260 (2000) (describing the "guided selection" approach to FR shuffling).

Human framework regions that may be used for humanization include but are not limited to: framework regions selected using the "best-fit" method (see, *e.g.,* Sims .et al. J. Immunol. 151:2296 (1993)); framework regions derived from the consensus sequence of human antibodies of a particular subgroup of light or heavy chain variable regions (see, *e.g.,* Carter et al. Proc. Natl. Acad. Sci. USA, 89:4285 (1992); and Presta et al. J. Immunol., 151:2623 (1993)); human mature (somatically mutated) framework regions or human germline framework regions (see, *e.g*., Almagro and Fransson, Front. Biosci. 13:1619-1633 (2008)); and framework regions derived from screening FR libraries (see, *e.g.,* Baca et al., J. Biol. Chem. 272:10678-10684 (1997) and Rosok et al., J. Biol. Chem. 271:22611-22618 (1996)).

### 4. Human Antibodies

In certain embodiments, an antibody provided herein is a human antibody. Human antibodies can be produced using various techniques known in the art. Human antibodies are described generally in van Dijk and van de Winkel, Curr. Opin. Pharmacol. 5: 368-74 (2001) and Lonberg, Curr. Opin. Immunol. 20:450-459 (2008).

Human antibodies may be prepared by administering an immunogen to a transgenic animal that has been modified to produce intact human antibodies or intact antibodies with human variable regions in response to antigenic challenge. Such animals typically contain all or a portion of the human immunoglobulin loci, which replace the endogenous immunoglobulin loci, or which are present extrachromosomally or integrated randomly into the animal's chromosomes. In such transgenic mice, the endogenous immunoglobulin loci have generally been inactivated. For review of methods for obtaining human antibodies from transgenic animals, see Lonberg, Nat. Biotech. 23:1117-1125 (2005). *See also, e.g.,* U.S. Patent Nos. 6,075,181 and 6,150,584 describing XENOMOUSE™ technology; U.S. Patent No. 5,770,429 describing HUMAB® technology; U.S. Patent No. 7,041,870 describing K-M MOUSE® technology, and U.S. Patent Application Publication No. US 2007/0061900, describing VELOCIMOUSE® technology). Human variable regions from intact antibodies generated by such animals may be further modified, *e.g.,* by combining with a different human constant region.

Human antibodies can also be made by hybridoma-based methods. Human myeloma and mouse-human heteromyeloma cell lines for the production of human monoclonal antibodies have been described. (See, *e.g*., Kozbor J. Immunol., 133: 3001 (1984); Brodeur et al., Monoclonal Antibody Production Techniques and Applications, pp. 51-63 (Marcel Dekker, Inc., New York, 1987); and Boerner et al., J. Immunol., 147: 86 (1991).) Human antibodies generated via human B-cell hybridoma technology are also described in Li et al., Proc. Natl. Acad. Sci. USA, 103:3557-3562 (2006). Additional methods include those described, for example, in U.S. Patent No. 7,189,826 (describing production of monoclonal human IgM antibodies from hybridoma cell lines) and Ni, Xiandai Mianyixue, 26(4):265-268 (2006) (describing human-human hybridomas). Human hybridoma technology (Trioma technology) is also described in Vollmers and Brandlein, Histology and Histopathology, 20(3):927-937 (2005) and Vollmers and Brandlein, Methods and Findings in Experimental and Clinical Pharmacology, 27(3):185-91 (2005).

Human antibodies may also be generated by isolating Fv clone variable domain sequences selected from human-derived phage display libraries. Such variable domain sequences may then be combined with a desired human constant domain. Techniques for selecting human antibodies from antibody libraries are described below.

### 5. Library-Derived Antibodies

Antibodies as deribed herein may be isolated by screening combinatorial libraries for antibodies with the desired activity or activities. For example, a variety of methods are known in the art for generating phage display libraries and screening such libraries for antibodies possessing the desired binding characteristics. Such methods are reviewed, e.g., in Hoogenboom et al. in Methods in Molecular Biology 178:1-37 (O'Brien et al., ed., Human Press, Totowa, NJ, 2001) and further described, *e.g.,* in the McCafferty et al., Nature 348:552-554; Clackson et al., Nature 352: 624-628 (1991); Marks et al., J. Mol. Biol. 222: 581-597 (1992); Marks and Bradbury, in Methods in Molecular Biology 248:161-175 (Lo, ed., Human Press, Totowa, NJ, 2003); Sidhu et al., J. Mol. Biol. 338(2): 299-310 (2004); Lee et al., J. Mol. Biol. 340(5): 1073-1093 (2004); Fellouse, Proc. Natl. Acad. Sci. USA 101(34): 12467-12472 (2004); and Lee et al., J. Immunol. Methods 284(1-2): 119-132(2004).

In certain phage display methods, repertoires of VH and VL genes are separately cloned by polymerase chain reaction (PCR) and recombined randomly in phage libraries, which can then be screened for antigen-binding phage as described in Winter et al., Ann. Rev. Immunol., 12: 433-455 (1994). Phage typically display antibody fragments, either as single-chain Fv (scFv) fragments or as Fab fragments. Libraries from immunized sources provide high-affinity antibodies to the immunogen without the requirement of constructing hybridomas. Alternatively, the naive repertoire can be cloned *(e.g.,* from human) to provide a single source of antibodies to a wide range of non-self and also self antigens without any immunization as described by Griffiths et al., EMBO J, 12: 725-734 (1993). Finally, naive libraries can also be made synthetically by cloning unrearranged V-gene segments from stem cells, and using PCR primers containing random sequence to encode the highly variable CDR3 regions and to accomplish rearrangement *in vitro,* as described by Hoogenboom and Winter, J. Mol. Biol., 227: 381-388 (1992). Patent publications describing human antibody phage libraries include, for example: US Patent No. 5,750,373, and US Patent Publication Nos. 2005/0079574, 2005/0119455, 2005/0266000, 2007/0117126, 2007/0160598, 2007/0237764, 2007/0292936, and 2009/0002360.

Antibodies or antibody fragments isolated from human antibody libraries are considered human antibodies or human antibody fragments herein.

### 6. Multispecific Antibodies

In certain embodiments, an antibody provided herein is a multispecific antibody, *e.g.* a bispecific antibody. Multispecific antibodies are monoclonal antibodies that have binding specificities for at least two different sites. In certain embodiments, one of the binding specificities is for Abeta and the other is for any other antigen. In certain embodiments, bispecific antibodies may bind to two different epitopes of Abeta. Bispecific antibodies may also be used to localize cytotoxic agents to cells. Bispecific antibodies can be prepared as full length antibodies or antibody fragments.

Techniques for making multispecific antibodies include, but are not limited to, recombinant co-expression of two immunoglobulin heavy chain-light chain pairs having different specificities (see Milstein and Cuello, Nature 305: 537 (1983)), WO 93/08829, and Traunecker et al., EMBO J. 10: 3655 (1991)), and "knob-in-hole" engineering (see, *e.g.,* U.S. Patent No. 5,731,168). Multi-specific antibodies may also be made by engineering electrostatic steering effects for making antibody Fc-heterodimeric molecules (WO 2009/089004A1); cross-linking two or more antibodies or fragments (see, *e.g.,* US Patent No. 4,676,980, and Brennan et al., Science, 229: 81 (1985)); using leucine zippers to produce bispecific antibodies (see, *e.g*., Kostelny et al., J. Immunol., 148(5):1547-1553 (1992)); using "diabody" technology for making bispecific antibody fragments (see, *e.g*., Hollinger et al., Proc. Natl. Acad. Sci. USA, 90:6444-6448 (1993)); and using single-chain Fv (sFv) dimers (see,e.g. Gruber et al., J. Immunol., 152:5368 (1994)); and preparing trispecific antibodies as described, *e.g*., in Tutt et al. J. Immunol. 147: 60 (1991).

Engineered antibodies with three or more functional antigen binding sites, including "Octopus antibodies," are also included herein (see, *e.g.* US 2006/0025576A1).

The antibody or fragment herein also includes a "Dual Acting FAb" or "DAF" comprising an antigen binding site that binds to Abeta as well as another, different antigen (see, US 2008/0069820, for example).

### 7. Antibody Variants

In certain embodiments, amino acid sequence variants of the antibodies provided herein are contemplated. For example, it may be desirable to improve the binding affinity and/or other biological properties of the antibody. Amino acid sequence variants of an antibody may be prepared by introducing appropriate modifications into the nucleotide sequence encoding the antibody, or by peptide synthesis. Such modifications include, for example, deletions from, and/or insertions into and/or substitutions of residues within the amino acid sequences of the antibody. Any combination of deletion, insertion, and substitution can be made to arrive at the final construct, provided that the final construct possesses the desired characteristics, *e.g*., antigen-binding.

### Substitution, Insertion, and Deletion Variants

In certain embodiments, antibody variants having one or more amino acid substitutions are provided. Sites of interest for substitutional mutagenesis include the HVRs and FRs. Conservative substitutions are shown in Table 1 under the heading of "conservative substitutions." More substantial changes are provided in Table 1 under the heading of "exemplary substitutions," and as further described below in reference to amino acid side chain classes. Amino acid substitutions may be introduced into an antibody of interest and the products screened for a desired activity, *e.g*., retained/improved antigen binding, decreased immunogenicity, or improved ADCC or CDC.

**TABLE 1**

| **Original Residue** | **Exemplary Substitutions** | **Conservative Substitutions** |
|---|---|---|
| Ala (A) | Val; Leu; Ile | Val |
| Arg (R) | Lys; Gln; Asn | Lys |
| Asn (N) | Gln; His; Asp, Lys; Arg | Gln |
| Asp (D) | Glu; Asn | Glu |
| Cys (C) | Ser; Ala | Ser |
| Gln (Q) | Asn; Glu | Asn |
| Glu (E) | Asp; Gln | Asp |
| Gly (G) | Ala | Ala |
| His (H) | Asn; Gln; Lys; Arg | Arg |
| Ile (I) | Leu; Val; Met; Ala; Phe; Norleucine | Leu |
| Leu (L) | Norleucine; Ile; Val; Met; Ala; Phe | Ile |
| Lys (K) | Arg; Gln; Asn | Arg |
| Met (M) | Leu; Phe; Ile | Leu |
| Phe (F) | Trp; Leu; Val; Ile; Ala; Tyr | Tyr |
| Pro (P) | Ala | Ala |
| Ser (S) | Thr | Thr |
| Thr (T) | Val; Ser | Ser |
| Trp (W) | Tyr; Phe | Tyr |
| Tyr (Y) | Trp; Phe; Thr; Ser | Phe |
| Val (V) | Ile; Leu; Met; Phe; Ala; Norleucine | Leu |

Amino acids may be grouped according to common side-chain properties:
(1) hydrophobic: Norleucine, Met, Ala, Val, Leu, Ile;
(2) neutral hydrophilic: Cys, Ser, Thr, Asn, Gln;
(3) acidic: Asp, Glu;
(4) basic: His, Lys, Arg;
(5) residues that influence chain orientation: Gly, Pro;
(6) aromatic: Trp, Tyr, Phe.

Non-conservative substitutions will entail exchanging a member of one of these classes for another class.

One type of substitutional variant involves substituting one or more hypervariable region residues of a parent antibody (*e.g*. a humanized or human antibody). Generally, the resulting variant(s) selected for further study will have modifications (e.g., improvements) in certain biological properties (e.g., increased affinity, reduced immunogenicity) relative to the parent antibody and/or will have substantially retained certain biological properties of the parent antibody. An exemplary substitutional variant is an affinity matured antibody. In certain embodiments, affinity matured antibodies will have nanomolar or even picomolar affinities for the target antigen. Affinity matured antibodies are produced by procedures known in the art, including, *e.g*., using phage display-based affinity maturation techniques such as those described herein. Briefly, one or more HVR residues are mutated and the variant antibodies displayed on phage and screened for a particular biological activity (*e.g*. binding affinity). Other procedures are also known. Marks et al. Bio/Technology 10:779-783 (1992) describes affinity maturation by VH and VL domain shuffling. Random mutagenesis of HVR and/or framework residues is described by: Barbas et al. Proc Nat. Acad. Sci, USA 91:3809-3813 (1994); Schier et al. Gene 169:147-155 (1996); Yelton et al. J. Immunol. 155:1994-2004 (1995); Jackson et al., J. Immunol. 154(7):3310-9 (1995); and Hawkins et al. J. Mol. Biol. 226:889-896 (1992).

Alterations *(e.g.,* substitutions) may be made in HVRs, *e.g.,* to improve antibody affinity. Such alterations may be made in HVR "hotspots," *i.e.,* residues encoded by codons that undergo mutation at high frequency during the somatic maturation process (see, *e.g.,* Chowdhury, Methods Mol. Biol. 207:179-196 (2008)), and/or SDRs (a-CDRs), with the resulting variant VH or VL being tested for binding affinity. Affinity maturation by constructing and reselecting from secondary libraries has been described, *e.g.,* in Hoogenboom et al. in Methods in Molecular Biology 178:1-37 (O'Brien et al., ed., Human Press, Totowa, NJ, (2001).) In some embodiments of affinity maturation, diversity is introduced into the variable genes chosen for maturation by any of a variety of methods (e.g., error-prone PCR, chain shuffling, or oligonucleotide-directed mutagenesis). A secondary library is then created. The library is then screened to identify any antibody variants with the desired affinity. Another method to introduce diversity involves HVR-directed approaches, in which several HVR residues (e.g., 4-6 residues at a time) are randomized. HVR residues involved in antigen binding may be specifically identified, *e.g*., using alanine scanning mutagenesis or modeling. CDR-H3 and CDR-L3 in particular are often targeted.

In certain embodiments, substitutions, insertions, or deletions may occur within one or more HVRs so long as such alterations do not substantially reduce the ability of the antibody to bind antigen. For example, conservative alterations (e.g., conservative substitutions as provided herein) that do not substantially reduce binding affinity may be made in HVRs. Such alterations may be outside of HVR "hotspots" or SDRs. In certain embodiments of the variant VH and VL sequences provided above, each HVR either is unaltered, or contains no more than one, two or three amino acid substitutions.

A useful method for identification of residues or regions of an antibody that may be targeted for mutagenesis is called "alanine scanning mutagenesis" as described by Cunningham and Wells (1989) Science, 244:1081-1085. In this method, a residue or group of target residues (*e*.*g*., charged residues such as arg, asp, his, lys, and glu) are identified and replaced by a neutral or negatively charged amino acid (*e*.*g*., alanine or polyalanine) to determine whether the interaction of the antibody with antigen is affected. Further substitutions may be introduced at the amino acid locations demonstrating functional sensitivity to the initial substitutions. Alternatively, or additionally, a crystal structure of an antigen-antibody complex to identify contact points between the antibody and antigen. Such contact residues and neighboring residues may be targeted or eliminated as candidates for substitution. Variants may be screened to determine whether they contain the desired properties.

Amino acid sequence insertions include amino- and/or carboxyl-terminal fusions ranging in length from one residue to polypeptides containing a hundred or more residues, as well as intrasequence insertions of single or multiple amino acid residues. Examples of terminal insertions include an antibody with an N-terminal methionyl residue. Other insertional variants of the antibody molecule include the fusion to the N- or C-terminus of the antibody to an enzyme *(e.g.* for ADEPT) or a polypeptide which increases the serum half-life of the antibody.

### Glvcosvlation variants

In certain embodiments, an antibody provided herein is altered to increase or decrease the extent to which the antibody is glycosylated. Addition or deletion of glycosylation sites to an antibody may be conveniently accomplished by altering the amino acid sequence such that one or more glycosylation sites is created or removed.

Where the antibody comprises an Fc region, the carbohydrate attached thereto may be altered. Native antibodies produced by mammalian cells typically comprise a branched, biantennary oligosaccharide that is generally attached by an N-linkage to Asn297 of the CH2 domain of the Fc region. See, *e.g.,* Wright et al. TIBTECH 15:26-32 (1997). The oligosaccharide may include various carbohydrates, *e.g*., mannose, N-acetyl glucosamine (GlcNAc), galactose, and sialic acid, as well as a fucose attached to a GlcNAc in the "stem" of the biantennary oligosaccharide structure. In some embodiments, modifications of the oligosaccharide in an antibody as described herein may be made in order to create antibody variants with certain improved properties.

In one embodiment, antibody variants are provided having a carbohydrate structure that lacks fucose attached (directly or indirectly) to an Fc region. For example, the amount of fucose in such antibody may be from 1% to 80%, from 1% to 65%, from 5% to 65% or from 20% to 40%. The amount of fucose is determined by calculating the average amount of fucose within the sugar chain at Asn297, relative to the sum of all glycostructures attached to Asn 297 (e. g. complex, hybrid and high mannose structures) as measured by MALDI-TOF mass spectrometry, as described in WO 2008/077546, for example. Asn297 refers to the asparagine residue located at about position 297 in the Fc region (Eu numbering of Fc region residues); however, Asn297 may also be located about ± 3 amino acids upstream or downstream of position 297, *i.e.,* between positions 294 and 300, due to minor sequence variations in antibodies. Such fucosylation variants may have improved ADCC function. See, *e.g.,* US Patent Publication Nos. US 2003/0157108 (Presta, L.); US 2004/0093621 (Kyowa Hakko Kogyo Co., Ltd). Examples of publications related to "defucosylated" or "fucose-deficient" antibody variants include: US 2003/0157108; WO 2000/61739; WO 2001/29246; US 2003/0115614; US 2002/0164328; US 2004/0093621; US 2004/0132140; US 2004/0110704; US 2004/0110282; US 2004/0109865; WO 2003/085119; WO 2003/084570; WO 2005/035586; WO 2005/035778; WO2005/053742; WO2002/031140; Okazaki et al. J. Mol. Biol. 336:1239-1249 (2004); Yamane-Ohnuki et al. Biotech. Bioeng. 87: 614 (2004). Examples of cell lines capable of producing defucosylated antibodies include Lec13 CHO cells deficient in protein fucosylation (Ripka et al. Arch. Biochem. Biophys. 249:533-545 (1986); US Pat Appl No US 2003/0157108 A1, Presta, L; and WO 2004/056312 A1, Adams *et al*., especially at Example 11), and knockout cell lines, such as alpha-1,6-fucosyltransferase gene, FUT8, knockout CHO cells (see, *e.g.,* Yamane-Ohnuki et al. Biotech. Bioeng. 87: 614 (2004); Kanda, Y. et al., Biotechnol. Bioeng., 94(4):680-688 (2006); and WO2003/085107).

Antibodies variants are further provided with bisected oligosaccharides, *e.g*., in which a biantennary oligosaccharide attached to the Fc region of the antibody is bisected by GlcNAc. Such antibody variants may have reduced fucosylation and/or improved ADCC function. Examples of such antibody variants are described, *e.g*., in WO 2003/011878 (Jean-Mairet et al.); US Patent No. 6,602,684 (Umana et al.); and US 2005/0123546 (Umana et al.). Antibody variants with at least one galactose residue in the oligosaccharide attached to the Fc region are also provided. Such antibody variants may have improved CDC function. Such antibody variants are described, *e.g.,* in WO 1997/30087 (Patel et al.); WO 1998/58964 (Raju, S.); and WO 1999/22764 (Raju, S.).

### Fc region variants

In certain embodiments, one or more amino acid modifications may be introduced into the Fc region of an antibody provided herein, thereby generating an Fc region variant. The Fc region variant may comprise a human Fc region sequence (e.g., a human IgG1, IgG2, IgG3 or IgG4 Fc region) comprising an amino acid modification (*e.g*. a substitution) at one or more amino acid positions.

In certain embodiments, the application contemplates an antibody variant that possesses some but not all effector functions, which make it a desirable candidate for applications in which the half life of the antibody in vivo is important yet certain effector functions (such as complement and ADCC) are unnecessary or deleterious. *In vitro* and/or in vivo cytotoxicity assays can be conducted to confirm the reduction/depletion of CDC and/or ADCC activities. For example, Fc receptor (FcR) binding assays can be conducted to ensure that the antibody lacks FcyR binding (hence likely lacking ADCC activity), but retains FcRn binding ability. The primary cells for mediating ADCC, NK cells, express FcλRIII only, whereas monocytes express FcλRI, FcλRII and FcλRIII. FcR expression on hematopoietic cells is summarized in Table 3 on page 464 of Ravetch and Kinet, Annu. Rev. Immunol. 9:457-492 (1991). Non-limiting examples *of in vitro* assays to assess ADCC activity of a molecule of interest is described in U.S. Patent No. 5,500,362 (see, *e.g.* Hellstrom, I. et al. Proc. Nat'l Acad. Sci. USA 83:7059-7063 (1986)) and Hellstrom, I et al., Proc. Nat'l Acad. Sci. USA 82:1499-1502 (1985); 5,821,337 (see Bruggemann, M. et al., J. Exp. Med. 166:1351-1361 (1987)). Alternatively, non-radioactive assays methods may be employed (see, for example, ACTI™ non-radioactive cytotoxicity assay for flow cytometry (CellTechnology, Inc. Mountain View, CA; and CytoTox 96® non-radioactive cytotoxicity assay (Promega, Madison, WI). Useful effector cells for such assays include peripheral blood mononuclear cells (PBMC) and Natural Killer (NK) cells. Alternatively, or additionally, ADCC activity of the molecule of interest may be assessed in vivo, *e.g.,* in a animal model such as that disclosed in Clynes et al. Proc. Nat'l Acad. Sci. USA 95:652-656 (1998). C1q binding assays may also be carried out to confirm that the antibody is unable to bind C1q and hence lacks CDC activity. See, *e.g.,* C1q and C3c binding ELISA in WO 2006/029879 and WO 2005/100402. To assess complement activation, a CDC assay may be performed (see, for example, Gazzano-Santoro et al., J. Immunol. Methods 202:163 (1996); Cragg, M.S. et al., Blood 101:1045-1052 (2003); and Cragg, M.S. and M.J. Glennie, Blood 103:2738-2743 (2004)). FcRn binding and in vivo clearance/half life determinations can also be performed using methods known in the art (see, *e.g.,* Petkova, S.B. et al., Int'l. Immunol. 18(12):1759-1769 (2006)).

Antibodies with reduced effector function include those with substitution of one or more of Fc region residues 238, 265, 269, 270, 297, 327 and 329 (U.S. Patent No. 6,737,056). Such Fc mutants include Fc mutants with substitutions at two or more of amino acid positions 265, 269, 270, 297 and 327, including the so-called "DANA" Fc mutant with substitution of residues 265 and 297 to alanine (US Patent No. 7,332,581).

Certain antibody variants with improved or diminished binding to FcRs are described. (See, *e.g*., U.S. Patent No. 6,737,056; WO 2004/056312, and Shields etal., J. Biol. Chem. 9(2): 6591-6604 (2001).)

In certain embodiments, an antibody variant comprises an Fc region with one or more amino acid substitutions which improve ADCC, *e*.*g*., substitutions at positions 298, 333, and/or 334 of the Fc region (EU numbering of residues).

In some embodiments, alterations are made in the Fc region that result in altered (*i*.*e*., either improved or diminished) C1q binding and/or Complement Dependent Cytotoxicity (CDC), *e.g.,* as described in US Patent No. 6,194,551, WO 99/51642, and Idusogie et al. J. Immunol. 164: 4178-4184 (2000).

Antibodies with increased half lives and improved binding to the neonatal Fc receptor (FcRn), which is responsible for the transfer of maternal IgGs to the fetus (Guyer et al., J. Immunol. 117:587 (1976) and Kim et al., J. Immunol. 24:249 (1994)), are described in US2005/0014934A1 (Hinton et al.). Those antibodies comprise an Fc region with one or more substitutions therein which improve binding of the Fc region to FcRn. Such Fc variants include those with substitutions at one or more of Fc region residues: 238, 256, 265, 272, 286, 303, 305, 307, 311, 312, 317, 340, 356, 360, 362, 376, 378, 380, 382, 413, 424 or 434, e.g., substitution of Fc region residue 434 (US Patent No. 7,371,826). See also Duncan & Winter, Nature 322:738-40 (1988); U.S. Patent No. 5,648,260; U.S. Patent No. 5,624,821; and WO 94/29351 concerning other examples of Fc region variants.

### Cysteine engineered antibody variants

In certain embodiments, it may be desirable to create cysteine engineered antibodies, e.g., "thioMAbs," in which one or more residues of an antibody are substituted with cysteine residues. In particular embodiments, the substituted residues occur at accessible sites of the antibody. By substituting those residues with cysteine, reactive thiol groups are thereby positioned at accessible sites of the antibody and may be used to conjugate the antibody to other moieties, such as drug moieties or linker-drug moieties, to create an immunoconjugate, as described further herein. In certain embodiments, any one or more of the following residues may be substituted with cysteine: V205 (Kabat numbering) of the light chain; A118 (EU numbering) of the heavy chain; and S400 (EU numbering) of the heavy chain Fc region. Cysteine engineered antibodies may be generated as described, *e.g*., in U.S. Patent No. 7,521,541.

### Antibody Derivatives

In certain embodiments, an antibody provided herein may be further modified to contain additional nonproteinaceous moieties that are known in the art and readily available. The moieties suitable for derivatization of the antibody include but are not limited to water soluble polymers. Non-limiting examples of water soluble polymers include, but are not limited to, polyethylene glycol (PEG), copolymers of ethylene glycol/propylene glycol, carboxymethylcellulose, dextran, polyvinyl alcohol, polyvinyl pyrrolidone, poly-1, 3-dioxolane, poly-1,3,6-trioxane, ethylene/maleic anhydride copolymer, polyaminoacids (either homopolymers or random copolymers), and dextran or poly(n-vinyl pyrrolidone)polyethylene glycol, propropylene glycol homopolymers, prolypropylene oxide/ethylene oxide co-polymers, polyoxyethylated polyols *(e.g.,* glycerol), polyvinyl alcohol, and mixtures thereof. Polyethylene glycol propionaldehyde may have advantages in manufacturing due to its stability in water. The polymer may be of any molecular weight, and may be branched or unbranched. The number of polymers attached to the antibody may vary, and if more than one polymer is attached, they can be the same or different molecules. In general, the number and/or type of polymers used for derivatization can be determined based on considerations including, but not limited to, the particular properties or functions of the antibody to be improved, whether the antibody derivative will be used in a therapy under defined conditions, etc.

In another embodiment, conjugates of an antibody and nonproteinaceous moiety that may be selectively heated by exposure to radiation are provided. In one embodiment, the nonproteinaceous moiety is a carbon nanotube (Kam et al., Proc. Natl. Acad. Sci. USA 102: 11600-11605 (2005)). The radiation may be of any wavelength, and includes, but is not limited to, wavelengths that do not harm ordinary cells, but which heat the nonproteinaceous moiety to a temperature at which cells proximal to the antibody-nonproteinaceous moiety are killed.

### Recombinant Methods and Compositions

Antibodies may be produced using recombinant methods and compositions, *e.g*., as described in U.S. Patent No. 4,816,567. In one embodiment, isolated nucleic acid encoding an anti-Abeta antibody described herein is provided. Such nucleic acid may encode an amino acid sequence comprising the VL and/or an amino acid sequence comprising the VH of the antibody *(e.g.,* the light and/or heavy chains of the antibody). In a further embodiment, one or more vectors (e.g., expression vectors) comprising such nucleic acid are provided. In a further embodiment, a host cell comprising such nucleic acid is provided. In one such embodiment, a host cell comprises (e.g., has been transformed with): (1) a vector comprising a nucleic acid that encodes an amino acid sequence comprising the VL of the antibody and an amino acid sequence comprising the VH of the antibody, or (2) a first vector comprising a nucleic acid that encodes an amino acid sequence comprising the VL of the antibody and a second vector comprising a nucleic acid that encodes an amino acid sequence comprising the VH of the antibody. In one embodiment, the host cell is eukaryotic, e.g. a Chinese Hamster Ovary (CHO) cell or lymphoid cell *(e.g.,* Y0, NS0, Sp20 cell). In one embodiment, a method of making an anti-Abeta antibody is provided, wherein the method comprises culturing a host cell comprising a nucleic acid encoding the antibody, as provided above, under conditions suitable for expression of the antibody, and optionally recovering the antibody from the host cell (or host cell culture medium).

For recombinant production of an anti-Abeta antibody, nucleic acid encoding an antibody, *e.g*., as described above, is isolated and inserted into one or more vectors for further cloning and/or expression in a host cell. Such nucleic acid may be readily isolated and sequenced using conventional procedures (e.g., by using oligonucleotide probes that are capable of binding specifically to genes encoding the heavy and light chains of the antibody).

Suitable host cells for cloning or expression of antibody-encoding vectors include prokaryotic or eukaryotic cells described herein. For example, antibodies may be produced in bacteria, in particular when glycosylation and Fc effector function are not needed. For expression of antibody fragments and polypeptides in bacteria, see, *e.g.,* U.S. Patent Nos. 5,648,237, 5,789,199, and 5,840,523. (See also Charlton, Methods in Molecular Biology, Vol. 248 (B.K.C. Lo, ed., Humana Press, Totowa, NJ, 2003), pp. 245-254, describing expression of antibody fragments in *E. coli.)* After expression, the antibody may be isolated from the bacterial cell paste in a soluble fraction and can be further purified.

In addition to prokaryotes, eukaryotic microbes such as filamentous fungi or yeast are suitable cloning or expression hosts for antibody-encoding vectors, including fungi and yeast strains whose glycosylation pathways have been "humanized," resulting in the production of an antibody with a partially or fully human glycosylation pattern. See Gerngross, Nat. Biotech. 22:1409-1414 (2004), and Li et al., Nat. Biotech. 24:210-215 (2006).

Suitable host cells for the expression of glycosylated antibody are also derived from multicellular organisms (invertebrates and vertebrates). Examples of invertebrate cells include plant and insect cells. Numerous baculoviral strains have been identified which may be used in conjunction with insect cells, particularly for transfection of Spodoptera frugiperda cells.

Plant cell cultures can also be utilized as hosts. *See*, *e.g*., US Patent Nos. 5,959,177, 6,040,498, 6,420,548, 7,125,978, and 6,417,429 (describing PLANTIBODIES™ technology for producing antibodies in transgenic plants).

Vertebrate cells may also be used as hosts. For example, mammalian cell lines that are adapted to grow in suspension may be useful. Other examples of useful mammalian host cell lines are monkey kidney CV1 line transformed by SV40 (COS-7); human embryonic kidney line (293 or 293 cells as described, *e.g.,* in Graham et al., J. Gen Virol. 36:59 (1977)); baby hamster kidney cells (BHK); mouse sertoli cells (TM4 cells as described, e.g., in Mather, Biol. Reprod. 23:243-251 (1980)); monkey kidney cells (CV1); African green monkey kidney cells (VERO-76); human cervical carcinoma cells (HELA); canine kidney cells (MDCK; buffalo rat liver cells (BRL 3A); human lung cells (W138); human liver cells (Hep G2); mouse mammary tumor (MMT 060562); TRI cells, as described, *e.g*., in Mather et al., Annals N.Y. Acad. Sci. 383:44-68 (1982); MRC 5 cells; and FS4 cells. Other useful mammalian host cell lines include Chinese hamster ovary (CHO) cells, including DHFR- CHO cells (Urlaub et al., Proc. Natl. Acad. Sci. USA 77:4216 (1980)); and myeloma cell lines such as Y0, NS0 and Sp2/0. For a review of certain mammalian host cell lines suitable for antibody production, see, e.g., Yazaki and Wu, Methods in Molecular Biology, Vol. 248 (B.K.C. Lo, ed., Humana Press, Totowa, NJ), pp. 255-268 (2003).

### Assays

Anti-Abeta antibodies provided herein may be identified, screened for, or characterized for their physical/chemical properties and/or biological activities by various assays known in the art.

### Binding assays and other assays

In one aspect, an antibody as described herein is tested for its antigen binding activity, *e*.*g*., by known methods such as ELISA, Western blot, etc.

In another aspect, competition assays may be used to identify an antibody that competes with an anti-Abeta antibody as described herein for binding to Abeta. In certain embodiments, such a competing antibody binds to the same epitope (*e*.*g*., a linear or a conformational epitope) that is bound by crenezumab or another anti-Abeta antibody specified herein. Detailed exemplary methods for mapping an epitope to which an antibody binds are provided in Morris (1996) "Epitope Mapping Protocols," in Methods in Molecular Biology vol. 66 (Humana Press, Totowa, NJ).

In an exemplary competition assay, immobilized Abeta in the desired form (*e*.*g*., monomeric, oligomeric, or fibril) is incubated in a solution comprising a first labeled antibody that binds to Abeta (e.g., crenezumab) and a second unlabeled antibody that is being tested for its ability to compete with the first antibody for binding to Abeta. The second antibody may be present in a hybridoma supernatant. As a control, immobilized Abeta is incubated in a solution comprising the first labeled antibody but not the second unlabeled antibody. After incubation under conditions permissive for binding of the first antibody to Abeta, excess unbound antibody is removed, and the amount of label associated with immobilized Abeta is measured. If the amount of label associated with immobilized Abeta is substantially reduced in the test sample relative to the control sample, then that indicates that the second antibody is competing with the first antibody for binding to Abeta. See Harlow and Lane (1988) Antibodies: A Laboratory Manual ch.14 (Cold Spring Harbor Laboratory, Cold Spring Harbor, NY).

### Activity assays

In one aspect, assays are provided for identifying anti-Abeta antibodies thereof having biological activity, for example the biological activity of crenezumab. Biological activity may include, but is not limited to, e.g., prevention of aggregation of monomeric Abeta into oligomeric Abeta, or disaggregation of oligomeric Abeta into monomeric Abeta. Antibodies having such biological activity in vivo and/or *in vitro* are also provided.

In certain embodiments, an antibody as described herein is tested for such biological activity.

### Methods and Compositions for Diagnostics and Detection

In certain embodiments, any of the anti-Abeta antibodies provided herein is useful for detecting the presence of Abeta in a biological sample. The term "detecting" as used herein encompasses quantitative or qualitative detection. In certain embodiments, a biological sample comprises a cell or tissue, such as serum, plasma, nasal swabs, sputum, cerebrospinal fluid, , aqueous humor of the eye and the like, or tissue or cell samples obtained from an organism such as samples containing neural or brain tissue.

In one embodiment, an anti-Abeta antibody for use in a method of diagnosis or detection is provided. In a further aspect, a method of detecting the presence of Abeta in a biological sample is provided. In certain embodiments, the method comprises contacting the biological sample with an anti-Abeta antibody as described herein under conditions permissive for binding of the anti-Abeta antibody to Abeta, and detecting whether a complex is formed between the anti-Abeta antibody and Abeta. Such method may be an *in vitro* or in vivo method.

Exemplary disorders that may be diagnosed using an antibody as described herein are diseases and disorders caused by or associated with amyloid or amyloid-like proteins. These include, but are not limited to, diseases and disorders caused by the presence or activity of amyloid-like proteins in monomeric, fibril, or polymeric state, or any combination of the three, including by amyloid plaques. Exemplary diseases include, but are not limited to, secondary amyloidosis and age-related amyloidosis, such as diseases including, but not limited to, neurological disorders such as Alzheimer's Disease ("AD"), diseases or conditions characterized by a loss of cognitive memory capacity such as, for example, mild cognitive impairment (MCI), Lewy body dementia, Down's syndrome, hereditary cerebral hemorrhage with amyloidosis (Dutch type), the Guam Parkinson-Demential complex and other diseases which are based on or associated with amyloid-like proteins such as progressive supranuclear palsy, multiple sclerosis, Creutzfeld Jacob disease, Parkinson's disease, HIV-related dementia, ALS (amyotropic lateral sclerosis), inclusion-body myositis (IBM), adult onset diabetes, endocrine tumor and senile cardiac amyloidosis, and various eye diseases including macular degeneration, drusen-related optic neuropathy, glaucoma, and cataract due to beta-amyloid deposition.

In certain embodiments, labeled anti-Abeta antibodies are provided. Labels include, but are not limited to, labels or moieties that are detected directly (such as fluorescent, chromophoric, electron-dense, chemiluminescent, and radioactive labels), as well as moieties, such as enzymes or ligands, that are detected indirectly, e.g., through an enzymatic reaction or molecular interaction. Exemplary labels include, but are not limited to, the radioisotopes ³²P, ¹⁴C, ^{125I}, ³H, and ¹³¹I, fluorophores such as rare earth chelates or fluorescein and its derivatives, rhodamine and its derivatives, dansyl, umbelliferone, luceriferases, e.g., firefly luciferase and bacterial luciferase (U.S. Patent No. 4,737,456), luciferin, 2,3-dihydrophthalazinediones, horseradish peroxidase (HRP), alkaline phosphatase, β-galactosidase, glucoamylase, lysozyme, saccharide oxidases, e.g., glucose oxidase, galactose oxidase, and glucose-6-phosphate dehydrogenase, heterocyclic oxidases such as uricase and xanthine oxidase, coupled with an enzyme that employs hydrogen peroxide to oxidize a dye precursor such as HRP, lactoperoxidase, or microperoxidase, biotin/avidin, spin labels, bacteriophage labels, stable free radicals, and the like.

### Pharmaceutical Formulations

Pharmaceutical formulations of an anti-Abeta antibody as described herein are prepared by mixing such antibody or molecule having the desired degree of purity with one or more optional pharmaceutically acceptable carriers (Remington's Pharmaceutical Sciences 16th edition, Osol, A. Ed. (1980)), in the form of lyophilized formulations or aqueous solutions. Pharmaceutically acceptable carriers are generally nontoxic to recipients at the dosages and concentrations employed, and include, but are not limited to: buffers such as phosphate, citrate, and other organic acids; antioxidants including ascorbic acid and methionine; preservatives (such as octadecyldimethylbenzyl ammonium chloride; hexamethonium chloride; benzalkonium chloride; benzethonium chloride; phenol, butyl or benzyl alcohol; alkyl parabens such as methyl or propyl paraben; catechol; resorcinol; cyclohexanol; 3-pentanol; and m-cresol); low molecular weight (less than about 10 residues) polypeptides; proteins, such as serum albumin, gelatin, or immunoglobulins; hydrophilic polymers such as polyvinylpyrrolidone; amino acids such as glycine, glutamine, asparagine, histidine, arginine, or lysine; monosaccharides, disaccharides, and other carbohydrates including glucose, mannose, or dextrins; chelating agents such as EDTA; sugars such as sucrose, mannitol, trehalose or sorbitol; salt-forming counter-ions such as sodium; metal complexes (*e*.*g*. Zn-protein complexes); and/or non-ionic surfactants such as polyethylene glycol (PEG). Exemplary pharmaceutically acceptable carriers herein further include insterstitial drug dispersion agents such as soluble neutral-active hyaluronidase glycoproteins (sHASEGP), for example, human soluble PH-20 hyaluronidase glycoproteins, such as rHuPH20 (HYLENEX®, Baxter International, Inc.). Certain exemplary sHASEGPs and methods of use, including rHuPH20, are described in US Patent Publication Nos. 2005/0260186 and 2006/0104968. In one aspect, a sHASEGP is combined with one or more additional glycosaminoglycanases such as chondroitinases.

In one embodiment, an antibody as described herein may be formulated in an arginine buffer. In one aspect, the arginine buffer may be an arginine succinate buffer. In one such aspect, the concentration of the arginine succinate buffer may be 50 mM or greater. In another such aspect, the concentration of the arginine succinate buffer may be 100 mM or greater. In another such aspect, the concentration of the arginine succinate buffer may be 150 mM or greater. In another such aspect, the concentration of the arginine succinate buffer may be 200 mM or greater. In another aspect, the arginine buffer formulation may further contain a surfactant. In another such aspect, the surfactant is a polysorbate. In another such aspect, the polysorbate is polysorbate 20. In another such aspect, the concentration of polysorbate 20 in the formulation is 0.1% or less. In another such aspect, the concentration of polysorbate 20 in the formulation is 0.05% or less. In another aspect, the pH of the arginine buffer formulation is between 4.5 and 7.0. In another aspect, the pH of the arginine buffer formulation is between 5.0 and 6.5. In another aspect, the pH of the arginine buffer formulation is between 5.0 and 6.0. In another aspect, the pH of the arginine buffer formulation is 5.5. In any of the foregoing embodiments and aspects, the antibody as described herein may be crenezumab.

Exemplary lyophilized antibody formulations are described in US Patent No. 6,267,958. Aqueous antibody formulations include those described in US Patent No. 6,171,586 and WO2006/044908, the latter formulations including a histidine-acetate buffer.

The formulation herein may also contain more than one active ingredients as necessary for the particular indication being treated, preferably those with complementary activities that do not adversely affect each other. For example, it may be desirable to further provide one or more compounds to prevent or treat symptoms of Alzheimer's Disease. Such active ingredients are suitably present in combination in amounts that are effective for the purpose intended.

Active ingredients may be entrapped in microcapsules prepared, for example, by coacervation techniques or by interfacial polymerization, for example, hydroxymethylcellulose or gelatin-microcapsules and poly-(methylmethacylate) microcapsules, respectively, in colloidal drug delivery systems (for example, liposomes, albumin microspheres, microemulsions, nanoparticles and nanocapsules) or in macroemulsions. Such techniques are disclosed in Remington's Pharmaceutical Sciences 16th edition, Osol, A. Ed. (1980).

Sustained-release preparations may be prepared. Suitable examples of sustained-release preparations include semipermeable matrices of solid hydrophobic polymers containing the antibody, which matrices are in the form of shaped articles, e.g. films, or microcapsules.

The formulations to be used for in vivo administration are generally sterile. Sterility may be readily accomplished, e.g., by filtration through sterile filtration membranes.

### Therapeutic Methods and Compositions

As shown herein, intravenous administration of crenezumab reduced disease progression in patients suffering from AD. Specifically, patients with mild to moderate AD, including patients with mild AD and ApoE4 positive patients, as well as patients with brain amyloid load typically seen in patients diagnosed with AD, showed a reduction in the rate of cognitive decline when treated with crenezumab as compared to a placebo. The milder the disease, based on increasing MMSE score, the greater the reduction in decline in the treatment arm as compared to the placebo arm. These results were further substantiated by other indications of target engagement by crenezumab, including an increase in the levels of Abeta detected in cerebrospinal fluid and reduction in the accumulation of amyloid in the brain. Furthermore, a comparatively high dose of antibody - 15 mg/kg - did not increase the incidence of the ARIA-type adverse events which have been observed in trials of other anti-Abeta antibodies.

Therefore, in one embodiment, an antibody as described herein is used to treat AD, including mild to moderate AD, mild AD, and early AD. In another embodiment, an antibody as described herein is used to treat an amyloidosis. In one such embodiment, the amyloidosis is mild cognitive impairment. In another such embodiment, the amyloidosis is Down's syndrome. In another such embodiment, the amyloidosis is hereditary cerebral hemorrhage with amyloidosis (Dutch type). In another such embodiment, the amyloidosis is the Guam Parkinson-Dementia complex. In another such embodiment, the amyloidosis is an ocular disease related to drusen or other amyloid deposit in the eye. In one aspect, the ocular disease is macular degeneration. In another aspect, the ocular disease is a drusen-related optic neuropathy. In another aspect, the ocular disease is glaucoma. In another aspect, the ocular disease is cataract. In any of the foregoing embodiments and aspects, the antibody as described herein may be crenezumab.

A patient is typically first assessed for the presence of one or more amyloidosis prior to determining the suitability of an antibody as described herein to treat such patient. As one nonlimiting example, AD may be diagnosed in a patient using the "NINCDS-ADRDA" (Neurological and Communicative Disorders and Stroke-Alzheimer's Disease Related Disorders Assessment) criteria. *See* McKhann, et al., 1984, Neurology 34:939-44. A potential patient to be administered one or more antibodies as described herein may also be tested for the presence or absence of one or more genetic markers which may predispose such patient either to (i) a higher or lower likelihood of such patient experiencing one or more amyloidoses, or (ii) a higher or lower likelihood of such patient experiencing one or more adverse events or side effects during the course of administration of an antibody as described herein. As one nonlimiting example, it is known that patients carrying the ApoE4 allele have a substantially higher risk of developing AD than those lacking the allele (Saunders et al., Neurology 1993; 43:1467-72; Prekumar et al., Am. J. Pathol. 1996; 148:2083-95), and that such patients were disproportionately represented in ARIA-type adverse events observed in the clinical trial of bapineuzumab, another anti-Abeta antibody (Sperling et al., Alzheimer's & Dementia 2011, 7:367-385; Salloway et al., N. Engl. J. Med. 2014, 370:322-333).

In some embodiments, the antibody as described herein is used to treat mild to moderate AD in a patient. The patient can be ApoE4 positive or ApoE4 negative. In some embodiments, the antibody as described herein is used to treat mild AD. In some embodiments, the antibody as described herein is used to treat an ApoE4 positive patient suffering from mild to moderate AD or mild AD. In some embodiments, the antibody as described herein is used to treat a patient suffering from mild AD.

In some embodiments, the antibody as described herein is used to treat a patient having an MMSE score of between 20 and 30, between 20 and 26, between 24 and 30, between 21 and 26, between 22 and 26, between 22 and 28, between 23 and 26, between 24 and 26, or between 25 and 26. In some embodiments, the patient has an MMSE score between 22 and 26. As used herein, an MMSE score between two numbers includes the numbers at each end of the range. For example, an MMSE score between 22 and 26 includes MMSE scores of 22 and 26.

In some embodiments, the antibodies as described herein are used to treat a patient who is 'amyloid positive,' e.g., a patient having brain amyloid deposits that are typical of a patient diagnosed with AD or a patient having a positive florbetapir PET scan. In some embodiments, the antibodies as described herein are used to reduce the accumulation of brain amyloid deposits or neuritic plaques (*i*.*e*., to reduce an increase in brain amyloid burden or load).

Furthermore, the antibodies as described herein are useful for treating mild to moderate AD without increasing the incidence of ARIA-E or ARIA-H. In some embodiments, the patients are suffering from mild AD. In some embodiments, the patients are ApoE4 positive. In some embodiments, the patients are ApoE4 positive and suffering from mild AD.

As evidenced in the Examples herein, the therapeutic effect is increased in patients with milder forms of AD. Consequently, in some embodiments, the antibody as described herein is used to treat a patient with early AD. In certain embodiments, the patient to be treated has one or more of the following characteristics: (a) mild cognitive impairement (MCI) due to AD; (b) one or more biomarkers indicative of Alzheimer's Disease without a clinically detectable deficit; (c) an objective memory loss quantified using the Free and Cued Selective Reminding Test (FCSRT) as a score of 27 or greater; an MMSE of 24-30; (d) a global Clinical Dementia Rating (CDR) of 0.5; and (e) a positive amyloid PET scan (as determined by a qualified reader).

Antibodies as described herein are formulated, dosed, and administered in a fashion consistent with good medical practice. Factors for consideration in this context include the particular disorder being treated, the particular mammal being treated, the clinical condition of the individual subject, the cause of the disorder, the site of delivery of the agent, the method of administration, the scheduling of administration, and other factors known to medical practitioners.

### Routes of Administration

An antibody as described herein (and any additional therapeutic agent) can be administered by any suitable means, including parenteral, intrapulmonary, and intranasal, and, if desired for local treatment, intralesional administration. Parenteral infusions include intramuscular, intravenous, intraarterial, intraperitoneal, or subcutaneous administration. Dosing can be by any suitable route, *e.g.* by injections, such as intravenous or subcutaneous injections, depending in part on whether the administration is brief or chronic. In one embodiment, the antibody is injected subcutaneously. In another embodiment, the antibody is injected intravenously. In another embodiment, the antibody is administered using a syringe (e.g., prefilled or not) or an autoinjector. In another embodiment, the antibody is inhaled.

### Dosing

For the treatment of an amyloidosis, the appropriate dosage of an antibody as described herein (when used alone or in combination with one or more other additional therapeutic agents) will depend on the specific type of disease to be treated, the type of antibody, the severity and course of the disease, previous therapy, the patient's clinical history and response to the antibody, and the discretion of the attending physician. The antibody is suitably administered to the patient at one time or over a series of treatments. Various dosing schedules including, but not limited to, single or multiple administrations over various time-points, bolus administration, and pulse infusion are contemplated herein.

Depending on the type and severity of the disease, about 0.3 mg/kg to 100 mg/kg *(e.g.* 15 mg/kg-100 mg/kg, or any dosage within that range) of antibody can be an initial candidate dosage for administration to the patient, whether, for example, by one or more separate administrations, or by continuous infusion. One typical daily dosage might range from about 15 mg/kg to 100 mg/kg or more, depending on the factors mentioned above. The dosage can be administered in a single dose or a divided dose (e.g., two doses of 15 mg/kg for a total dose of 30 mg/ kg). For repeated administrations over several weeks or longer, depending on the condition, the treatment would generally be sustained until a desired suppression of disease symptoms occurs. One exemplary dosage of the antibody would be in the range from about 10 mg/kg to about 50 mg/kg. Thus, one or more doses of about 0.5 mg/kg, 1 mg/kg, 1.5 mg/kg, 2.0 mg/kg, 3 mg/kg, 4.0 mg/kg, 5 mg/kg, 10 mg/kg, 15 mg/kg, 20 mg/kg, 25 mg/kg, 30 mg/kg, 35 mg/kg, 40 mg/kg, 50 mg/ kg, 60 mg/ kg, 70 mg/kg, 80 mg/kg, 90 mg/kg, or 100 mg/kg (or any combination thereof) may be administered to the patient. In some embodiments, the total dose administered is in the range of 50 mg to 2500 mg. An exemplary dose of about 50 mg, about 100 mg, 200 mg, 300 mg, 400 mg, about 500 mg, about 600 mg, about 700 mg, about 720 mg, about 1000 mg, about 1050 mg, about 1100 mg, about 1200 mg, about 1300 mg, about 1400 mg, about 1500 mg, about 1600 mg, about 1700 mg, about 1800 mg, about 1900 mg, about 2000 mg, about 2050 mg, about 2100 mg, about 2200 mg, about 2300 mg, about 2400 mg, or about 2500 mg (or any combination thereof) may be administered to the patient. Such doses may be administered intermittently, *e*.*g*. every week, every two weeks, every three weeks, every four weeks, every month, every two months, every three months, or every six months. In some embodiments, the patient receives from one to thirty five doses (*e*.*g*. about eighteen doses of the antibody). However, other dosage regimens may be useful. The progress of this therapy can be monitored by conventional techniques and assays.

In certain embodiments, an antibody as described herein is administered at a dose of 15 mg/kg, 30 mg/kg, 40 mg/kg, 45 mg/kg, 50 mg/kg, 60 mg/kg or a flat dose, e.g., 300 mg, 500 mg, 700 mg, 800 mg, or higher. In some embodiments, the dose is administered by intravenous injection every 2 weeks or every 4 weeks for a period of time. In some embodiments, the dose is administered by subcutaneous injection every 2 weeks or every 4 weeks for a period of time. In certain embodiments, the period of time is 6 months, one year, eighteen months, two years, five years, ten years, 15 years, 20 years, or the lifetime of the patient.

### Monitoring/Assessing Response to Therapeutic Treatment

As used in methods of the present disclosure, the antibody, or antigen-binding fragment hereof, provides therapeutic effect or benefit to the patient. In certain embodiments, the therapeutic benefit is a delay in, or inihibition of, progression of AD or a reduction in clinical, functional, or cognitive decline. In some embodiments, therapeutic effect or benefit is reflected in a "patient response" or "response" (and grammatical variations thereof). Patient response can be assessed using any endpoint indicating a benefit to the patient, including, without limitation, (1) inhibition, to some extent, of disease progression, including slowing down and complete arrest; (2) reduction in amount of plaque or reduction in brain amyloid accumulation; (3) improvement in one or more assessment metrics, including but not limited to ADAS-Cog, iADL, and CDR-SOB scales; (4) improvement in daily functioning of the patient; (5) increase in concentration of one or more biomarkers, *e*.*g*., Abeta, in cerebrospinal fluid; and (6) decrease in one or more biomarkers indicative of the presence of AD. An assessment of patient response may also include an assessment of any adverse events that may occur that may be correlated with the treatment.

In one embodiment, the cognitive ability and daily functioning of the patient is assessed prior to, during, and/or after a course of therapy with an antibody as described herein. A number of cognitive and functional assessment tools have been developed for use in assessing, diagnosing, and scoring mental function, cognition, and neurological deficit. These tools include, but are not limited to, the ADAS-Cog, including the 12 item ADAS-Cog (ADAS-Cog12), the 13-item ADAS-Cog (ADAS-Cog13), the 14-item ADAS-Cog (ADAS-Cog14); the CDR-SOB, including CDR Judgment and Problem solving and CDR Memory components; the Instrumental Activities of Daily Living (iADL); and the MMSE.

"ADAS-Cog" refers to the Alzheimer's Disease Assessment Scale Cognitive Subscale, a multi-part cognitive assessment. *See* Rosen et al., 1984, Amer. J. Psych. 141:1356-1364; Mohs et al., 1997, Alzheimer's Disease Assoc. Disorders 11(2):S13-S21. The higher the numerical score on the ADAS-Cog, the greater the tested patient's deficit or impairment relative to another individual with a lower score. The ADAS-Cog may be used as one measure for assessing whether a treatment for AD is therapeutically effective. An increase in ADAS-Cog score is indicative of worsening in the patient's condition, whereas a decrease in ADAS-Cog score denotes improvement in the patient's condition. As used herein, a "decline in ADAS-Cog performance" or an "increase in ADAS-Cog score" indicates a worsening in the patient's condition and may reflect progression of AD. The ADAS-Cog is an examiner-administered battery that assesses multiple cognitive domains, including memory, comprehension, praxis, orientation, and spontaneous speech (Rosen et al. 1984, Am J Psychiatr 141:1356-64; Mohs et al. 1997, Alzheimer Dis Assoc Disord 11(S2):S13-S21). The ADAS-Cog is a standard primary endpoint in AD treatment trials (Mani 2004, Stat Med 23:305-14). The ADAS-Cog12 is the 70-point version of the ADAS-Cog plus a 10-point Delayed Word Recall item assessing recall of a learned word list. Other ADAS-Cog scales include the ADAS-Cog13 and ADAS-Cog14.

In some embodiments, the methods of treatment provided herein provide a reduction in cognitive decline as measured by an ADAS-Cog score that is at least about 30%, at least about 35%, at least about 40%, or at least about 45% lower relative to placebo.

"MMSE" refers to the Mini Mental State Examination, which provides a score between 1 and 30. *See* Folstein, et al., 1975, J. Psychiatr. Res. 12:189-98. Scores of 26 and lower are generally considered to be indicative of a deficit. The lower the numerical score on the MMSE, the greater the tested patient's deficit or impairment relative to another individual with a lower score. An increase in MMSE score may be indicative of improvement in the patient's condition, whereas a decrease in MMSE score may denote worsening in the patient's condition.

"CDR-SOB" refers to the Clinical Dementia Rating Scale / Sum of Boxes. *See* Hughes *et al*, 1982. CDR-assesses 6 components: memory, orientation, judgment/problem solving, community affairs, home and hobbies, and personal care. The test is administered to both the patient and the caregiver and each component (or each "box"), is scored on a scale of 0 to 3. A complete CDR-SOB score is based on the sum of the scores across all 6 boxes. Subscores can be obtained for each of the boxes or components individually as well, *e*.*g*., CDR/ Memory or CDR/ Judgment and Problem solving. As used herein, a "decline in CDR-SOB performance" or an "increase in CDR-SOB score" indicates a worsening in the patient's condition and may reflect progression of AD. In some embodiments, the methods of treatment provided herein provide a reduction in decline in CDR-SOB performance of at least about 30%, at least about 35%, or at least about 40% relative to placebo.

"iADL" refers to the Instrumental Activities of Daily Living scale. *See* Lawton, M.P., and Brody, E.M., 1969, Gerontologist 9:179-186. This scale measures the ability to perform typical daily activities such as housekeeping, laundry, operating a telephone, shopping, preparing meals, etc. The lower the score, the more impaired the individual is in conducting activities of daily living. In some embodiments, the methods of treatment provided herein provide a reduction in decline of at least about 10%, at least about 15%, or at least about 20% on the iADL scale relative to placebo.

Brain amyloid load or burden can be determined using neurological imaging techniques and tools, for example using PET (positron emission tomography) scanning. Serial PET scans of a patient taken over time, *e*.*g*., before and after administration of a treatment (or at one or more intervals throughout the course of a treatment regimen), can permit detection of increased, decreased, or unchanged amyloid burden in the brain. This technique can further be used to determine whether amyloid accumulation is increasing or decreasing. In some embodiments, detection of amyloid deposits in the brain is performed using florbetapir ¹⁸F. In some embodiments, a florbetapir PET scan is considered positive if, based on a centralized visual read of the scan, it establishes the presence of moderate-to-frequent neuritic plaques.

### Co-Administration

The antibody need not be, but is optionally formulated with one or more agents currently used to prevent or treat the disorder in question or one or more of its symptoms. The effective amount of such other agents depends on the amount of antibody present in the formulation, the type of disorder or treatment, and other factors discussed above. These are generally used in the same dosages and with administration routes as described herein, or about from 1 to 99% of the dosages described herein, or in any dosage and by any route that is empirically/clinically determined to be appropriate. It will be understood by one of ordinary skill in the art that an antibody as described herein may be co-administered simultaneously with any of the foregoing compounds, or may be administered prior to or subsequent to administration of any of the foregoing compounds.

When treating an amyloidosis with an antibody as described herein, a neurological drug may be co-administered. Such neurological drug may be selected from the group including, but not limited to, an antibody or other binding molecule (including, but not limited to a small molecule, a peptide, an aptamer, or other protein binder) that specifically binds to a target selected from: beta secretase, tau, presenilin, amyloid precursor protein or portions thereof, amyloid beta peptide or oligomers or fibrils thereof, death receptor 6 (DR6), receptor for advanced glycation endproducts (RAGE), parkin, and huntingtin; a cholinesterase inhibitor (*i.e.*, galantamine, donepezil, rivastigmine and tacrine); an NMDA receptor antagonist (*i.e.*, memantine), a monoamine depletor (*i.e.*, tetrabenazine); an ergoloid mesylate; an anticholinergic antiparkinsonism agent (*i.e.*, procyclidine, diphenhydramine, trihexylphenidyl, benztropine, biperiden and trihexyphenidyl); a dopaminergic antiparkinsonism agent (*i.e.*, entacapone, selegiline, pramipexole, bromocriptine, rotigotine, selegiline, ropinirole, rasagiline, apomorphine, carbidopa, levodopa, pergolide, tolcapone and amantadine); a tetrabenazine; an anti-inflammatory (including, but not limited to, a nonsteroidal anti-inflammatory drug (*i.e.*, indomethicin and other compounds listed above); a hormone (*i*.*e*., estrogen, progesterone and leuprolide); a vitamin (*i.e.*, folate and nicotinamide); a dimebolin; a homotaurine (*i.e.*, 3-aminopropanesulfonic acid; 3APS); a serotonin receptor activity modulator (*i.e.*, xaliproden); an, an interferon, and a glucocorticoid or corticosteroid. In some embodiments, one or more anti-Abeta antibodies other than crenezumab are co-administered. Non-limiting examples of such anti-Abeta antibodies include solanezumab, bapineuzumab, aducanumab, and gantenerumab. The term "corticosteroid" includes, but is not limited to fluticasone (including fluticasone propionate (FP)), beclometasone, budesonide, ciclesonide, mometasone, flunisolide, betamethasone and triamcinolone. "Inhalable corticosteroid" means a corticosteroid that is suitable for delivery by inhalation. Exemplary inhalable corticosteroids are fluticasone, beclomethasone dipropionate, budenoside, mometasone furoate, ciclesonide, flunisolide, and triamcinolone acetonide.

When treating an amyloidosis that is an ocular disease or disorder with an antibody as described herein, a neurological drug may be selected that is an anti-angiogenic ophthalmic agent (*i.e.*, bevacizumab, ranibizumab and pegaptanib), an ophthalmic glaucoma agent (*i*.*e*., carbachol, epinephrine, demecarium bromide, apraclonidine, brimonidine, brinzolamide, levobunolol, timolol, betaxolol, dorzolamide, bimatoprost, carteolol, metipranolol, dipivefrin, travoprost and latanoprost), a carbonic anhydrase inhibitor (*i.e.*, methazolamide and acetazolamide), an ophthalmic antihistamine (*i.e.*, naphazoline, phenylephrine and tetrahydrozoline), an ocular lubricant, an ophthalmic steroid (*i*.*e*., fluorometholone, prednisolone, loteprednol, dexamethasone, difluprednate, rimexolone, fluocinolone, medrysone and triamcinolone), an ophthalmic anesthetic (*i.e.*, lidocaine, proparacaine and tetracaine), an ophthalmic anti-infective (*i.e.*, levofloxacin, gatifloxacin, ciprofloxacin, moxifloxacin, chloramphenicol, bacitracin/polymyxin b, sulfacetamide, tobramycin, azithromycin, besifloxacin, norfloxacin, sulfisoxazole, gentamicin, idoxuridine, erythromycin, natamycin, gramicidin, neomycin, ofloxacin, trifluridine, ganciclovir, vidarabine), an ophthalmic anti-inflammatory agent (*i.e.*, nepafenac, ketorolac, flurbiprofen, suprofen, cyclosporine, triamcinolone, diclofenac and bromfenac), and an ophthalmic antihistamine or decongestant (*i.e.*, ketotifen, olopatadine, epinastine, naphazoline, cromolyn, tetrahydrozoline, pemirolast, bepotastine, naphazoline, phenylephrine, nedocromil, lodoxamide, phenylephrine, emedastine and azelastine).It is understood that any of the above formulations or therapeutic methods may be carried out using an immunoconjugate as described herein in place of or in addition to an anti-Abeta antibody.

### Articles of Manufacture

The present application also describes an article of manufacture containing materials useful for the treatment, prevention and/or diagnosis of the disorders described above. The article of manufacture comprises a container and a label or package insert on or associated with the container. Suitable containers include, for example, bottles, vials, syringes, IV solution bags, etc. The containers may be formed from a variety of materials such as glass or plastic. The container holds a composition which is by itself or combined with another composition effective for treating, preventing and/or diagnosing the condition and may have a sterile access port (for example the container may be an intravenous solution bag or a vial having a stopper pierceable by a hypodermic injection needle). At least one active agent in the composition is an antibody as described herein. The label or package insert indicates that the composition is used for treating the condition of choice. Moreover, the article of manufacture may comprise (a) a first container with a composition contained therein, wherein the composition comprises an antibody as described herein; and (b) a second container with a composition contained therein, wherein the composition comprises a further cytotoxic or otherwise therapeutic agent. The article of manufacture in this embodiment as described herein may further comprise a package insert indicating that the compositions can be used to treat a particular condition. Alternatively, or additionally, the article of manufacture may further comprise a second (or third) container comprising a pharmaceutically-acceptable buffer, such as bacteriostatic water for injection (BWFI), phosphate-buffered saline, Ringer's solution and dextrose solution. It may further include other materials desirable from a commercial and user standpoint, including other buffers, diluents, filters, needles, and syringes.

It is understood that any of the above articles of manufacture may include an immunoconjugate as described herein in place of or in addition to an anti-Abeta antibody.

### EXAMPLES

### EXAMPLE 1 -- Clinical Study of Crenezumab, a Humanized Anti-Ap Monoclonal Antibody, in the Treatment of Mild to Moderate Alzheimer's Disease

### Study Design and Objectives

A randomized, double blind Phase II trial was conducted, using a placebo control, to evaluate the impact of the humanized monoclonal anti-amyloid beta ("Aβ") antibody crenezumab in patients diagnosed with mild to moderate Alzheimer's Disease (AD). Patients included in the study were, at the time of screening, between the ages of 50 and 80, and had a diagnosis of probable AD according to the NINCDS-ADRDA criteria with: a Mini-Mental State Examination (MMSE) score of 18 to 26 points, a Geriatric Depression Scale (GDS-15) score of less than 6, completion of 6 years of education (or good work history consistent with exclusion of mental retardation or other pervasive developmental disorders). Additionally, for those patients receiving concurrent AD treatment (such as acetylcholinesterase inhibitors or memantine), the patient was confirmed to have been on the medication for at least 3 months and at a stable dose for at least 2 months prior to randomization. At least 50% of the enrolled patients were ApoE4 positive (carrying at least one ApoE4 allele). Patients concurrently receiving one or more non-excluded prescription or over the counter medication, such as non-anticholinergic antidepressant(s), atypical antipsychotic(s), non-benzodiazepine anxiolytic(s), soporific(s), centrally acting anticholinergic antihistamine(s), and centrally acting anticholinergic antispasmodic(s), were also allowed to enroll provided that the dose administered was constant for at least 1 month prior to randomization and remained the same for the duration of the study.

Individuals were excluded from the trial if: they suffer from a severe or unstable medical condition that, in the opinion of the investigator or sponsor, would interfere with the patient's ability to complete the study assessments or would require the equivalent of institutional or hospital care; there is a history or presence of clinically evident vascular disease potentially affecting the brain; there is a history of severe, clinically significant central nervous system trauma (such as persistent neurological deficit or structural brain damage); they have been hospitalized in the 4 weeks before screening; they have previously been treated with crenezumab or any other agent that targets Aβ; or if they have received treatment with any biological therapy (other than routine vaccinations) within the longer of 5 half-lives of the therapeutic agent in the biological therapy or 3 months before screening.

The study had three periods - a screening period lasting up to 35 days, a treatment period lasting 68 weeks (referred to herein as Week 1, Week 2, etc., up to Week 69), and a safety follow-up period lasting a further 16 weeks (referred to herein as Week 70, etc., up to Week 85). Treatment (or placebo) was administered via intravenous infusion.

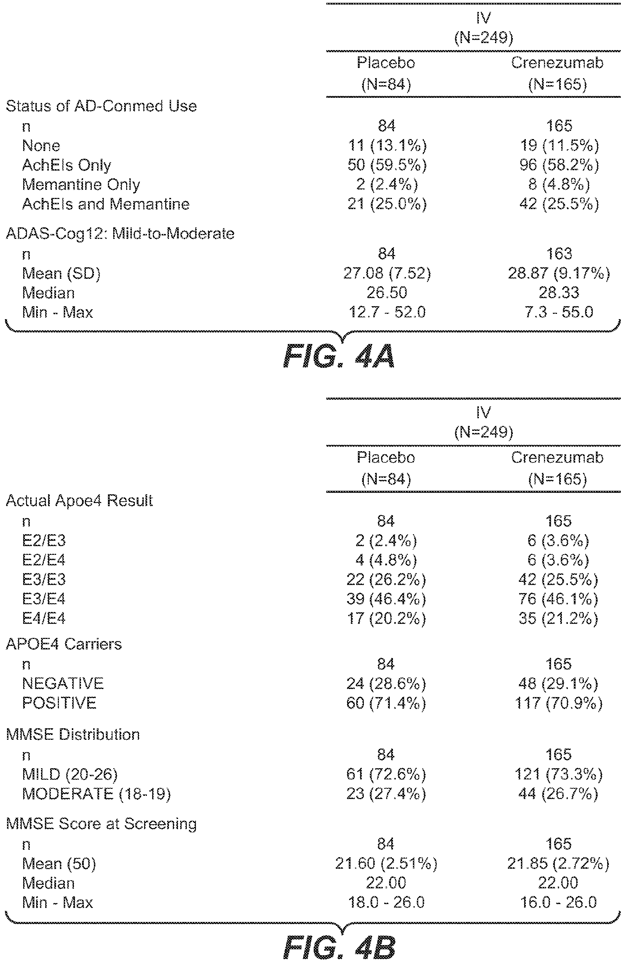

Patients are enrolled in the trial and randomized into one of two arms, a treatment (*i*.*e*., crenezumab) arm and a placebo arm in a 2:1 (treatment arm:placebo arm) randomization. 249 patients with an MMSE score from 18 to 26 (categorized as mild to moderate AD) were enrolled in the trial, of whom 165 received treatment and 84 received placebo. 121 patients in treatment arm and 61 patients in the placebo arm had an MMSE score between 20 and 26 (categorized as mild AD). Within the treatment art 117 (or 70.9%) were ApoE4 positive. In the placebo arm, 60 patients (or 71.4%) were ApoE4 positive. *See* **FIGS. 4A-B** (Tabulating Patient Disposition).

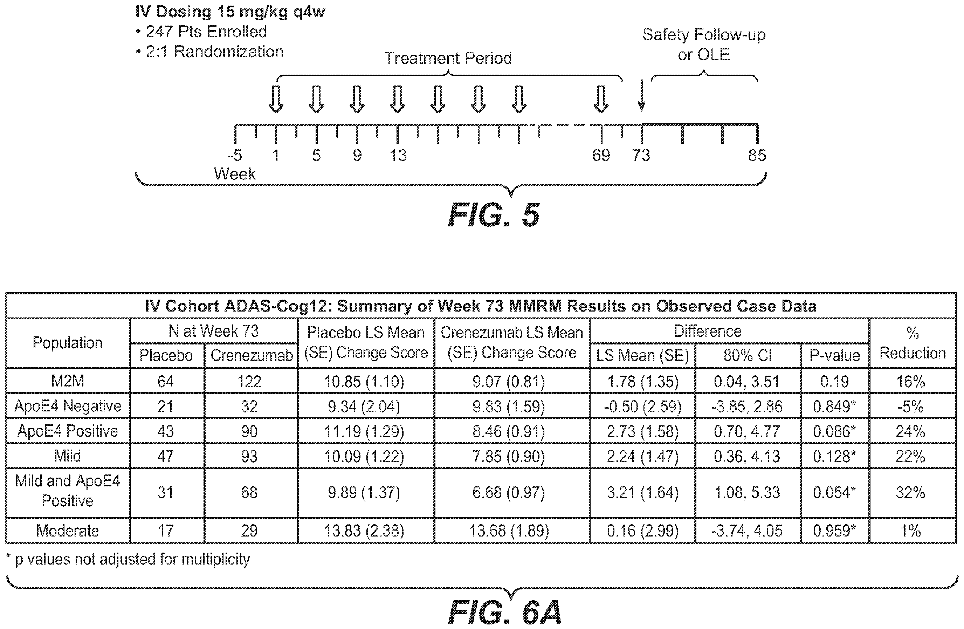

A safety run-in assessment of 43 days was performed to determine the safety and tolerability of a 15 mg/kg intravenous dose versus a 10 mg/kg intravenous dose and a dose of 15 mg/kg was chosen. Patients in both arms of the trial received a blinded intravenous injection every four weeks for 68 weeks; based on the results of the safety run-in, patients in the treatment arm receive 15 mg/kg, while patients in the placebo arm received an intravenous injection of placebo. *See* **FIG.5** (Protocol Schematic).

Patients were assessed after 72 weeks for (a) change in ADAS-Cog12 score and CDR-SOB score at Weeks 25, 49, and 73 from the baseline score at the start of trial, to evaluate inhibition of disease progression and (b) safety and tolerability of crenezumab as compared to placebo. To estimate statistical significance of any measured change, analysis of covariance, confidence intervals, and least squares estimates of the difference in the mean change from baseline were calculated.

The safety and tolerability of crenezumab was assessed by measuring the frequency and severity of treatment emergent adverse events throughout the trial, especially instances of symptomatic or asymptomatic ARIA-E (including cerebral vasogenic edema), symptomatic or asymptomatic ARIA-H (including cerebral microhemorrhage), and cerebral macrohemorrhage. The presence and/or number of cerebral vasogenic edema cases during the screening period (before the start of dosing) or during the treatment period (after the start of dosing with placebo or crenezumab) was assessed by fluid attenuated inversion recovery magnetic resonance imaging (FLAIR MRI). *See, e*.*g*., Sperling et al., 2011, Alzheimer's & Dementia 7:367-385. The presence and/or number of cerebral microhemorrhage(s) during the screening period (before the start of dosing) or during the treatment period (after the start of dosing with placebo or crenezumab) was assessed by transverse magnetization relaxation time with additional inhomogeneous dephasing gradient recalled echo magnetic resonance imaging (T2*-weighted GRE MRI).

### Results

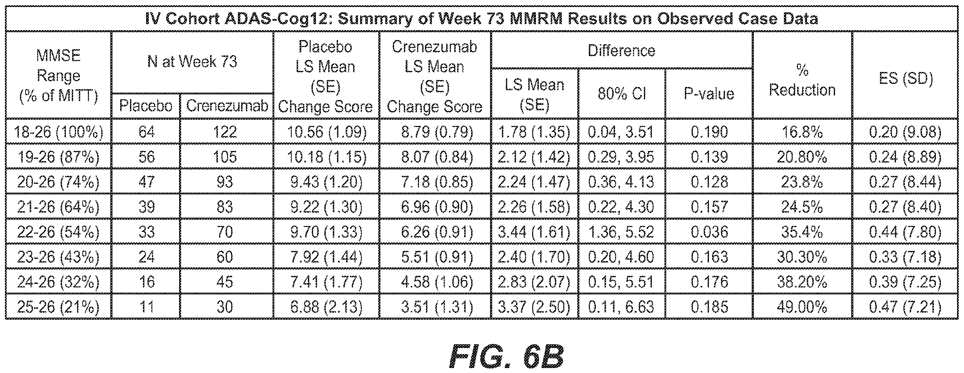

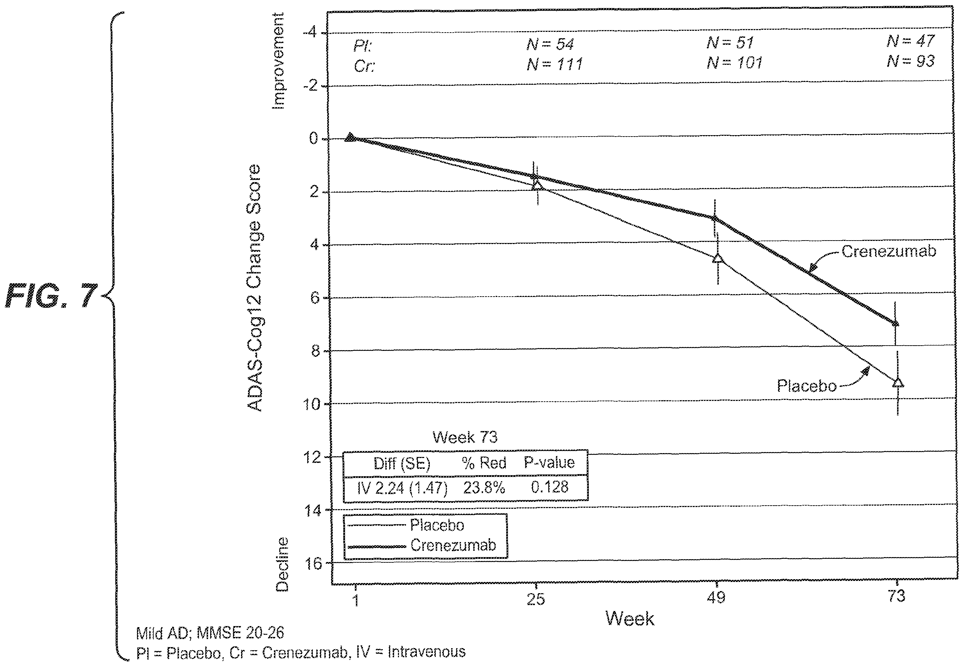

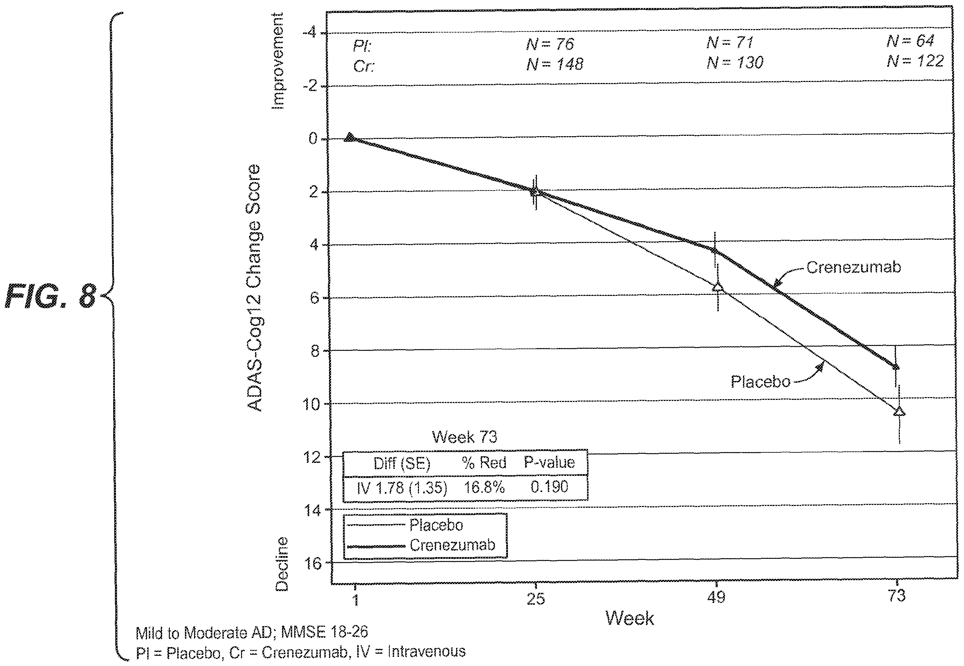

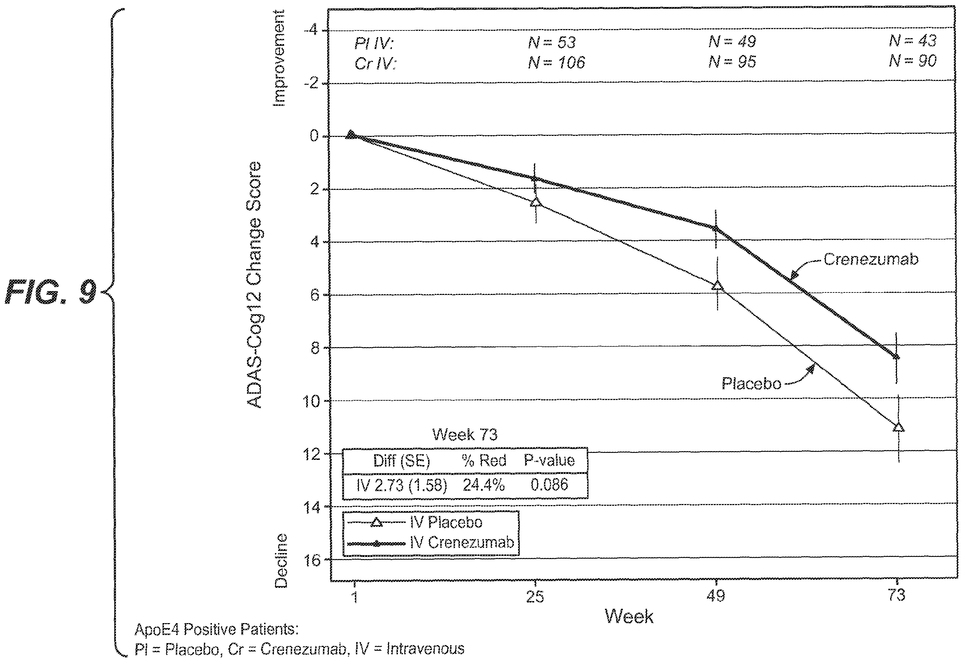

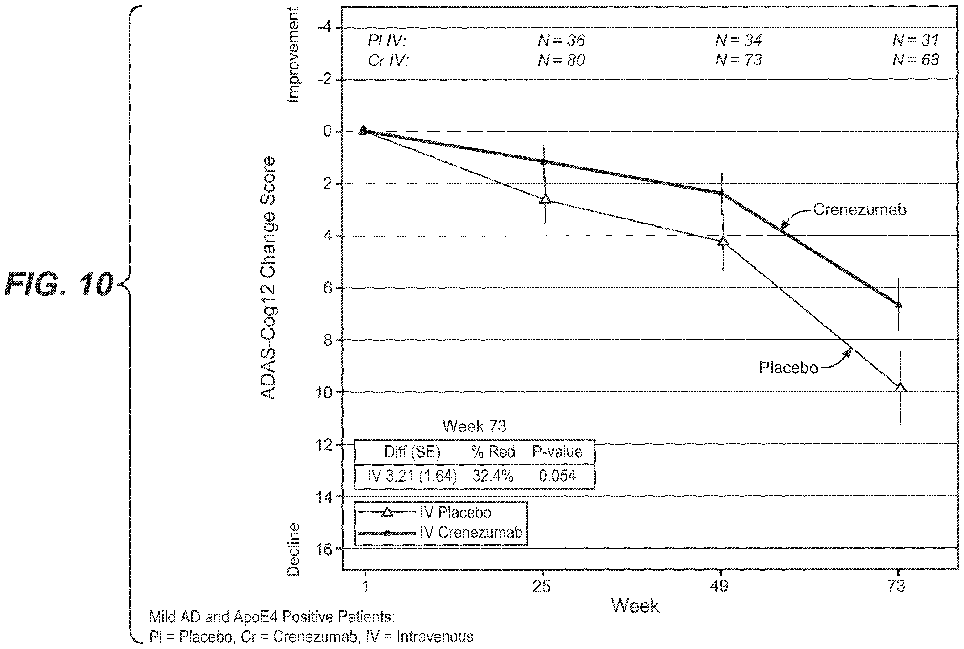

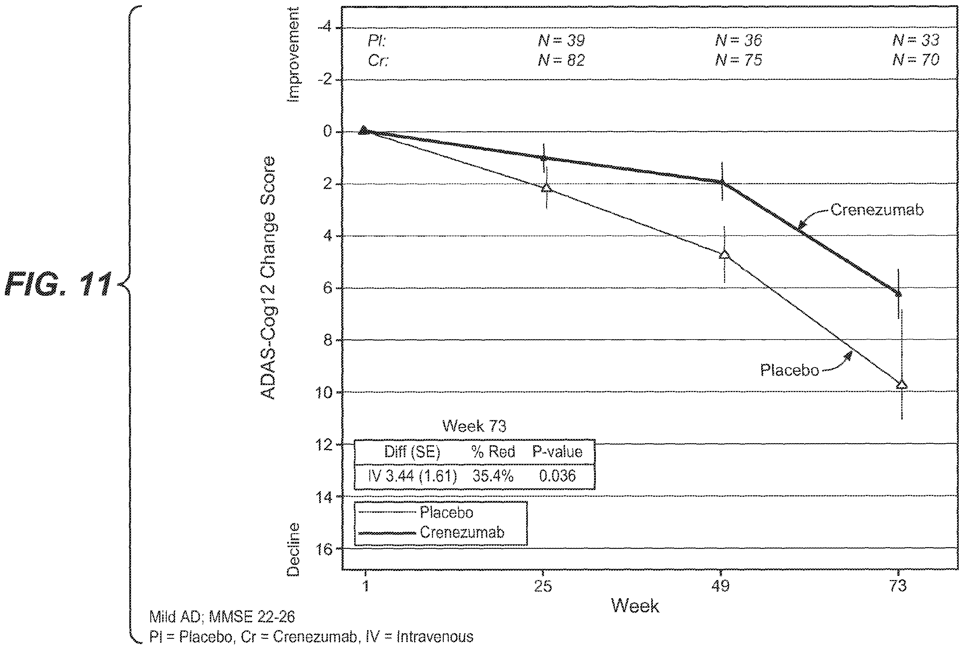

ADAS-Cog12 measurements at 73 weeks demonstrate that patients received crenezumab showed less disease progression than patients who received placebo. As summarized in the tables shown in **FIG. 6A-B** and in the charts shown in **FIGS. 7-8**, the change in the ADAS-Cog12 score was about 24% (p=0.12) less in the treatment arm than in the placebo arm for patients with mild AD and about 16% (p=0.19) less in the treatment arm than in the placebo arm, for patients with mild to moderate AD. This effect was also seen between ApoE4 positive patients in the treatment arm versus placebo arm: there was 24.4% (p=0.08, not adjusted for multiplicity) less increase in the ADAS-Cog12 score (where an increase in the ADAS-Cog12 score is indicative of disease progression) in the patients receiving crenezumab relative to the patients receiving placebo. *See* **FIG. 6A** and **FIG. 9**. The ApoE4 positive patients included patients with both mild and moderate AD. The effect was even more pronounced when the results for both mild and ApoE4 positive patients were pooled: a reduction of 32.4% (p=0.05, not adjusted for multiplicity) was seen in the treatment arm relative to the placebo arm. *See* **FIG. 6A** and **FIG. 10**. The treatment effect increased with increasing MMSE score at enrollment. As shown in FIG. 6B, the higher the MMSE score, the greater the percent reduction in ADAS-Cog12 in the treatment arm versus the placebo arm, ranging from about 16% for patients with an MMSE between 18-26 up to a 49% reduction in patients with an MMSE between 25 and 26. *See also,* **FIG 11**. For patients, having an MMSE score between 22 and 26, the percent reduction in ADAS-Cog12 in the treatment arm compared to the placebo arm was about 35%.

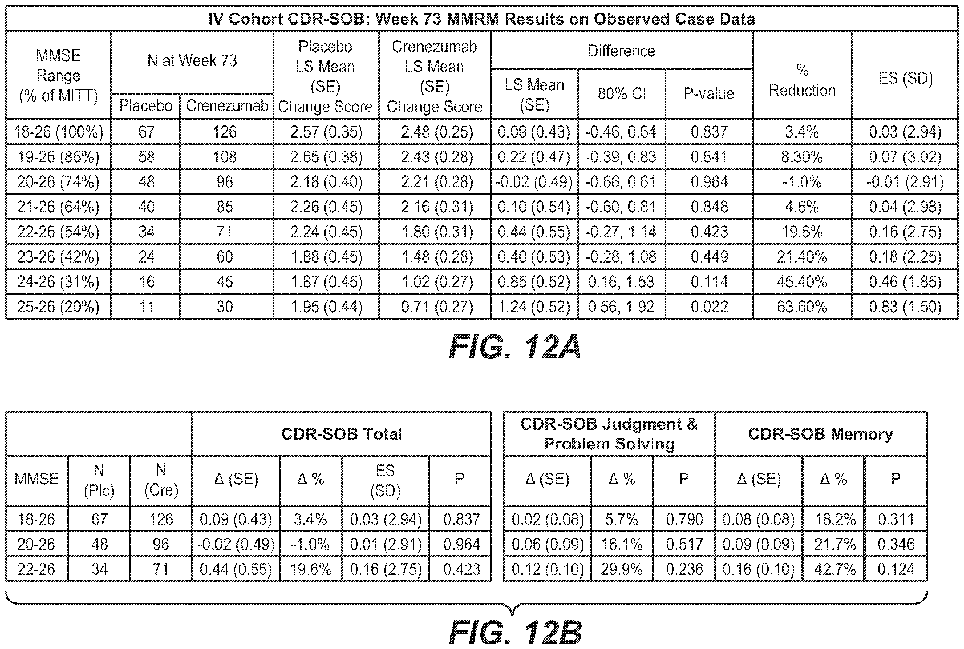

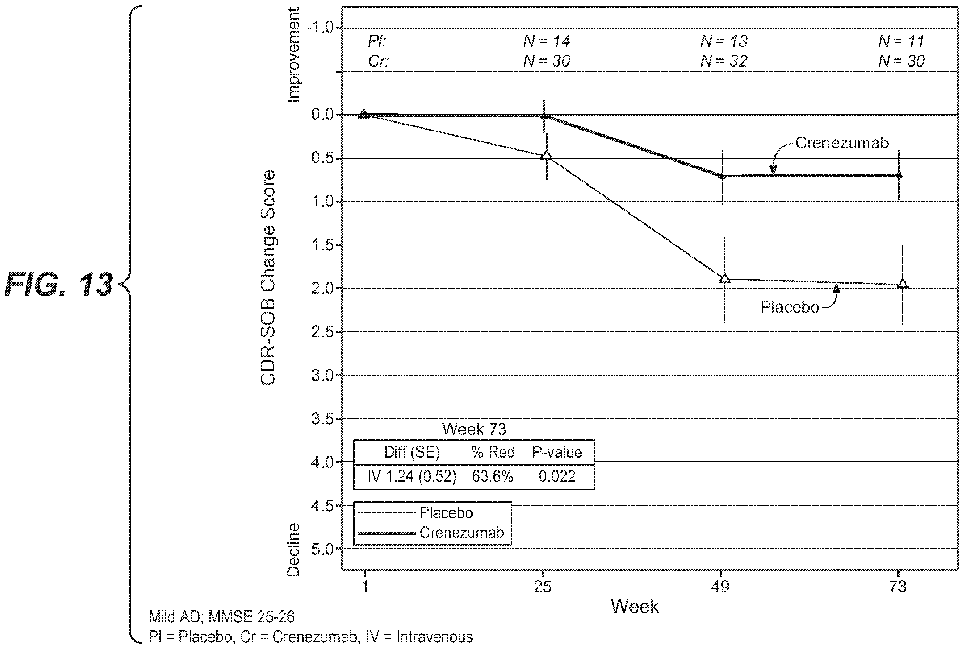

The change in CDR-SOB showed a similar trend in treatment effect. As shown in **FIG. 12A**, a 19% reduction in the change in CDR-SOB scores was seen in the treatment arm versus placebo for patients having an MMSE of 22-26, and this effect was even more pronounced in patients having an MMSE score of 25-26, where the percent reduction was about 63% (see also **FIG 13**). **FIG. 12B** shows that when looking at the Memory or the Judgment and Problem solving component scores for patients having an MMSE of 22-26, the percent reduction was about 42% and 30% respectively.

The study further demonstrated that crenezumab did not increase the incidence of ARIA-type events when dosed at 15 mg/kg. A single, asymptomatic ARIA-E event was observed in the study, in a patient receiving crenezumab. The number of ARIA-H incidents was balanced between the treatment and placebo arms.

These data demonstrate that crenezumab inhibits disease progression without increasing the incidence of a treatment emergent adverse event such as ARIA-E or ARIA-H when administered at a dose of 15 mg/kg in patients suffering from mild to moderate AD, particularly in patients with mild AD and/or who are ApoE4 positive.

### EXAMPLE 2 -- Clinical Study of Crenezumab, a Humanized Anti-Ap Monoclonal Antibody, in the Treatment of Mild to Moderate Alzheimer's Disease and to Evaluate the Impact on Amyloid Load

### Study Design and Objectives

A randomized, double blind Phase II trial was conducted, using a placebo control, to evaluate the impact of the humanized monoclonal anti-amyloid beta ("Aβ") antibody crenezumab in patients diagnosed with mild to moderate Alzheimer's Disease (AD). Patients included in the study were, at the time of screening, between the ages of 50 and 80, and had a diagnosis of probable AD according to the NINCDS-ADRDA criteria with: a Mini-Mental State Examination (MMSE) score of 18 to 26 points, a Geriatric Depression Scale (GDS-15) score of less than 6, completion of 6 years of education (or good work history consistent with exclusion of mental retardation or other pervasive developmental disorders). Only patients with a positive florbetapir PET ("amyloid positive") scan at screening, indicative of increased brain amyloid load in the range expected for patients diagnosed with AD as assessed by florbetapir-PET scan, were enrolled. Additionally, at least 50% of the enrolled patients were ApoE4 positive.

Patients were enrolled in the trial and randomized into one of two arms, a treatment (*i.e.*, crenezumab) arm and a placebo arm in a 2:1 (treatment arm:placebo arm) randomization. 52 patients across both arms of the trial received a blinded intravenous injection every four weeks for 73 weeks. In the treatment arm, patients received a 15 mg/kg dose of crenezumab. Patients were stratified according to: ApoE4 status (carrier versus non-carrier) and MMSE score.

Data were collected for changes in: ADAS-Cog12, amyloid burden as measured using florbetapir-PET, and Abeta levels in cerebrospinal fluid (CSF). Florbetapir PET scans were acquired at the screening, 12 month, and 18 month visits using florbetapir 10 mCi, with a 50-min. uptake period and 30 min. emission scan. Images from 6X5 minute frames (or 1X15 minute frames on scanners without dynamic capability) were normalized to standard space where a template was used to extract the mean signals from several regions of interest (ROIs). Baseline T1-weighted MRI scans were used to refine the volumes of the template ROIs. Analyses were conducted using cerebellar cortex or subcortical white matter as a reference region. CSF was collected at screening and prior to dosing at month 18. CSF Abeta, tau and p-tau(181) were measured. ANCOVA or mixed model for repeated measures was used for statistical analysis of treatment differences at study endpoints.

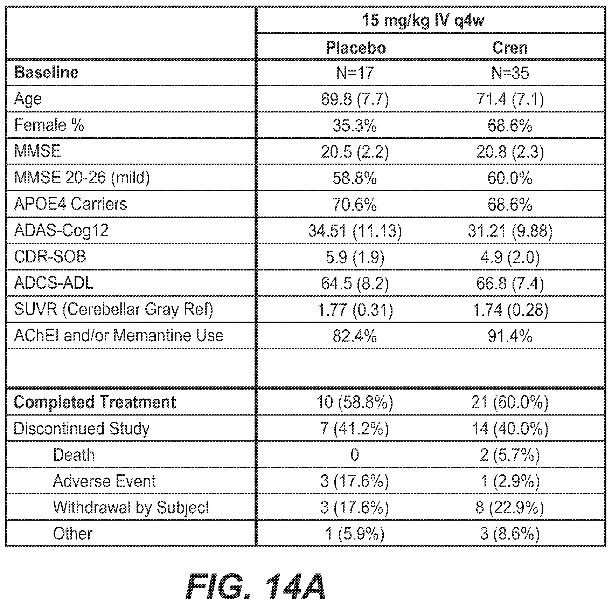

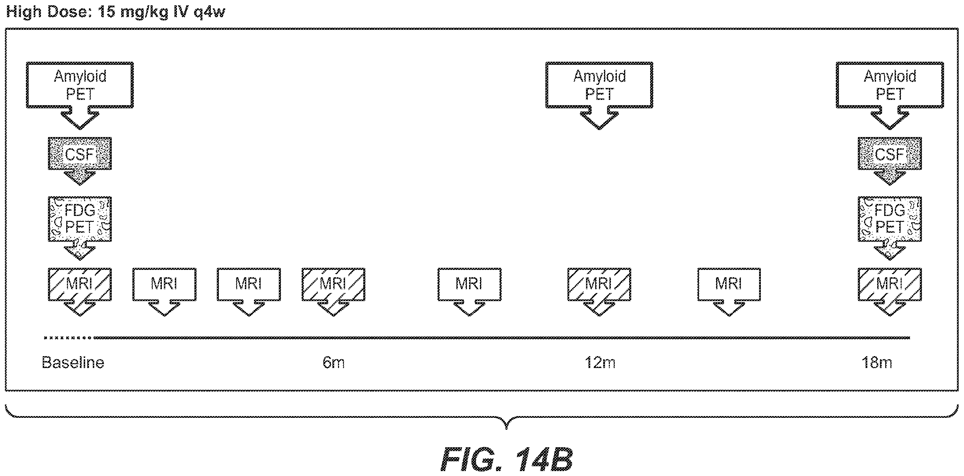

Patient characteristics, adverse events, and timing of PET scans, MRI scans, and CSF sampling are shown in **FIG. 14A-B**.

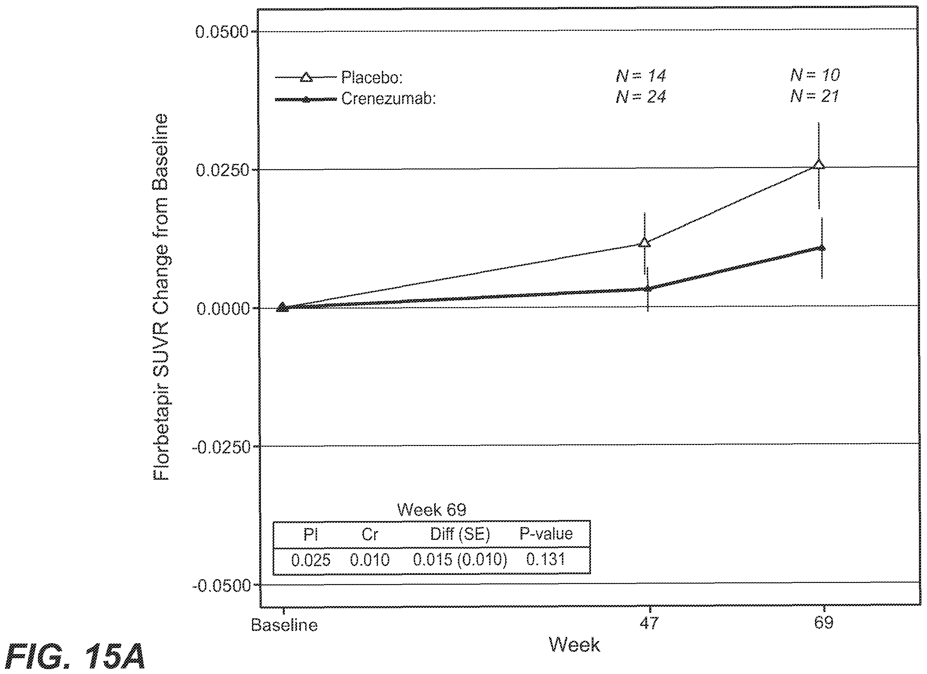

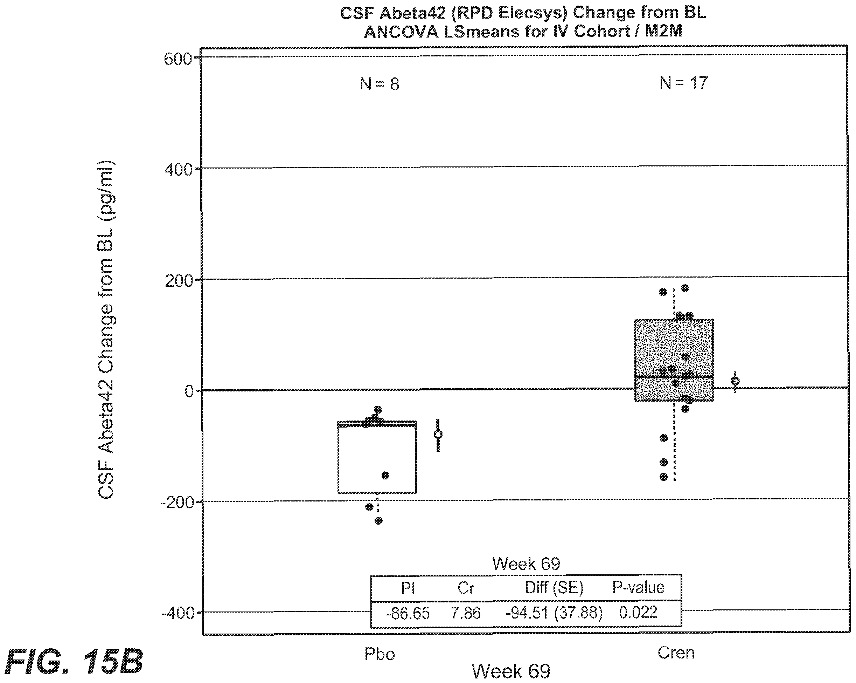

**Results.** ADAS-Cog12 measurements at the end of the treatment period demonstrate that patients who received crenezumab showed less disease progression than patients who received placebo. A 54.3% reduction in cognitive decline was observed in patients with an initial MMSE score between 20 and 26 (p=0.2). Consistent with this observed slowing in disease progression, a decrease in the accumulation of amyloid deposits was also observed by PET analysis (with a subcortical white matter reference region) in patients treated with crenezumab versus patient receiving placebo. *See* **FIG. 15A**. Furthermore, an increase in cerebrospinal fluid concentration of Abeta was detected in the treatment arm, consistent with engagement of the target by crenezumab. *See* **FIG. 15B**. A similar increase in cerebrospinal fluid concentration of Abeta was detected in patients treated with 300 mg subcutaneous administration of crenezumab every two weeks versus patients receiving placebo.

These data demonstrate that crenezumab engages its target, amyloid beta, and inhibits disease progression when administered at a dose of 15 mg/kg in patients suffering from mild to moderate AD, particularly in patients with mild AD, including in patients having a brain amyloid burden that is typical of that seen in patients diagnosed with AD.

**SEQUENCE LISTING KEY**

| **SEQ ID NO:** | **Sequence** |
|---|---|
| 1 | Human Aβ1-42 amino acid sequence: DAEFRHDSGYEVHHQKLVFFAEDVGSNKGAIIGLMVGGVVIA |
| 2 | Crenezumab HVR-H1 amino acid sequence: GFTFSSYGMS |
| 3 | Crenezumab HVR-H2 amino acid sequence: SINSNGGSTYYPDSVK |
| 4 | Crenezumab HVR-H3 amino acid sequence: GDY |
| 5 | Crenezumab heavy chain amino acid sequence (HVR regions marked in bold text): |
| | |
| 6 | Crenezumab HVR-L1 amino acid sequence: RSSQSLVYSNGDTYLH |
| 7 | Crenezumab HVR-L2 amino acid sequence: KVSNRFS |
| 8 | Crenezumab HVR-L3 amino acid sequence: SQSTHVPWT |
| 9 | Crenezumab light chain amino acid sequence (HVR regions marked in bold and underlined text): |
| | |

## Claims

1. Crenezumab for use in a method of treating Alzheimer's Disease (AD) comprising delaying the development of AD or slowing the progression of AD in a patient with an MMSE score of 20 or above, without increasing the risk of an adverse event in said patient relative to a placebo, wherein the adverse event is selected from: (i) Amyloid-Related Imaging Abnormality-Edema (ARIA-E) and(ii) Amyloid-Related Imaging Abnormality-Hemorrhage (ARIA-H);
wherein the crenezumab is administered at a dose of 15 mg/kg to 100 mg/kg of the patient's body weight, said dose being administered repeatedly every four weeks or every month.

2. The crenezumab for the use of claim 1, wherein the crenezumab is administered at a dose of 60 mg/kg of the patient's body weight.

3. The crenezumab for the use of claim 1 or claim 2, wherein the treatment reduces the decline in cognitive capacity in the patient and the decline in cognitive capacity is assessed by determining the patient's score before and after administration of said crenezumab using a 12-item Alzheimer's Disease Assessment Scale -Cognition (ADAS-Cog12), 13-item Alzheimer's Disease Assessment Scale - Cognition (ADAS-Cog13), or 14-item Alzheimer's Disease Assessment Scale - Cognition (ADAS-Cog14) test.

4. The crenezumab for the use of claim 3, wherein the reduction in cognitive decline as measured by ADAS-Cog is at least 30%, at least 35%, at least 40%, or at least 45% relative to placebo.

5. The crenezumab for the use of any one of claims 1 to 4, wherein the patient is ApoE4 positive.

6. The crenezumab for the use of claim any one of claims 1 to 5, wherein the patient is amyloid positive.

7. The crenezumab for the use of any one of claims 1 to 6, wherein the patient has a Mini-Mental State Examination (MMSE) score between 20 and 26, between 24 and 30, between 21 and 26, between 22 and 26, between 22 and 28, between 23 and 26, between 24 and 26, or between 25 and 26 before initiation of treatment.

8. The crenezumab for the use of any one of claims 1 to 7, wherein the crenezumab is formulated for intravenous injection.

9. The crenezumab for the use of any one of claims 1 to 8, wherein the adverse event is ARIA-E.

10. The crenezumab for the use of claim 9, wherein:
(a) if a treatment emergent ARIA-E is detected, the method comprises halting administration of the crenezumab and, optionally, administering treatment for ARIA-E; or
(b) if a treatment emergent ARIA-E is detected, the method comprises halting administration of the crenezumab and, optionally, administering treatment for ARIA-E and the method further comprises resuming administration of said crenezumab after the ARIA-E is resolved and the crenezumab is administered at a lower dose than before administration was halted.

11. The crenezumab for the use of any one of claims 1 to 8, wherein if one or more new ARIA-Es is detected in the patient during treatment with said crenezumab, the method comprises administering no more crenezumab, and, optionally, administering a corticosteroid.

12. The crenezumab for the use of any one of claims 1 to 11, wherein the patient is being concurrently treated with one or more agents selected from the group consisting of: a therapeutic agent that specifically binds to a target; a cholinesterase inhibitor; an NMDA receptor antagonist; a monoamine depletor; an ergoloid mesylate; an anticholinergic antiparkinsonism agent; a dopaminergic antiparkinsonism agent; a tetrabenazine; an antiinflammatory agent; a hormone; a vitamin; a dimebolin; a homotaurine; a serotonin receptor activity modulator; an interferon; a glucocorticoid; an anti-Abeta antibody other than crenezumab; an antibiotic; and an anti-viral agent.

13. The crenezumab for the use of claim 12, wherein the agent is a cholinesterase inhibitor selected from the group consisting of galantamine, donepezil, rivastigmine and
tacrine.

14. The crenezumab for the use of claim 12, wherein the agent is an NMDA receptor antagonist, optionally, memantine or a salt thereof.

15. The crenezumab for the use of claim 12, wherein the agent is a therapeutic agent that specifically binds to a target and the target is selected from the group consisting of beta secretase, tau, presenilin, amyloid precursor protein or portions thereof, amyloid beta peptide or oligomers or fibrils thereof, death receptor 6 (DR6), receptor for advanced glycation end products (RAGE), parkin, and huntingtin.

16. The crenezumab for the use of claim 12, wherein the agent is
(a) a monoamine depletor, optionally tetrabenazine; or
(b) an anticholinergic antiparkinsonism agent selected from the group consisting of procyclidine, diphenhydramine, trihexylphenidyl, benztropine, biperiden and trihexyphenidyl; or
(c) a dopaminergic antiparkinsonism agent selected from the group consisting of: entacapone, selegiline, pramipexole, bromocriptine, rotigotine, selegiline, ropinirole, rasagiline, apomorphine, carbidopa, levodopa, pergolide, tolcapone and amantadine; or
(d) an anti-inflammatory agent selected from the group consisting of: a nonsteroidal anti-inflammatory drug and indomethacin; or
(e) a hormone selected from the group consisting of: estrogen, progesterone and leuprolide; or
(f) a vitamin selected from the group consisting of: folate and nicotinamide; or
(g) a homotaurine, which is 3-aminopropanesulfonic acid or 3APS; or
(h) xaliproden; or
(i) an anti-Abeta antibody other than crenezumab, optionally wherein the antibody is selected from the group consisting of solanezumab, bapineuzumab, aducanumab, and gantenerumab.

## Patentansprüche

1. Crenezumab zur Verwendung in einem Verfahren zur Behandlung von Alzheimer-Krankheit (AK), das eine Verzögerung der Entwicklung von AK oder eine Verlangsamung der Progression von AK bei einem Patienten mit 20 oder mehr MMSE-Punkten umfasst, ohne dass das Nebenwirkungsrisiko bei dem Patienten im Vergleich zu einem Placebo steigt, wobei die Nebenwirkung aus Folgenden ausgewählt ist: (i) Amyloid-bezogenen, durch Bildgebung nachgewiesenen Anomalien in Form von Ödemen (Amyloid-Related Imaging Abnormality-Edema, ARIA-E) und (ii) Amyloid-bezogenen, durch Bildgebung nachgewiesenen Anomalien in Form von Blutungen (Amyloid-Related Imaging Abnormality-Hemorrhage, ARIA-H);
wobei Crenezumab in einer Dosis von 15 mg/kg bis 100 mg/kg des Körpergewichts des Patienten verabreicht wird, wobei die Dosis wiederholt alle vier Wochen oder einmal pro Monat verabreicht wird.

2. Crenezumab zur Verwendung nach Anspruch 1, wobei Crenezumab in einer Dosis von 60 mg/kg des Körpergewichts des Patienten verabreicht wird.

3. Crenezumab zur Verwendung nach Anspruch 1 oder Anspruch 2, wobei die Behandlung die Verschlechterung der kognitiven Leistungsfähigkeit des Patienten verringert und die Verschlechterung der kognitiven Leistungsfähigkeit durch Bestimmen der Punkte des Patienten unter Verwendung eines Tests mit einer Alzheimer-Krankheit-Beurteilungsskala - Kognition mit 12 Punkten (ADAS-Cog12), einer Alzheimer-Krankheit-Beurteilungsskala - Kognition mit 13 Punkten (ADAS-Cog13) oder einer Alzheimer-Krankheit-Beurteilungsskala - Kognition mit 14 Punkten (ADAS-Cog14) vor und nach der Verabreichung von Crenezumab bewertet wird.

4. Crenezumab zur Verwendung nach Anspruch 3, wobei die mittels ADAS-Cog gemessene Verringerung der kognitiven Verschlechterung zumindest 30 %, zumindest 35 %, zumindest 40 % oder zumindest 45 % bezogen auf ein Placebo beträgt.

5. Crenezumab zur Verwendung nach einem der Ansprüche 1 bis 4, wobei der Patient ApoE4-positiv ist.

6. Crenezumab zur Verwendung nach einem der Ansprüche 1 bis 5, wobei der Patient Amyloid-positiv ist.

7. Crenezumab nach einem der Ansprüche 1 bis 6, wobei der Patient vor Behandlungsbeginn zwischen 20 und 26, zwischen 24 und 30, zwischen 21 und 26, zwischen 22 und 26, zwischen 22 und 28, zwischen 23 und 26, zwischen 24 und 26 oder zwischen 25 und 26 Punkten in einer "Mini-Mental State Examination" (MMSE) aufweist.

8. Crenezumab zur Verwendung nach einem der Ansprüche 1 bis 7, wobei Crenezumab zur intravenösen Injektion formuliert ist.

9. Crenezumab zur Verwendung nach einem der Ansprüche 1 bis 8, wobei die Nebenwirkung ARIA-E ist.

10. Crenezumab zur Verwendung nach Anspruch 9, wobei:
(a) wenn während der Behandlung ARIA-E auftritt, das Verfahren das Stoppen der Verabreichung von Crenezumab und gegebenenfalls die Verabreichung einer Behandlung für ARIA-E umfasst; oder
(b) wenn während der Behandlung ARIA-E auftritt, das Verfahren das Stoppen der Verabreichung von Crenezumab und gegebenenfalls die Verabreichung einer Behandlung für ARIA-E umfasst und das Verfahren weiters das Wiederaufnehmen der Verabreichung von Crenezumab umfasst, nachdem das ARIA-E verschwunden ist, wobei Crenezumab in einer geringeren Dosis verabreicht wird als vor dem Stoppen der Verabreichung.

11. Crenezumab zur Verwendung nach einem der Ansprüche 1 bis 8, wobei das Verfahren bei Nachweis von einem oder mehreren ARIA-Es in dem Patienten während der Behandlung mit Crenezumab umfasst, dass kein weiteres Crenezumab verabreicht wird und gegebenenfalls ein Corticosteroid verabreicht wird.

12. Crenezumab zur Verwendung nach einem der Ansprüche 1 bis 11, wobei der Patient gleichzeitig mit einem oder mehreren Mitteln behandelt wird, die aus folgender Gruppe ausgewählt sind: einem Therapeutikum, das spezifisch an ein Target bindet; einem Cholinesterase-Inhibitor; einem NMDA-Rezeptor-Antagonisten; einem Monoamin-Abreicherungsmittel; einem Ergoloidmesylat; einem anticholinergen Antiparkinsonikum; einem dopaminergen Antiparkinsonikum; einem Tetrabenazin; einem Antiinflammatorikum; einem Hormon; einem Vitamin; einem Dimebolin; einem Homotaurin; einem Serotoninrezeptoraktivitätsmodulator; einem Interferon; einem Glucocorticoid; einem anderen Anti-Aß-Antikörper als Crenezumab; und einem Virostatikum.

13. Crenezumab zur Verwendung nach Anspruch 12, wobei das Mittel ein Cholinesterase-Inhibitor ist, der aus der aus Galantamin, Donepezil, Rivastigmin und Tacrin bestehenden Gruppe ausgewählt ist.

14. Crenezumab zur Verwendung nach Anspruch 12, wobei das Mittel ein NMDA-Rezeptor-Antagonist, gegebenenfalls Memantin oder ein Salz davon ist.

15. Crenezumab zur Verwendung nach Anspruch 12, wobei das Mittel ein Therapeutikum ist, das spezifisch an ein Target bindet, und das Target aus der aus β-Sekretase, Tau, Presenilin, Amyloid-Vorläuferprotein oder Teilen davon, Amyloid-β-Peptid oder Oligomeren oder Fibrillen davon, Death-Rezeptor 6 (DR6), einem Rezeptor für fortgeschrittene Glykationsendprodukte (RAGE), Parkin und Huntingtin bestehenden Gruppe ausgewählt ist.

16. Crenezumab zur Verwendung nach Anspruch 12, wobei das Mittel Folgendes ist:
(a) ein Monoamin-Abreicherungsmittel, gegebenenfalls Tetrabenazin; oder
(b) ein anticholinerges Antiparkinsonikum, das aus der aus Procyclidin, Diphenhydramin, Trihexylphenidyl, Benzotropin, Biperiden und Trihexyphenidyl bestehenden Gruppe ausgewählt ist; oder
(c) ein dopaminerges Antiparkinsonikum, das aus folgender Gruppe ausgewählt ist: Entacapon, Selegilin, Pramipexol, Bromocriptin, Rotigotin, Selegilin, Ropinirol, Rasagilin, Apomorphin, Carbidopa, Levodopa, Pergolid, Tolcapon und Amantadin; oder
(d) ein Antiinflammatorikum, das aus folgender Gruppe ausgewählt ist: einem nichtsteroidalen Antiinflammatorikum und Indomethacin; oder
(e) ein Hormon, das aus folgender Gruppe ausgewählt ist: Östrogen, Progesteron und Leuprolid; oder
(f) ein Vitamin, das aus folgender Gruppe ausgewählt ist: Folat und Nicotinamid; oder
(g) ein Homotaurin, das 3-Aminopropansulfonsäure oder 3APS ist; oder
(h) Xaliproden; oder
(i) ein anderer Anti-Aβ-Antikörper als Crenezumab, wobei der Antikörper gegebenenfalls aus der aus Solanezumab, Bapineuzumab, Aducanumab und Gantenerumab bestehenden Gruppe ausgewählt ist.

## Revendications

1. Crénézumab pour une utilisation dans une méthode de traitement de la maladie d'Alzheimer (MA) comprenant le retard du développement de la MA ou le ralentissement de la progression de la MA chez un patient avec un score MMSE supérieur ou égal à 20, sans augmenter le risque d'un événement indésirable chez ledit patient par rapport à un placebo, dans lequel l'événement secondaire est choisi parmi : (i) une anomalie de l'imagerie liée à l'amyloïde-oedème (ARIA-E) et (ii) une anomalie de l'imagerie liée à l'amyloïde-hémorragie (ARIA-H) ;
dans lequel le crénézumab est administré à une dose de 15 mg/kg à 100 mg/kg du poids corporel du patient, ladite dose étant administrée de manière répétée toutes les quatre semaines ou tous les mois.

2. Crénézumab pour une utilisation selon la revendication 1, dans lequel le crénézumab est administré à une dose de 60 mg/kg du poids corporel du patient.

3. Crénézumab pour une utilisation selon la revendication 1 ou la revendication 2, dans lequel le traitement réduit le déclin de la capacité cognitive chez le patient et le déclin de la capacité cognitive est évalué en déterminant le score du patient avant et après l'administration dudit crénézumab en utilisant un test de cognition avec une échelle d'évaluation de la maladie d'Alzheimer à 12 critères (ADAS-Cog12), une échelle d'évaluation de la maladie d'Alzheimer à 13 critères (ADAS-Cog13) ou une échelle d'évaluation de la maladie d'Alzheimer à 14 critères (ADAS-Cog14).

4. Crénézumab pour une utilisation selon la revendication 3, dans lequel la réduction du déclin cognitif comme mesuré par ADAS-Cog est d'au moins 30 %, d'au moins 35 %, d'au moins 40 % ou d'au moins 45 % par rapport au placebo.

5. Crénézumab pour une utilisation selon l'une quelconque des revendications 1 à 4, dans lequel le patient est positif à l'ApoE4.

6. Crénézumab pour une utilisation selon l'une quelconque des revendications 1 à 5, dans lequel le patient est positif à l'amyloïde.

7. Crénézumab pour une utilisation selon l'une quelconque des revendications 1 à 6, dans lequel le patient a un score de mini-examen de l'état mental (MMSE) compris entre 20 et 26, entre 24 et 30, entre 21 et 26, entre 22 et 26, entre 22 et 28, entre 23 et 26, entre 24 et 26 ou entre 25 et 26 avant l'initiation du traitement.

8. Crénézumab pour une utilisation selon l'une quelconque des revendications 1 à 7, dans lequel le crénézumab est formulé pour une injection intraveineuse.

9. Crénézumab pour une utilisation selon l'une quelconque des revendications 1 à 8, dans lequel l'événement indésirable est l'ARIA-E.

10. Crénézumab pour une utilisation selon la revendication 9, dans lequel :
(a) si une ARIA-E suite au traitement est détectée, la méthode comprend la modification de l'administration du crénézumab, et éventuellement l'administration d'un traitement pour l'ARIA-E ; ou
(b) si une ARIA-E suite au traitement est détectée, la méthode comprend la modification de l'administration du crénézumab, et éventuellement l'administration d'un traitement pour l'ARIA-E et la méthode comprend en outre la reprise de l'administration dudit crénézumab une fois que l'ARIA-E est réglée et le crénézumab est administré à une plus faible dose qu'avant la modification de l'administration.

11. Crénézumab pour une utilisation selon l'une quelconque des revendications 1 à 8, dans lequel si une ou plusieurs nouvelles ARIA-E sont détectées chez le patient pendant le traitement avec ledit crénézumab, la méthode comprend l'arrêt de l'administration de crénézumab, et éventuellement l'administration d'un corticoïde.

12. Crénézumab pour une utilisation selon l'une quelconque des revendications 1 à 11, dans lequel le patient est actuellement traité par un ou plusieurs agents choisis dans le groupe constitué par : un agent thérapeutique qui se lie spécifiquement à une cible ; un inhibiteur de la cholinestérase ; un antagoniste des récepteurs NMDA : un agent de déplétion monoaminé ; un mésylate ergoloïde ; un agent antiparkinsonien anticholinergique ; un agent antiparkinsonien dopaminergique ; une tétrabenazine ; un agent antiinflammatoire ; une hormone ; une vitamine ; une diméboline ; une homotaurine ; un modulateur de l'activité du récepteur de la sérotonine ; un interféron ; un glucocorticoïde ; un anticorps anti-Abêta autre que le crénézumab ; un antibiotique ; et un agent anti-viral.

13. Crénézumab pour une utilisation selon la revendication 12, dans lequel l'agent est un inhibiteur de la cholinestérase choisi dans le groupe constitué par la galantamine, le donépézil, la rivastigmine et la tacrine.

14. Crénézumab pour une utilisation selon la revendication 12, dans lequel l'agent est un antagoniste des récepteurs NMDA, éventuellement la mémantine, ou un sel de celle-ci.

15. Crénézumab pour une utilisation selon la revendication 12, dans lequel l'agent est un agent thérapeutique qui se lie spécifiquement à une cible et la cible est choisie dans le groupe constitué par la bêta sécrétase, la tau, la préséniline, la protéine précurseur d'amyloïde ou des parties de celle-ci, un peptide bêta-amyloïde ou des oligomères ou des fibrilles de celui-ci, le récepteur de mort 6 (DR6), le récepteur des produits terminaux de glycation (RAGE), la parkine et la huntingtine.

16. Crénézumab pour une utilisation selon la revendication 12, dans lequel l'agent est
(a) un agent de déplétion monoaminé, éventuellement la tétrabénazine ; ou
(b) un agent antiparkinsonien anticholinergique choisi dans le groupe constitué par la procyclidine, la diphénhydramine, le trihexylphénidyle, la benzatropine, le bipéridène et le trihexyphénidyle ; ou
(c) un agent antiparkinsonien dopaminergique choisi dans le groupe constitué par : l'entacapone, la sélégiline, le pramipexole, la bromocriptine, la rotigotine, la sélégiline, le ropinirole, la rasagiline, l'apomorphine, le carbidopa, la lévodopa, le pergolide, la tolcapone et l'amantadine ; ou
(d) un agent anti-inflammatoire choisi dans le groupe constitué par : un médicament anti-inflammatoire non stéroïdien et l'indométhacine ; ou
(e) une hormone choisie dans le groupe constitué par : l'oestrogène, la progestérone et le leuprolide ; ou
(f) une vitamine choisie dans le groupe constitué par : l'acide folique et le nicotinamide ; ou
(g) une homotaurine, qui est l'acide 3-aminopropanesulfonique ou 3APS ; ou
(h) le xaliprodène ; ou
(i) un anticorps anti-Abêta autre que le crénézumab, éventuellement dans lequel l'anticorps est choisi dans le groupe constitué par le solanézumab, le bapineuzumab, l'aducanumab et le ganténérumab.


### SEQUENCE LISTING

<110> GENENTECH, INC., ET AL.
<120> METHODS OF TREATING ALZHEIMER'S DISEASE
<130> P5696R1-WO
<140>
   <141>
<150> 62/081,992
   <151> 2014-11-19
<150> 62/010,259
   <151> 2014-06-10
<150> 61/971,479
   <151> 2014-03-27
<150> 61/937,472
   <151> 2014-02-08
<160> 9
<170> PatentIn version 3.5
<210> 1
   <211> 42
   <212> PRT
   <213> Homo sapiens
<400> 1
<210> 2
   <211> 10
   <212> PRT
   <213> Artificial Sequence
<220>
   <221> source
   <223> /note="Description of Artificial Sequence: Synthetic peptide"
<400> 2
   Gly Phe Thr Phe Ser Ser Tyr Gly Met Ser
   1               5                   10
<210> 3
   <211> 16
   <212> PRT
   <213> Artificial Sequence
<220>
   <221> source
   <223> /note="Description of Artificial Sequence: Synthetic peptide"
<400> 3
   Ser Ile Asn Ser Asn Gly Gly Ser Thr Tyr Tyr Pro Asp Ser Val Lys
   1               5                   10                  15
<210> 4
   <211> 3
   <212> PRT
   <213> Artificial Sequence
<220>
   <221> source
   <223> /note="Description of Artificial Sequence: Synthetic peptide"
<400> 4
   Gly Asp Tyr
   1
<210> 5
   <211> 438
   <212> PRT
   <213> Artificial Sequence
<220>
   <221> source
   <223> /note="Description of Artificial Sequence: Synthetic polypeptide"
<400> 5
   Glu Val Gln Leu Val Glu Ser Gly Gly Gly Leu Val Gln Pro Gly Gly
   1               5                   10                  15
   Ser Leu Arg Leu Ser Cys Ala Ala Ser Gly Phe Thr Phe Ser Ser Tyr
               20                  25                  30
   Gly Met Ser Trp Val Arg Gln Ala Pro Gly Lys Gly Leu Glu Leu Val
           35                  40                  45
   Ala Ser Ile Asn Ser Asn Gly Gly Ser Thr Tyr Tyr Pro Asp Ser Val
       50                  55                  60
   Lys Gly Arg Phe Thr Ile Ser Arg Asp Asn Ala Lys Asn Ser Leu Tyr
   65                  70                  75                  80
   Leu Gln Met Asn Ser Leu Arg Ala Glu Asp Thr Ala Val Tyr Tyr Cys
                   85                  90                  95
   Ala Ser Gly Asp Tyr Trp Gly Gln Gly Thr Thr Val Thr Val Ser Ser
               100                 105                 110
   Ala Ser Thr Lys Gly Pro Ser Val Phe Pro Leu Ala Pro Cys Ser Arg
           115                 120                 125
   Ser Thr Ser Glu Ser Thr Ala Ala Leu Gly Cys Leu Val Lys Asp Tyr
       130                 135                 140
   Phe Pro Glu Pro Val Thr Val Ser Trp Asn Ser Gly Ala Leu Thr Ser
   145                 150                 155                 160
   Gly Val His Thr Phe Pro Ala Val Leu Gln Ser Ser Gly Leu Tyr Ser
                   165                 170                 175
   Leu Ser Ser Val Val Thr Val Pro Ser Ser Ser Leu Gly Thr Lys Thr
               180                 185                 190
   Tyr Thr Cys Asn Val Asp His Lys Pro Ser Asn Thr Lys Val Asp Lys
           195                 200                 205
   Arg Val Glu Ser Lys Tyr Gly Pro Pro Cys Pro Pro Cys Pro Ala Pro
       210                 215                 220
   Glu Phe Leu Gly Gly Pro Ser Val Phe Leu Phe Pro Pro Lys Pro Lys
   225                 230                 235                 240
   Asp Thr Leu Met Ile Ser Arg Thr Pro Glu Val Thr Cys Val Val Val
                   245                 250                 255
   Asp Val Ser Gln Glu Asp Pro Glu Val Gln Phe Asn Trp Tyr Val Asp
               260                 265                 270
   Gly Val Glu Val His Asn Ala Lys Thr Lys Pro Arg Glu Glu Gln Phe
           275                 280                 285
   Asn Ser Thr Tyr Arg Val Val Ser Val Leu Thr Val Leu His Gln Asp
       290                 295                 300
   Trp Leu Asn Gly Lys Glu Tyr Lys Cys Lys Val Ser Asn Lys Gly Leu
   305                 310                 315                 320
   Pro Ser Ser Ile Glu Lys Thr Ile Ser Lys Ala Lys Gly Gln Pro Arg
                   325                 330                 335
   Glu Pro Gln Val Tyr Thr Leu Pro Pro Ser Gln Glu Glu Met Thr Lys
               340                 345                 350
   Asn Gln Val Ser Leu Thr Cys Leu Val Lys Gly Phe Tyr Pro Ser Asp
   355 360 365
   Ile Ala Val Glu Trp Glu Ser Asn Gly Gln Pro Glu Asn Asn Tyr Lys
   370 375 380
   Thr Thr Pro Pro Val Leu Asp Ser Asp Gly Ser Phe Phe Leu Tyr Ser
   385 390 395 400
   Arg Leu Thr Val Asp Lys Ser Arg Trp Gln Glu Gly Asn Val Phe Ser
   405 410 415
   Cys Ser Val Met His Glu Ala Leu His Asn His Tyr Thr Gln Lys Ser
   420 425 430
   Leu Ser Leu Ser Leu Gly
   435
<210> 6
   <211> 16
   <212> PRT
   <213> Artificial Sequence
<220>
   <221> source
   <223> /note="Description of Artificial Sequence: Synthetic peptide"
<400> 6
   Arg Ser Ser Gln Ser Leu Val Tyr Ser Asn Gly Asp Thr Tyr Leu His
   1 5 10 15
<210> 7
   <211> 7
   <212> PRT
   <213> Artificial Sequence
<220>
   <221> source
   <223> /note="Description of Artificial Sequence: Synthetic peptide"
<400> 7
   Lys Val Ser Asn Arg Phe Ser
   1 5
<210> 8
   <211> 9
   <212> PRT
   <213> Artificial Sequence
<220>
   <221> source
   <223> /note="Description of Artificial Sequence: Synthetic peptide"
<400> 8
   Ser Gln Ser Thr His Val Pro Trp Thr
   1 5
<210> 9
   <211> 219
   <212> PRT
   <213> Artificial Sequence
<220>
   <221> source
   <223> /note="Description of Artificial Sequence: Synthetic polypeptide"
<400> 9
   Asp Ile Val Met Thr Gln Ser Pro Leu Ser Leu Pro Val Thr Pro Gly
   1               5                   10                  15
   Glu Pro Ala Ser Ile Ser Cys Arg Ser Ser Gln Ser Leu Val Tyr Ser
               20                  25                  30
   Asn Gly Asp Thr Tyr Leu His Trp Tyr Leu Gln Lys Pro Gly Gln Ser
           35                  40                  45
   Pro Gln Leu Leu Ile Tyr Lys Val Ser Asn Arg Phe Ser Gly Val Pro
       50                  55                  60
   Asp Arg Phe Ser Gly Ser Gly Ser Gly Thr Asp Phe Thr Leu Lys Ile
   65                  70                  75                  80
   Ser Arg Val Glu Ala Glu Asp Val Gly Val Tyr Tyr Cys Ser Gln Ser
                   85                  90                  95
   Thr His Val Pro Trp Thr Phe Gly Gln Gly Thr Lys Val Glu Ile Lys
               100                 105                 110
   Arg Thr Val Ala Ala Pro Ser Val Phe Ile Phe Pro Pro Ser Asp Glu
           115                 120                 125
   Gln Leu Lys Ser Gly Thr Ala Ser Val Val Cys Leu Leu Asn Asn Phe
       130                 135                 140
   Tyr Pro Arg Glu Ala Lys Val Gln Trp Lys Val Asp Asn Ala Leu Gln
   145                 150                 155                 160
   Ser Gly Asn Ser Gln Glu Ser Val Thr Glu Gln Asp Ser Lys Asp Ser
                   165                 170                 175
   Thr Tyr Ser Leu Ser Ser Thr Leu Thr Leu Ser Lys Ala Asp Tyr Glu
               180                 185                 190
   Lys His Lys Val Tyr Ala Cys Glu Val Thr His Gln Gly Leu Ser Ser
           195                 200                 205
   Pro Val Thr Lys Ser Phe Asn Arg Gly Glu Cys
       210                 215